# EUROPEAN PATENT APPLICATION

(11) **EP 3 556 399 A1**
(43) Date of publication of application: **23.10.2019**
(21) Application number: 17882909.9
(22) Date of filing: 19.12.2017
(51) Int. Cl.: A61K 47/64, A61K 47/65, A61K 47/68, A61K 9/00, C07K 7/08, C07K 14/00, A61K 47/62, A61K 47/10

(54) **BRAIN TARGETING LONG-ACTING PROTEIN CONJUGATE**

(30) Priority: 19.12.2016 KR 20160173863; 28.09.2017 KR 20170126537
(71) Applicant: Hanmi Pharm. Co., Ltd., Hwaseong-si, Gyeonggi-do 18536 (KR)
(72) Inventor: JEONG, Eui Joon, Hwaseong-si Gyeonggi-do 18469 (KR); MOON, Mi Jin, Hwaseong-si Gyeonggi-do 18469 (KR); KIM, Jeong A, Hwaseong-si Gyeonggi-do 18469 (KR); JUNG, Sung Youb, Hwaseong-si Gyeonggi-do 18469 (KR)
(74) Representative: Mewburn Ellis LLP
(86) International application number: PCT/KR2017/015055
(87) International publication number: WO 2018/117613

(57) **Abstract**

The present invention relates to a long-acting conjugate for brain targeting comprising a peptide for brain targeting and a physiologically active material, and to a long-acting conjugate comprising a physiologically active material with improved durability and stability, which can pass through the blood-brain barrier (BBB) and comprises a physiologically active material. The long-acting conjugate for brain targeting of the present invention comprising a peptide for brain targeting and a physiologically active material can pass through the blood-brain barrier, thus enabling the treatment of diseases associated with brain diseases, and additionally, can maintain the activity of a physiologically active material *in vivo* and increase its half-life in the blood.

## Description

### [Technical Field]

The present invention relates to a long-acting protein conjugate capable of targeting the brain and a method of preparing the same. Additionally, the present invention relates to a novel peptide for brain targeting and use thereof.

### [Background Art]

Despite the growing need for effective delivery of therapeutic agents to the brain, the development of new agents for the brain is progressing at a much slower rate than those for the rest of the body. This slow progress is largely due to the situation where most drugs cannot penetrate into the brain through the brain capillary wall that forms the blood-brain barrier (hereinafter, BBB). The BBB is a structure established by tight junctions between the endothelial cells of the cerebral blood vessels and astrocytes that further strengthen these tight junctions, and it has the role of preventing substances in the blood from diffusing freely into the brain parenchyma through the blood vessel walls. For this reason, many drugs developed for the treatment of brain diseases have a problem in that they cannot pass through the BBB well. Almost 100% of macro-molecular drugs and over 98% of small-molecule drugs cannot pass through the BBB. Only a very small number of drugs, for example, small-molecule drugs which have high lipid solubility and molecular weights of 400 to 500daltons or less, can actually pass through the BBB. Additionally, among these small molecules capable of passing through the BBB, only a small fraction can pass through the BBB in a pharmaceutically significant amount.

Only some brain diseases, such as depression, affective disorder, chronic pain, and epilepsy, can respond to small-molecule drugs that pass through the BBB. A greater majority of brain diseases, such as Alzheimer's disease, stroke/neuroprotection, brain and spinal cord injury, brain cancer, HIV infection in the brain, ataxia-producing disorders, amyotrophic lateral sclerosis (ALS), Huntington disease, childhood inborn genetic errors affecting the brain, Parkinson's disease, and multiple sclerosis do not respond to conventional lipid-soluble small-molecular weight drugs.

Currently, development of carriers capable of delivering drugs through the BBB is actively underway. Meanwhile, although peptides with specific amino acid sequences are known to pass through the BBB and are able to deliver drugs, siRNAs, *etc.,* however, the research on the possibility of delivering a large-sized physiologically active material with a particular structure is still far below expectation (Kumar et al., Nature. 2007, 448: 39 to 43). Additionally, there is an increased need for the development and study of new and improved drugs with respect to the treatment of brain diseases, for which only a very few effective small-molecule drugs are available.

### [Disclosure]

### [Technical Problem]

An object of the present invention is to provide a long-acting conjugate for brain targeting, which can pass through the blood-brain barrier (BBB), maintain effectiveness of physiological activity, and contain a bio-compatible material capable of extending the half-life of a physiologically active material.

Another object of the present invention is to provide a long-acting conjugate for brain targeting, in which a physiologically active material is linked, by a mutual binding, to an immunoglobulin Fc region of a peptide for brain targeting to which the immunoglobulin Fc region is fused.

Still another object of the present invention is to provide a long-acting conjugate for brain targeting of Formula 1, which is a long-acting conjugate containing a peptide for brain targeting and a physiologically active material.

X-L₁-F-L₂-Y

wherein,
X is a physiologically active material;
Y is a peptide for brain targeting;
L₁ and L₂ are peptide linkers or non-peptide linkers, in which when L₁ and L₂ are peptide linkers, the peptide linkers include 0 to 1,000 amino acid(s); and
F is an immunoglobulin constant region comprising an FcRn-binding region.

Still another object of the present invention is to provide a polynucleotide which encodes a peptide for brain targeting, to which the immunoglobulin Fc region is linked by fusion or a non-peptide linker, or a physiologically active material; a vector containing the polynucleotide; a transformant containing the vector; and a kit which includes one or more of the polynucleotide, vector, and transformant.

Still another object of the present invention is to provide a long-acting conjugate for brain targeting after the peptide conjugate for brain targeting is encoded and then chemically linked, wherein an immunoglobulin Fc region of a peptide conjugate for brain targeting (which is fused to a peptide for brain targeting of the peptide conjugate for brain targeting) is linked to a physiologically active material by a mutual binding..

Still another object of the present invention is to provide a method for preparing the long-acting conjugate for brain targeting after the peptide conjugate for brain targeting is encoded and then chemically linked, wherein an immunoglobulin Fc region of a peptide conjugate for brain targeting (which is fused to a peptide for brain targeting of the peptide conjugate for brain targeting) is linked to a physiologically active material by a mutual binding.

Still another object of the present invention is to provide a method for preparing the long-acting conjugate for brain targeting of Formula 1 above.

Still another object of the present invention is to provide a composition containing the long-acting conjugate for brain targeting.

Still another object of the present invention is to provide a use of the long-acting conjugate for brain targeting in the preparation of pharmaceutical agents.

Still another object of the present invention is to provide a novel peptide for brain targeting.

Still another object of the present invention is to provide a polynucleotide encoding the peptide for brain targeting; a vector containing the polynucleotide; a transformant containing the vector; and a kit which includes one or more of the polynucleotide, vector, and transformant.

### [Technical Solution]

To achieve the above objects, an aspect of the present invention provides a long-acting conjugate for brain targeting, which can pass through the BBB, maintain effectiveness of physiological activity, and contain a bio-compatible material capable of extending the half-life of a physiologically active material; or a method for preparing the long-acting conjugate for brain targeting.

In a more specific embodiment, the present invention provides a long-acting conjugate for brain targeting of Formula 1:

[Formula 1] X-L₁-F-L₂-Y

wherein,
X is a physiologically active material;
Y is a peptide for brain targeting;
L₁ and L₂ are peptide linkers or non-peptide linkers, in which when L₁ and L₂ are peptide linkers, the peptide linkers include 0 to 1,000 amino acids; and
F is an immunoglobulin constant region comprising an FcRn-binding region.

In a specific embodiment, F may be in a dimeric form.

In the long-acting conjugate for brain targeting according to the previous embodiments, the long-acting conjugate passes through the blood-brain barrier and delivers a physiologically active material into the brain tissue.

In the long-acting conjugate for brain targeting according to the previous embodiments, the peptide for brain targeting includes a peptide, protein, or antibody, and in which each of the peptide, protein, or antibody contains an amino acid sequence allowing passage through the blood-brain barrier.

In the long-acting conjugate for brain targeting according to any one of the previous embodiments, the peptide for brain targeting may pass through the blood-brain barrier through a pathway by passive transport or a pathway by receptor-mediated transport, but the transport pathway is not limited thereto. For example, the peptide for brain targeting may be a peptide, protein, or antibody that can pass through the blood-brain barrier, but their types and sizes are not particularly limited.

In the long-acting conjugate for brain targeting according to any one of the previous embodiments, the term "amino acid" refers to an amino acid moiety which includes any naturally occurring or non-naturally occurring or synthetic amino acid residue, that is, any moiety which includes at least one carboxyl residue and at least one amino residue, which are directly linked by one, two, three, or more carbon atom(s), typically by a single (α) carbon atom.

In the long-acting conjugate for brain targeting according to any one of the previous embodiments, the peptide for brain targeting passes through the blood-brain barrier through a pathway by receptor-mediated transport.

In the long-acting conjugate for brain targeting according to any one of the previous embodiments, the pathway by receptor-mediated transport is characterized in that the passage through the blood-brain barrier is performed by a receptor-mediated transport pathway through any one selected from the group consisting of an insulin receptor, transferrin receptor, low-density lipoprotein receptor, low-density lipoprotein receptor-related protein, leptin receptor, nicotinic acetylcholine receptor, glutathione transporter, calcium-activated potassium channel, and receptor for advanced glycation endproducts (RAGE), and ligands of the receptors and antibody binding to the receptors or ligands, but is not limited thereto. The peptide for brain targeting may be selected from the group consisting of a peptide, protein, or antibody that can pass through the blood-brain barrier, or it may be a partial peptide sequence isolated from the peptide, protein, or antibody, but is not limited thereto.

In the long-acting conjugate for brain targeting according to any one of the previous embodiments, the physiologically active material may be selected from the group consisting of toxins; or glucagon like peptide-1 (GLP-1) receptor agonists; glucagon receptor agonists; gastric inhibitory polypeptide (GIP) receptor agonists; fibroblast growth factor (FGF) receptor agonists; cholecystokinin receptor agonists; gastrin receptor agonists; melanocortin receptor agonists; human growth hormone; growth hormone-releasing hormone; growth hormone-releasing peptide; interferons; interferon receptors; colony-stimulating factors (granulocyte colony-stimulating factors); interleukins; interleukin receptors; enzymes; interleukin-binding proteins; cytokine-binding proteins; macrophage-activating factors; macrophage peptides; B cell factors; T cell factors; protein A; allergy-inhibiting factors; necrosis glycoproteins; immunotoxins; lymphotoxins; tumor necrosis factors; tumor suppressors; transforming growth factors; α-1 antitrypsin; albumin; α-lactalbumin; apolipoprotein-E; erythropoietin; high-glycosylated erythropoietin; angiopoietins; hemoglobins; thrombin; thrombin receptor-activating peptide; thrombomodulin; blood coagulation factor VII; blood coagulation factor VIIa; blood coagulation factor VIII; blood coagulation factor IX; blood coagulation factor XIII; plasminogen activators; fibrin-binding peptides; urokinase; streptokinase; hirudin; protein C; C-reactive protein; renin inhibitor; collagenase inhibitors; superoxide dismutase; leptin; platelet-derived growth factor; epithelial growth factor; epidermal growth factor; angiostatin; angiotensin; bone morphogenetic growth factor; bone morphogenetic protein; calcitonin; insulin; atriopeptin; cartilage-inducing factor; elcatonin; connective tissue-activating factor; tissue factor pathway inhibitor; follicle-stimulating hormone; luteinizing hormone; luteinizing hormone-releasing hormone; nerve growth factors; axogenesis factor-1; brain-natriuretic peptide; glial-derived neurotrophic factor; netrin; neutrophil inhibitory factor; neurotrophic factor; neurturin; parathyroid hormone; relaxin; secretin; somatomedin; insulin-like growth factor; adrenocortical hormone; glucagon; cholecystokinin; pancreatic polypeptides; gastrin-releasing peptides; corticotropin-releasing factor; thyroid-stimulating hormone; autotaxin; lactoferrin; myostatin; activity-dependent neuroprotective protein (ADNP), β-secretase1 (BACE1), amyloid precursor protein (APP), neural cell adhesion molecule (NCAM), amyloid β, tau, receptor for advanced glycation endproducts (RAGE), α-synuclein, or agonists or antagonists thereof; receptors, receptor agonists; cell surface antigens; monoclonal antibody; polyclonal antibody; antibody fragments; virus-derived vaccine antigens; hybrid polypeptides or chimeric polypeptides that activate at least one receptor agonist; and analogues thereof.

In the long-acting conjugate for brain targeting according to any one of the previous embodiments, the toxin is selected from the group consisting of maytansine or a derivative thereof, auristatin or a derivative thereof, duocarmycin or a derivative thereof, and pyrrolobenzodiazepine (PBD) or a derivative thereof;
the glucagon like peptide-1 (GLP-1) receptor agonist is selected from the group consisting of native exendin-3 or native exendin-4, and analogues thereof;
the FGF receptor agonist is selected from the group consisting of FGF1 or an analogue thereof, FGF19 or an analogue thereof, FGF21 or an analogue thereof, and FGF23 or an analogue thereof;
the interferon is selected from the group consisting of interferon-a, interferon-β, and interferon-γ;
the interferon receptor is selected from the group consisting of interferon-a receptor, interferon-β receptor, interferon-y receptor, and soluble type I interferon receptors;
the interleukin is selected from the group consisting of interleukin-1, interleukin-2, interleukin-3, interleukin-4, interleukin-5, interleukin-6, interleukin-7, interleukin-8, interleukin-9, interleukin-10, interleukin-11, interleukin-12, interleukin-13, interleukin-14, interleukin-15, interleukin-16, interleukin-17, interleukin-18, interleukin-19, interleukin-20, interleukin-21, interleukin-22, interleukin-23, interleukin-24, interleukin-25, interleukin-26, interleukin-27, interleukin-28, interleukin-29, and interleukin-30;
the interleukin receptor is interleukin- 1 receptor or interleukin-4 receptor;
the enzyme is selected from the group consisting of β-glucosidase, α-galactosidase, β-galactosidase, iduronidase, iduronate-2-sulfatase, galactose-6-sulfatase, acid α-glucosidase, acid ceramidase, acid sphingomyelinsase, galactocerebrosidsase, arylsulfatase A, arylsulfatase B, β-hexosaminidase A, β-hexosaminidase B, heparin *N*-sulfatase, α-D-mannosidase, β-glucuronidase, *N*-acetylgalactosamine-6 sulfatase, lysosomal acid lipase, α-*N*-acetyl-glucosaminidase, glucocerebrosidase, butyrylcholinesterase, chitinase, glutamate decarboxylase, imiglucerase, lipase, uricase, platelet-activating factor acetylhydrolase, neutral endopeptidase, myeloperoxidase, α-galactosidase-A, agalsidase α, agalsidase β, α-L-iduronidase, butyrylcholinesterase, chitinase, glutamate decarboxylase, and imiglucerase;
the interleukin-binding protein is IL-18bp;
the cytokine-binding protein is tumor necrosis factor (TNF)-binding protein;
the nerve growth factors are selected from the group consisting of nerve growth factor, ciliary neurotrophic factor, axogenesis factor-1, brain-natriuretic peptide, glial-derived neurotrophic factor, netrin, neutrophil inhibitory factor, neurotrophic factor, and neurturin;
the myostatin receptor is selected from the group consisting of TNFR (P75), TNFR (P55), IL-1 receptor, VEGF receptor, and B cell activating factor receptor;
the myostatin receptor antagonist is IL1-Ra;
the cell surface antigen is selected from the group consisting of CD2, CD3, CD4, CD5, CD7, CD11a, CD11b, CD18, CD19, CD20, CD23, CD25, CD33, CD38, CD40, CD45, and CD69; and
the antibody fragments are selected from the group consisting of scFv, Fab, Fab', F(ab')₂, and Fd.

In the long-acting conjugate for brain targeting according to any one of the previous embodiments, the GLP-1 receptor agonist is selected from the group consisting of a native exendin-4; an exendin-4 derivative in which the N-terminal amine group of exendin-4 is deleted; an exendin-4 derivative in which the N-terminal amine group of exendin-4 is substituted with a hydroxyl group; an exendin-4 derivative in which the N-terminal amine group of exendin-4 is modified with a dimethyl group; an exendin-4 derivative in which the N-terminal amine group of exendin-4 is substituted with a carboxyl group; an exendin-4 derivative in which the α-carbon of the 1^{st} amino acid of exendin-4, histidine, is deleted; an exendin-4 derivative in which the 12^{th} amino acid of exendin-4, lysine, is substituted with serine, and an exendin-4 derivative in which the 12^{th} amino acid of exendin-4, lysine, is substituted with arginine.

In the long-acting conjugate for brain targeting according to any one of the previous embodiments, Li is linked to the N-terminal region of F, and L₂ is linked to the C-terminal region of F.

In the long-acting conjugate for brain targeting according to any one of the previous embodiments, the F-L₂-Y of Formula 1 is represented by the following Formula 2: 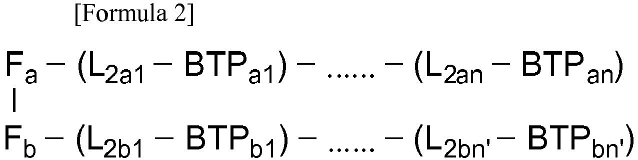 in which:
Fₐ and F_{b} are each a single-stranded polypeptide chain, which comprises an immunoglobulin Fc region comprising a hinge region, CH2 domain, and CH3 domain, in which Fₐ and F_{b} are linked by a disulfide bond in the hinge region, whereby the conjugate comprises an Fc fragment, and Fₐ and Li are covalently bonded;
each of BTPₐ₁, ......., BTPₐₙ, being the same as or different from one another, is a peptide for brain targeting;
each of BTP_{b1}, ......, BTP_{bn'}, being the same as or different from one another, is a peptide for brain targeting;
each of L₂ₐ₁, ......, L₂ₐₙ, being the same as or different from one another, is a peptide linker; and
each of L_{2b1}, ......, L_{2bn'}, being the same as or different from one another, is a peptide linker;
in which n and n' are each independently an integer.

In the long-acting conjugate for brain targeting according to any one of the previous embodiments, Li is linked to the N-terminus of Fₐ, and L₂ₐ₁ and L_{2b1} are each linked to the C-terminus of Fₐ and F_{b}.

In the long-acting conjugate for brain targeting according to any one of the previous embodiments, n = n', and each meets the conditions of L₂ₐ₁ = L_{2b1}, ......, L₂ₐₙ = L_{2bn'} and BTPₐ₁ = BTP_{b1}, ......, BTPₐₙ = BTP_{bn'}.

In the long-acting conjugate for brain targeting according to any one of the previous embodiments, F is Fₐ and F_{b}, L₂-Y is (L₂ₐ-Y)ₙ and (L₂b-Y)_{n'}, in which the long-acting conjugate for brain targeting is represented by Formula 3 below: 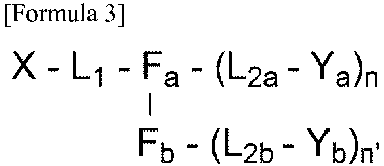
in which X is a physiologically active material;
Li is a peptide linker or non-peptide linker;
Fₐ and F_{b} are each an immunoglobulin Fc region which contain a hinge region, CH2 domain, and CH3 domain, and Fₐ and F_{b} are covalently bonded with each other by a disulfide bond between cysteine residues in the hinge region;
Yₐ and Y_{b}, being peptides for brain targeting, are the same kind as each other or different kinds from each other;
L₂ₐ and L_{2b}, being peptide linkers respectively, are the same kind as each other or different kinds from each other; and
n and n' are each independently an integer of 1 or more.

In the long-acting conjugate for brain targeting according to any one of the previous embodiments, Li is linked to the N-terminal region of Fₐ, and L₂ₐ and L_{2b} are each linked to the C-terminal region of Fₐ and F_{b}, respectively.

In the long-acting conjugate for brain targeting according to any one of the previous embodiments, Li is linked to an amine group or thiol group of X.

In the long-acting conjugate for brain targeting according to any one of the previous embodiments, Li is linked to the N-terminal amine group of X, the amine group located at a side chain of a lysine residue, or the -SH group (thiol group) located at a side chain of a cysteine residue.

In the long-acting conjugate for brain targeting according to any one of the previous embodiments, X, which is a physiologically active material, is a polypeptide consisting of 2 to 1,000 amino acids.

In the long-acting conjugate for brain targeting according to any one of the previous embodiments, n and n' are integers from 1 to 5.

In the long-acting conjugate for brain targeting according to any one of the previous embodiments, Li is a non-peptide linker having a size of 0.5 kDa to 100 kDa.

In the long-acting conjugate for brain targeting according to any one of the previous embodiments, the non-peptide linker is polyethylene glycol.

In the long-acting conjugate for brain targeting according to any one of the previous embodiments, L₂, being a peptide linker, is (GS)ₘ, (GGS)ₘ, (GGGS)ₘ, or (GGGGS)ₘ, in which m is 1 to 10.

In the long-acting conjugate for brain targeting according to any one of the previous embodiments, one of L₁ and L₂ of Formula 1 is a peptide linker and the other is a non-peptide linker.

In the long-acting conjugate for brain targeting according to any one of the previous embodiments, when Li of Formula 1 is a non-peptide linker and L₂ is a peptide linker, the peptide linker contains 0 to 1,000 amino acids.

In the long-acting conjugate for brain targeting according to any one of the previous embodiments, with respect to the long-acting conjugate for brain targeting of Formula 1, the N-terminus of X and the N-terminus of F are linked by Li and the N-terminus of Y and the C-terminus of F are linked by L₂.

In the long-acting conjugate for brain targeting according to any one of the previous embodiments, with respect to the long-acting conjugate for brain targeting of Formula 1, an end of Li is linked to a lysine residue or cysteine residue, *etc.* of X and the other end of Li is linked to the N-terminus of F; and the N-terminus of Y and the C-terminus of F are linked by L₂.

In the long-acting conjugate for brain targeting according to any one of the previous embodiments, when any one or both of L₁ and L₂ of Formula 1are a non-peptide linker, the non-peptide linker is selected from the group consisting of polyethylene glycol, polypropylene glycol, an ethylene glycol-propylene glycol copolymer, polyoxyethylated polyol, polyvinyl alcohol, a polysaccharide, dextran, polyvinyl ethyl ether, a biodegradable polymer, a lipid polymer, chitins, hyaluronic acid, a fatty acid, a high molecular weight polymer, a low molecular weight compound, a nucleotide, and a combination thereof.

In the long-acting conjugate for brain targeting according to any one of the previous embodiments, when any one or both of L₁ and L₂ of Formula 1 are a peptide linker, X and F, or F and Y are linked to each other by L₁ and L₂ via a covalent chemical bond, a non-covalent chemical bond, or a combination thereof; and Li and L₂ each contain 0 to 1,000 amino acids. When the peptide linker contains zero amino acid, they are linked by a peptide bond, which is a covalent bond.

In the long-acting conjugate for brain targeting according to any one of the previous embodiments, when any one or both of L₁ and L₂ of Formula 1 are a peptide linker, L₁ and L₂ contain no amino acids, in which (i) X and F, or F and Y are linked by a peptide bond; or (ii) X and F, and F and Y are linked by a peptide bond.

In the long-acting conjugate for brain targeting according to any one of the previous embodiments, when any one of L₁ and L₂ is a peptide linker and the other of the two is a non-peptide linker, the peptide linker is a linker containing 0 to 1,000 amino acid(s) and the non-peptide linker is polyethylene glycol.

In the long-acting conjugate for brain targeting according to any one of the previous embodiments, F of Formula 1 includes an immunoglobulin Fc region.

In the long-acting conjugate for brain targeting according to any one of the previous embodiments, the immunoglobulin Fc region includes one to four domains selected from the group consisting of CH1, CH2, CH3, and CH4 domains.

In the long-acting conjugate for brain targeting according to any one of the previous embodiments, the immunoglobulin Fc region further includes a hinge region.

In the long-acting conjugate for brain targeting according to any one of the previous embodiments, the immunoglobulin Fc region is an Fc region of IgG.

In the long-acting conjugate for brain targeting according to any one of the previous embodiments, with respect to the immunoglobulin Fc region, the constant region is derived from IgG1 or IgG4.

In the long-acting conjugate for brain targeting according to any one of the previous embodiments, the immunoglobulin Fc region is an IgG4 Fc region.

In the long-acting conjugate for brain targeting according to any one of the previous embodiments, the immunoglobulin Fc region is an aglycosylated IgG4 Fc region of a human sequence.

Another aspect of the present invention provides a method for preparing a long-acting conjugate for brain targeting of Formula 1.

In a specific embodiment, the method includes (i) preparing: (a) X-L₁-F, in which X, which is a physiologically active material, Li, which is a peptide or non-peptide linker, and F including an immunoglobulin Fc region, are linked; and (b) L₂-Y, in which Y, which is a peptide for brain targeting, and L₂, which is a peptide or non-peptide linker, are linked; and (ii) linking (a) X-L₁-F and (b) L₂-Y.

In another specific embodiment, Li of (a) is a peptide linker, and L₂ of (b) is a non-peptide linker.

In another specific embodiment, the method includes (i) preparing: (a) X-Li, in which X, which is a physiologically active material, and Li, which is a peptide or non-peptide linker, are linked; and (b) F-L₂-Y, in which Y, which is a peptide for brain targeting, L₂, which is a peptide or non-peptide linker, and F including an immunoglobulin Fc region, are linked; and (ii) linking (a) X-Li and (b) F-L₂-Y.

In still another specific embodiment, Li of (a) is a non-peptide linker, and L₂ of (b) is a peptide linker.

In still another specific embodiment, the method is a method for preparing the a long-acting conjugate for brain targeting of Formula 1, which includes (i) preparing F-L₂-Y, in which Y, which is a peptide for brain targeting, L₂, which is a peptide or non-peptide linker, and F including an immunoglobulin Fc region, are linked; and (ii) linking F-L₂-Y and X by L₁, which is a peptide or non-peptide linker.

In still another specific embodiment, Li is a non-peptide linker, and L₂ is a peptide linker.

In still another specific embodiment, the non-peptide linker and X and Y are linked by each of their respective functional groups, and in particular, the reactive group of the non-peptide linker is selected from the group consisting of an aldehyde group, a maleimide group, and a succinimide derivative.

Still another aspect of the present invention provides a long-acting conjugate comprising different physiologically active materials each other, in which a peptide conjugate for brain targeting, a peptide for brain targeting is fused to the immunoglobulin Fc region, is encoded and then chemically bound, and a different physiologically active material is linked to the chemically bound immunoglobulin Fc region.

The chemically bound immunoglobulin Fc region conjugate includes two immunoglobulin Fc regions (a dimeric form of an Fc region in which monomeric Fc regions are linked), and the physiologically active material which respectively binds to each of the two immunoglobulin Fc regions may be linked to a single site of the immunoglobulin Fc region (a monomer Fc region) or both sites of the two immunoglobulin Fc regions (each dimeric form of Fc region).

The chemical binding may be a covalent bond, more specifically a disulfide bond, and even more specifically a disulfide bond formed in a hinge region of two immunoglobulin Fc regions, but the binding is not particularly limited thereto.

More specifically, the long-acting conjugate may be in a form in which a dimer is formed by a chemical bond between (i) a first immunoglobulin Fc region, in which a peptide for brain targeting is linked to the C-terminal region of one molecule of an immunoglobulin Fc region through the above-described peptide linker (*e.g.,* a peptide bond), and (ii) a second immunoglobulin Fc region, in which a peptide for brain targeting is linked to the C-terminal region of one molecule of an immunoglobulin Fc region through the above-described peptide linker (*e.g.,* a peptide bond), and one molecule of a physiologically active material may be linked to the N-terminal region of one of the two immunoglobulin Fc region molecules; or may be in a form in which a physiologically active material is linked to each of the N-terminal regions of both of the immunoglobulin Fc region molecules, but the forms are not particularly limited thereto. The first and second immunoglobulin Fc regions, which are in the form of a fusion protein, may be fused in-frame by a peptide bond between the immunoglobulin Fc region and the peptide for brain targeting, and the immunoglobulin Fc region may include a hinge region, CH2 domain, and CH3 domain, but the immunoglobulin Fc region is not particularly limited thereto.

The chemical bond between the first and second immunoglobulin Fc regions may specifically be a covalent bond, more specifically a disulfide bond, and more specifically, a disulfide bond formed in the hinge region of the two immunoglobulin Fc regions, but the chemical bond is not particularly limited thereto.

Still another aspect of the present invention provides a composition containing the long-acting protein conjugate for brain targeting.

Still another aspect of the present invention provides a use of the long-acting protein conjugate for brain targeting in the preparation of pharmaceutical drugs.

Still another aspect of the present invention provides a novel peptide for brain targeting.

In a specific embodiment, the peptide for brain targeting is one consisting of any one amino acid sequence selected from amino acid sequences of SEQ ID NOS: 2, 4, 6, 8, 10, 20, 22, 24, 26, 28, 30, 32, 34, 36, 38, and 40.

Still another aspect of the present invention provides an isolated polynucleotide encoding the peptide for brain targeting.

Still another aspect of the present invention provides a recombinant expression vector containing the polynucleotide.

Still another aspect of the present invention provides a transformant containing the recombinant expression vector.

### [Advantageous Effects]

The long-acting conjugate for brain targeting of the present invention, which contains a peptide for brain targeting and a physiologically active material, has the effects that it can pass through the blood-brain barrier (BBB) *in vivo,* maintain physiological activity, and significantly increase blood half-life. The long-acting conjugate for brain targeting also has improved stability and thus can be used as a therapeutic agent for treating brain-related diseases. Additionally, the present invention provides a novel peptide for brain targeting, and thus a novel long-acting conjugate for brain targeting can be prepared for use as a therapeutic agent for treating brain-related diseases.

### [Brief Description of Drawings]

FIG. 1 shows the experimental results of transcytosis confirming the level of passage of peptides for brain targeting through the blood-brain barrier.
FIG. 2 shows the results of a brain distribution experiment performed in mice confirming the level of passage of peptides for brain targeting through the blood-brain barrier.
FIG. 3 shows the results confirming the level of passage of peptides for brain targeting, to which an immunoglobulin Fc region is linked, through the blood-brain barrier and the level of passage of an immunoglobulin Fc region alone through the blood-brain barrier.
FIG. 4 shows the experimental results of transcytosis confirming the level of passage of a long-acting conjugate for brain targeting, which contains a GIP derivative (*i*.*e*., a physiologically active material), through the blood-brain barrier.
FIG. 5 shows the results confirming the ability of a long-acting conjugate for brain targeting containing idursulfase (*i.e.,* a physiologically active material) with regard to the maintenance of physiological activity.

### [Best Mode]

Hereinafter, the present invention will be described in detail. Meanwhile, each of the explanations and exemplary embodiments disclosed herein can be applied to their respective other explanations and exemplary embodiments. That is, all of the combinations of various factors disclosed herein belong to the scope of the present invention. Additionally, the scope of the present invention should not be limited by the specific disclosure provided hereinbelow.

Additionally, an ordinary person skilled in the art will recognize or be able to confirm using no more than routine experimentation with respect to a number of equivalents to the specific embodiments of the invention described in the present invention. Additionally, such equivalents are intended to be included in the present invention.

Over the entire specification of the present invention, the conventional one-letter and three-letter codes for amino acids are used. Additionally, three-letter codes which are generally allowed for other amino acids, such as diaminopropionic acid (DAP), are used. Additionally, the amino acids mentioned herein are abbreviated according to the nomenclature rules of IUPAC-IUB.

An aspect of the present invention provides a long-acting conjugate for brain targeting which contains a peptide for brain targeting and a physiologically active material. More specifically, the long-acting conjugate for brain targeting may be one in which a peptide for brain targeting, which is linked based on an immunoglobulin Fc region by a peptide linker and/or direct fusion, or non-peptide linker, and a physiologically active material are linked, but is not limited thereto. The long-acting conjugate for brain targeting of the present invention is characterized in that a peptide for brain targeting is linked to an immunoglobulin Fc region by a peptide linker, direct fusion, or non-peptide linker, is not linked to a physiologically active material.

In a more specific embodiment, the present invention provides a long-acting conjugate for brain targeting of Formula 1 below:

[Formula 1] X-L₁-F-L₂-Y

in Formula 1,
X is a physiologically active material;
Y is a peptide for brain targeting;
L₁ and L₂ are peptide linkers or non-peptide linkers;
when L₁ and L₂ are peptide linkers, the peptide linkers contain 0 to 1,000 amino acids; and
F is an immunoglobulin constant region comprising an FcRn-binding region.

The long-acting conjugate form of the present invention is characterized in that a physiologically active material is linked based on a peptide for brain targeting and an Fc region. Specifically, the long-acting conjugate of the present invention is characterized in that the peptide for brain targeting is linked to an immunoglobulin Fc region by a peptide linker and the physiologically active material is linked thereto by a non-peptide linker; however, both types may be linked by a peptide linker and/or non-peptide linker, but the linkage is not limited thereto. The peptide linker may be a linker containing 0 to 1,000 amino acid(s), but any peptide linker which can form the conjugate of the present invention can be included without limitation. When the peptide linker contains no amino acids, the conjugate may be in a form fused by a peptide bond instead of a linker.

The "F-L₂-Y" of Formula 1 of the long-acting conjugate for brain targeting may be a long-acting conjugate for brain targeting having the structure of Formula 2 below.

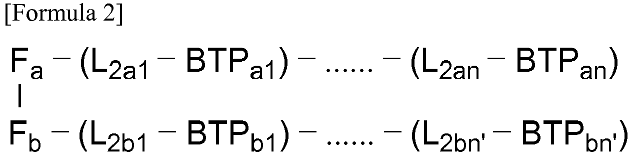

In Formula 2 above,
Fₐ and F_{b} are each a single-stranded polypeptide chain, which comprises an immunoglobulin Fc region comprising a hinge region, CH2 domain, and CH3 domain, in which Fₐ and F_{b} are linked by a disulfide bond in the hinge region, and thereby contains an Fc fragment, and Fₐ and Li are covalently bonded with each other;
each of BTPₐ₁, ......, BTPₐₙ, being the same as or different from one another, is a peptide for brain targeting;
each of BTP_{b1}, ......, BTP_{bn'}, being the same as or different from one another, is a peptide for brain targeting;
each of L₂ₐ₁, ......, L₂ₐₙ, being the same as or different from one another, is a peptide linker; and
each of L_{2b1}, ......, L_{2bn'}, being the same as or different from one another, is a peptide linker;
in which n and n' may be each independently an integer.

In the long-acting conjugate for brain targeting, Li may be linked to the N-terminus of Fₐ and L₂ₐ₁ and L_{2b1} may be each linked to the C-terminus of Fₐ and F_{b}, respectively.

In the long-acting conjugate for brain targeting of Formula 2, n = n', and each may meet the conditions of L₂ₐ₁ = L_{2b1}, ......, L₂ₐₙ = L_{2bn'} and BTPₐ₁ = BTP_{b1}, ......, BTPₐₙ = BTP_{bn'}, but the conditions are not limited thereto.

F of Formula 1 of the long-acting conjugate for brain targeting is Fₐ and F_{b} and "L₂-Y" is (L₂ₐ-Y)ₙ and (L₂b-Y)_{n'} and the long-acting conjugate for brain targeting may be represented by Formula 3 below.

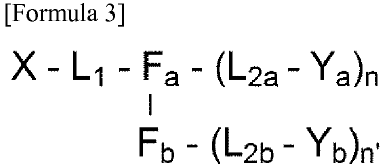

In Formula 3 above,
X is a physiologically active material;
Li is a peptide linker or non-peptide linker;
Fₐ and F_{b} are each an immunoglobulin Fc region which contain a hinge region, CH2 domain, and CH3 domain, and Fₐ and F_{b} are covalently bonded with each other by a disulfide bond between cysteine residues in the hinge region;
Yₐ and Y_{b}, being peptides for brain targeting, are the same kind as each other or different kinds from each other;
L₂ₐ and L_{2b}, being peptide linkers, are the same kind as each other or different kinds from each other; and
n and n' are each independently an integer of 1 or more.

When n and n' are each independently an integer of 2 or more, the long-acting conjugate for brain targeting may be one in which at least two of the same or different peptides for brain targeting are linked, and the at least two same or different peptides for brain targeting may be in a form linked in tandem, but the linkage form is not limited thereto.

N and n' may be an integer of 1 or 2 or more, and n and n' may be the same or different integers, but n and n' are not limited thereto. n and n' may be 1 to 10, and 1 to 5, for example, n and n' may be each 1, 2, 3, 4, 5, 6, 7, 8, 9, or 10, but the number of n is included without limitation, if the Fc region and a physiologically active material, being linked as parts constituting a moiety of the long-acting conjugate for brain targeting of the present invention to be delivered by passing through the BBB.

The long-acting conjugate for brain targeting may be one in which the N-terminal region of X and the N-terminal region of F are linked by Li, and the N-terminal region of Y and the C-terminal region of F are linked by L₂. Additionally, the long-acting conjugate for brain targeting may be in a form in which an end of Li is linked to a lysine residue or cysteine residue, *etc.* of X and the other end of Li is linked to the N-terminal region of F; and the N-terminal region of Y and the C-terminal region of F are linked by L₂.

The long-acting conjugate for brain targeting may be in a form in which Li is linked to the N-terminal region of F, and L₂ is linked to the C-terminal region of F; or Li is linked to the N-terminal region of Fₐ, and L₂ₐ and L_{2b} are each linked to the C-terminal region of Fₐ and F_{b}, respectively.

Li may be linked to an amine group or thiol group of X; and Li may be linked to the N-terminal amine group of X, the amine group located at a side chain of a lysine residue, or the -SH group (thiol group) located at a side chain of a cysteine residue, but is not limited thereto.

As used herein, the term "N-terminal region" refers to the amino terminal of a peptide or protein, and for the purposes of the present invention, refers to a site capable of binding to a linker including a non-peptide linker, *etc.* For example, the amino acid residues around the N-terminal region as well as the most terminal amino acid residues of the N-terminal region may all be included, and specifically, may include the first to twentieth amino acid residues from the most terminal region, but the sites of the amino acid residues are not particularly limited thereto.

As used herein, the term "C-terminal region" refers to the carboxyl terminal of a peptide or protein, and for the purposes of the present invention, refers to a site capable of binding to a linker including a non-peptide linker, *etc.* For example, the amino acid residues around the C-terminal as well as the most terminal amino acid residues of the C-terminal region may all be included, and specifically, may include the first to twentieth amino acid residues from the most terminal, but the sites of the amino acid residues are not particularly limited thereto.

As used herein, the term "long-acting conjugate for brain targeting" refers to a conjugate, which includes a peptide for brain targeting and a physiologically active material, and an immunoglobulin constant region, and has a structure in which the peptide for brain targeting and physiologically active material are each linked directly or indirectly to an FcRn-binding region, respectively. The term "long-acting conjugate for brain targeting" may be used interchangeably with "long-acting conjugate", and it refers to a material, where a physiologically active material that is included in the long-acting conjugate is linked to a biocompatible material which includes an immunoglobulin constant region capable of extending the duration of activity of the physiologically active material and has an FcRn-binding region, and the peptide linker and/or non-peptide linker containing 0 to 1,000 amino acid(s).

The long-acting conjugate for brain targeting can deliver a physiologically active material into the brain tissue by passing through the blood-brain barrier, but is not particularly limited thereto.

Hereinafter, the elements constituting the long-acting conjugate for brain targeting will be described in more detail.

As used herein, the term "peptide for brain targeting" includes a peptide, or protein, or antibody that contains an amino acid sequence that can pass through the BBB. The peptide for brain targeting corresponds to one moiety that constitutes a long-acting conjugate for brain targeting of the present invention.

Additionally, the peptide for brain targeting may be a sequence which is separated from known peptides, proteins, or antibodies and has an activity that can pass through the BBB.

As used herein, the term "amino acid" refers to an amino acid moiety which includes any naturally occurring or non-naturally occurring or synthetic amino acid residue, that is, any moiety which includes at least one carboxyl residue and at least one amino residue, which are directly linked by one, two, three, or more carbon atom(s), typically by a single (α) carbon atom.

The peptide for brain targeting of the present invention may be characterized in that it can pass through the blood-brain barrier through a pathway by passive transport or a pathway by receptor-mediated transport, but the transport pathway is not limited thereto. For example, the peptide for brain targeting may be a peptide, protein, or antibody that can pass through the blood-brain barrier, but their types and sizes are not particularly limited.

Meanwhile, the peptide for brain targeting may be one that passes through the blood-brain barrier through a pathway by receptor-mediated transport.

Meanwhile, the peptide for brain targeting may be one that passes through the blood-brain barrier through a pathway by receptor-mediated transport.

Specifically, the peptide for brain targeting may pass through the blood-brain barrier by the receptor-mediated transport pathway through any one selected from the group consisting of an insulin receptor, transferrin receptor, low-density lipoprotein receptor, low-density lipoprotein receptor-related protein, leptin receptor, nicotinic acetylcholine receptor, glutathione transporter, calcium-activated potassium channel, and receptor for advanced glycation endproducts (RAGE), and ligands of the receptors and an antibody binding to the receptors or ligands, but the pathway is not particularly limited thereto; and in addition, may be selected from the group consisting of peptides, proteins, and antibodies that can pass through the blood-brain barrier, or may be partial peptide sequences isolated from the peptides, proteins, and antibodies, but the pathway is not particularly limited thereto. For example, HAITYPRH peptide (BTP1 peptide) of SEQ ID NO: 2, THR peptide (BTP2 peptide) of SEQ ID NO: 4, Angiopep-2 peptide (BTP3 peptide) of SEQ ID NO: 6, ApoB peptide (BTP4 peptide) of SEQ ID NO: 8, ApoE peptide (BTP5 peptide) of SEQ ID NO: 10, *etc.* may be used, but any peptide which can deliver the linked F-L₂-Y structure to the brain by passing through the blood-brain barrier can be included without limitation.

In another specific embodiment, BTP6 peptide of SEQ ID NO: 20, BTP7 peptide of SEQ ID NO: 22, BTP8 peptide of SEQ ID NO: 24, BTP9 peptide of SEQ ID NO: 26, BTP10 peptide of SEQ ID NO: 28, BTP11 peptide of SEQ ID NO: 30, BTP12 peptide of SEQ ID NO: 32, BTP13 peptide of SEQ ID NO: 34, BTP14 peptide of SEQ ID NO: 36, BTP15 peptide of SEQ ID NO: 38, or BTP16 peptide of SEQ ID NO: 40, but the peptide is not limited thereto.

In another specific embodiment, the peptide for brain targeting may be:
(1) Angiopep-2: TFFYGGSRGKRNNFKTEEY-*OH* (SEQ ID NO: 41),
(2) ApoB (3371-3409): SVIDALQYKLEGTTRLTRKRGLKLATALSLSNKFVEGS (SEQ ID NO: 42),
(3) ApoE (159-167)₂: (LRKLRKRLL)₂ (SEQ ID NO: 43),
(4) Peptide-22: Ac-C(&)MPRLRGC(&)-*NH₂* (SEQ ID NO: 44),
(5) THR: THRPPMWSPVWP-*NH₂* (SEQ ID NO: 45),
(6) THR *retro-enantio:* pwvpswmpprht-*NH₂* (SEQ ID NO: 46),
(7) CRT: C(&)RTIGPSVC(&) (SEQ ID NO: 47),
(8) Leptin 30: YQQILTSMPSRNVIQISNDLENLRDLLHVL (SEQ ID NO: 48),
(9) RVG29: YTIWMPENPRPGTPCDIFTNSRGKRASNG-OH (SEQ ID NO: 49)
(10) ^{D}CDX: GreirtGraerwsekf-OH (SEQ ID NO: 50)
(11) Apamin: C(&₁)NC(&₂)KAPETALC(&₁)-ARRC(&₂)QQH-*NH*₂ (SEQ ID NO: 51),
(12) MiniAp-4: [Dap](&)KAPETALD(&) (SEQ ID NO: 52),
(13) GSH: γ-L-glutamyl-CG-OH
(14) G23: HLNILSTLWKYRC (SEQ ID NO: 53),
(15) g7: GFtGFLS(O-β-Glc)-*NH₂*(SEQ ID NO: 54),
(16) TGN: TGNYKALHPHNG (SEQ ID NO: 55),
(17) TAT (47-57): YGRKKRRQRRR-*NH₂* (SEQ ID NO: 56),
(18) SynB1: RGGRLSYSRRRFSTSTGR (SEQ ID NO: 57),
(19) Diketopiperazines: &(N-MePhe)-(N-MePhe)diketopiperazines, or
(20) PhPro: (Phenylproline)₄-*NH*₂ (SEQ ID NO: 58),
   but the peptide is not limited thereto.

In the sequences above, & is a symbol according to the nomenclature for cyclic peptides described in (J. Pept. Res., 2005, 65, 550 to 555; J. Spengler *et al*.), and "&" indicates the connecting point. For example, the first "&" in a single chain indicates the position of an end of a chemical bond and the second "&" indicates the position to which the chemical bond is attached. For example, "&Ala-Ala-Phe-Leu-Pro&" means that a chemical bond is formed between alanine and proline and thereby forms a cyclic peptide. In the case where two or more chemical bonds are present, codes such as &1 and &2 may be used so as to indicate the position where the corresponding chemical bond is formed. For example, in the case where it is indicated as "&1Asp(&2)-Trp-Phe-Dpr(&2)-Leu-Met&1", it means that a chemical bond is formed between Asp and Met, and a chemical bond is formed between Asp and Dpr. Meanwhile, [Dap] stands for diaminopropionic acid.

As used herein, the term "physiologically active material", which may be a constitution that forms a moiety of the long-acting conjugate for brain targeting, collectively refers to materials which have certain physiological activity *in vivo,* and these materials have a variety of physiological activities. The physiologically active material, X, may be a polypeptide consisting of 2 amino acids to 1,000 amino acids, 2 amino acids to 950 amino acids, 2 amino acids to 900 amino acids, 2 amino acids to 850 amino acids, 2 amino acids to 800 amino acids, 2 amino acids to 750 amino acids, 2 amino acids to 700 amino acids, 5 amino acids to 700 amino acids, 5 amino acids to 650 amino acids, 5 amino acids to 600 amino acids, 5 amino acids to 550 amino acids, 5 amino acids to 500 amino acids, 5 amino acids to 450 amino acids, 5 amino acids to 400 amino acids, 5 amino acids to 350 amino acids, 5 amino acids to 300 amino acids, 5 amino acids to 250 amino acids, 5 amino acids to 200 amino acids, 5 amino acids to 150 amino acids, 5 amino acids to 100 amino acids, 5 amino acids to 90 amino acids, about 5 amino acids to about 80 amino acids, 5 amino acids to 70 amino acids, 5 amino acids to 60 amino acids, 5 amino acids to 50 amino acids, 5 amino acids to 40 amino acids, t 5 amino acids to 30 amino acids, 5 amino acids to 25 amino acids, or 5 amino acids to 20 amino acids, but is not limited thereto. The polypeptides that constitute the physiologically active material include all of peptides, oligopeptides, polypeptides, and proteins.

The physiologically active material may be a toxin or physiologically active polypeptide, and may include various physiologically active polypeptides, such as cytokines, interleukin, interleukin-binding proteins, enzymes, antibodies, growth factor, transcription factors, blood coagulation factors, vaccines, structural proteins, ligand proteins or receptors, cell surface antigens, and receptor antagonists which are used for the purpose of preventing or treating human diseases, physiologically active peptides secreted in the small intestine and the pancreas which exhibit a therapeutic effect for diabetes and obesity, and G protein-coupled receptors (GPCR) agonists or antagonists, or analogues thereof but the physiologically active materials are not limited thereto.

As used herein, the term "analogue of X" refers to a material which can exhibit the same type of activity as that of X, and it includes all of agonists of X, derivatives of X, fragments of X, variants of X, *etc.*

Specifically, the toxin may be selected from maytansine and/or a derivative thereof, auristatin and/or a derivative thereof, duocarmycin and/or a derivative thereof, pyrrolobenzodiazepine (PBD) and/or a derivative thereof, which are effective for the apoptosis of cancer cells, but any toxin which exhibits an effect for the apoptosis of cancer cells can be included without limitation.

Specifically, examples of the physiologically active polypeptide may include GLP-1 receptor agonists, glucagon receptor agonists, gastric inhibitory polypeptide (GIP) receptor agonists, fibroblast growth factor (FGF) receptor agonists (FGF1, FGF19, FGF21, FGF23, *etc*.), cholecystokinin receptor agonists, gastrin receptor agonists, melanocortin receptor agonists, human growth hormone, growth hormone-releasing hormone, growth hormone-releasing peptide, interferons and interferon receptors (*e.g.,* interferon-a, -β and -γ, soluble type I interferon receptors, *etc*.), colony-stimulating factors, interleukins (*e.g.,* interleukin-1, -2, -3, -4, -5, -6, -7, -8, -9, -10, - 11, -12, -13, -14, -15, -16, -17, -18, -19, -20, -21, -22, -23, -24, -25, -26, -27, -28, -29, -30, *etc*.) and interleukin receptors (*e.g.,* IL-1 receptor, IL-4 receptor, *etc*.), enzymes (*e.g.,* β-glucosidase), α-galactosidase, β-galactosidase, iduronidase, iduronate-2-sulfatase, galactose-6-sulfatase, acid α-glucosidase, acid ceramidase, acid sphingomyelinsase, galactocerebrosidsase, arylsulfatase A, B, β-hexosaminidase A, B, heparin *N*-sulfatase, α-D-mannosidase, β-glucuronidase, N-acetylgalactosamine-6 sulfatase, lysosomal acid lipase, α-*N*-acetyl-glucosaminidase, glucocerebrosidase, butyrylcholinesterase, chitinase, glutamate decarboxylase, imiglucerase, lipase, uricase, platelet-activating factor acetylhydrolase, neutral endopeptidase, myeloperoxidase, α-galactosidase-A, agalsidase α, β, α-L-iduronidase, butyrylcholinesterase, chitinase, glutamate decarboxylase, imiglucerase, lipase, uricase, platelet-activating factor acetylhydrolase, neutral endopeptidase, and myeloperoxidase, *etc*.), interleukins and cytokine-binding proteins (*e.g.,* IL-18bp, TNF-binding protein, *etc*.), macrophage-activating factors, macrophage peptides, B cell factors, T cell factors, protein A, allergy-inhibiting factors, necrosis glycoproteins, immunotoxins, lymphotoxins, tumor necrosis factors, tumor suppressors, transforming growth factors, α-1 antitrypsin, albumin, α-lactalbumin, apolipoprotein-E, erythropoietin, high-glycosylated erythropoietin, angiopoietin, hemoglobins, thrombin, thrombin receptor-activating peptide, thrombomodulin, blood coagulation factor VII, blood coagulation factor VIIa, blood coagulation factor VIII, blood coagulation factor IX, blood coagulation factor XIII, plasminogen activators, fibrin-binding peptide, urokinase, streptokinase, hirudin, protein C, C-reactive protein, renin inhibitor, collagenase inhibitors, superoxide dismutase, leptin, platelet-derived growth factor, epithelial growth factor, epidermal growth factor, angiostatin, angiotensin, bone morphogenetic growth factor; bone morphogenetic protein, calcitonin, insulin, atriopeptin, cartilage-inducing factor, elcatonin, connective tissue-activating factor, tissue factor pathway inhibitor, follicle-stimulating hormone, luteinizing hormone, luteinizing hormone-releasing hormone, nerve growth factors (*e.g.,* nerve growth factor, ciliary neurotrophic factor, axogenesis factor-1, brain-natriuretic peptide, glial-derived neurotrophic factor, netrin, neutrophil inhibitory factor, neurotrophic factor, neurturin), parathyroid hormone, relaxin, secretin, somatomedin, insulin-like growth factor, adrenocortical hormone, glucagon, cholecystokinin, pancreatic polypeptide, gastrin-releasing peptides, corticotropin-releasing factor, thyroid-stimulating hormone, autotaxin, lactoferrin, myostatin, activity-dependent neuroprotective protein (ADNP), β-secretase1 (BACE1), amyloid precursor protein (APP), neural cell adhesion molecule (NCAM), amyloid β, tau, receptor for advanced glycation endproducts (RAGE), α-synuclein, or agonists or antagonists thereof, receptors thereof (*e.g*., TNFR(P75), TNFR(P55), IL-1 receptor, VEGF receptor, B cell activating factor receptor, *etc.*), receptor antagonist (*e.g.,* IL1-Ra, *etc*.), cell surface antigens (*e.g.,* CD 2, 3, 4, 5, 7, 11a, 11b, 18, 19, 20, 23, 25, 33, 38, 40, 45, 69, *etc*.), monoclonal antibody, polyclonal antibody, antibody fragments (*e.g*., scFv, Fab, Fab', F(ab')2 and Fd), virus-derived vaccine antigens, hybrid polypeptides or chimeric polypeptides that activate at least one receptor agonist, *etc.,* but the physiologically active polypeptide is not limited thereto.

As used herein, the term "iduronate-2-sulfatase", which is a sulfatase enzyme related to Hunter syndrome (MPS-II), is an enzyme necessary for lysosomal degradation of heparin sulfate and dermatan sulfate. In a specific embodiment of the present invention, "idursulfase", which is a purified type of iduronate-2-sulfatase, may be used as iduronate-2-sulfatase, and it is obvious that idursulfase is included in the iduronate-2-sulfatase. The idursulfase may be, for example, idursulfase α or idursulfase β, but is not limited thereto.

Iduronate-2-sulfatase can be prepared or manufactured by a method known in the art, and specifically, it can be purified from animal cells into which an animal cell expression vector was inserted and cultured and after cultivation thereof, or enzymes commercially available may be purchased for use, but the preparation methods of iduronate-2-sulfatase are not limited thereto.

The physiologically active polypeptide to be applicable in the present invention may be in a native form, or those prepared by genetic recombination in a prokaryotic cell such as *Escherichia coli* or a eukaryotic cell such as a yeast cell, insect cell, or animal cell. Additionally, the physiologically active polypeptide may be an analogue having activity equivalent to that of the native form or of the same species (*e.g.,* a mutant derivative at one or more amino acid positions), but the applicable physiologically active polypeptide is not limited thereto.

Meanwhile, the GLP-1 receptor agonist may be selected from the group consisting of a native exendin-4; an exendin-4 derivative in which the N-terminal amine group of exendin-4 is deleted; an exendin-4 derivative in which the N-terminal amine group of exendin-4 is substituted with a hydroxyl group; an exendin-4 derivative in which the N-terminal amine group of exendin-4 is modified with a dimethyl group; an exendin-4 derivative in which the N-terminal amine group of exendin-4 is substituted with a carboxyl group; an exendin-4 derivative in which the α-carbon of the 1^{st} amino acid of exendin-4, histidine, is deleted; an exendin-4 derivative in which the 12^{th} amino acid of exendin-4, lysine, is substituted with serine, and an exendin-4 derivative in which the 12^{th} amino acid of exendin-4, lysine, is substituted with arginine, but the GLP-1 receptor agonist is not particularly limited thereto.

In a specific embodiment, the present invention provides a long-acting conjugate for brain targeting which is characterized in that a physiologically active material is linked by a mutual binding to an immunoglobulin Fc region of a peptide for brain targeting, which is fused with the immunoglobulin Fc region.

As used herein, the term "F" refers to a material which includes an immunoglobulin constant region and an FcRn-binding region, and specifically, it corresponds to one moiety that constitutes the long-acting conjugate for brain targeting of the present invention. For example, F may be an immunoglobulin Fc region including an FcRn-binding region.

In another specific embodiment, the immunoglobulin Fc region of the peptide for brain targeting, which is fused with an immunoglobulin Fc region, may be a biocompatible material other than an immunoglobulin Fc region.

As used herein, the term "biocompatible material" refers to a material which can extend the duration of activity by increasing the *in vivo* half-life of a physiologically active material when the material is fused or covalently bound to a physiologically active material to form a conjugate. For example, since the top priority lies on the increase of half-life and bioavailability and maintenance of sustained activity, examples of those materials which can be fused or linked to a physiologically active material may be various biocompatible materials, *e.g*., polyethylene glycol, fatty acids, cholesterol, albumin and a fragment thereof, albumin binding materials, elastin, elastin's water-soluble precursor and polymers of several repeat units of a partial sequence of elastin protein, antibodies, antibody fragments, FcRn-binding materials, *in vivo* connective tissues, nucleotides, fibronectin, transferrin, polysaccharides, polymers, *etc.* may be included without limitation.

Since an immunoglobulin Fc region is a biodegradable polypeptide that can be metabolized *in vivo,* it is safe for use as a drug carrier. Additionally, the immunoglobulin Fc region has a relatively low molecular weight compared to the whole molecule of immunoglobulin, it is advantageous in terms of preparation, purification, and yield of a conjugate. In addition, as the Fab region, which exhibits high heterogeneity due to the difference in amino acid sequences from antibody to antibody, is removed, it is expected that the homogeneity of materials can be greatly increased and the risk of inducing blood antigenicity can also be lowered.

The peptide conjugate for brain targeting, which is fused with an immunoglobulin Fc region, is linked to the immunoglobulin Fc region by a peptide bond and is produced as a long-acting conjugate in host cells expressing the same. The expression host may be a microorganism such as *Escherichia coli* or a yeast cell, insect cell, animal cell, *etc.,* without limitation.

As used herein, the term "immunoglobulin constant region" may be a constitution that forms a moiety of a long-acting conjugate for brain targeting, and includes the heavy chain constant region 2 (CH2) and the heavy chain constant region 3 (CH3), excluding the heavy chain and the light chain variable regions and the light chain constant region (CL1) of the immunoglobulin. A hinge region may be included in the heavy chain constant regions.

The immunoglobulin constant region may be an immunoglobulin Fc region.

The immunoglobulin constant region of F may include one to four domains selected from the group consisting of CH1, CH2, CH3, and CH4 domains.

The immunoglobulin constant region of the present invention may be 1) a CH1 domain, a CH2 domain, a CH3 domain, and a CH4 domain, 2) a CH1 domain and a CH2 domain, 3) a CH1 domain and a CH3 domain, 4) a CH2 domain and a CH3 domain, and 5) a combination between one or two or more domains selected from a CH1 domain, a CH2 domain, a CH3 domain, and a CH4 domain and a hinge region (or part of a hinge region) of an immunoglobulin (*e.g*., a combination between a CH2 domain and a CH3 domain and a hinge region (or part of the hinge region)), and 6) a dimer between each domain of the heavy chain constant region and the light chain constant region. The hinge region of the present invention includes not only a native hinge region but also a part of all of the hinge regions known in the art.

Specifically, the immunoglobulin constant region of F may include a hinge region, a CH2 domain, and a CH3 domain, but is not particularly limited thereto.

Additionally, the immunoglobulin Fc region of the present invention includes not only the native amino acid sequence but also a sequence derivative (mutant) thereof. An amino acid sequence derivative refers to an amino acid sequence which has a difference in at least one amino acid residue due to deletion, insertion, non-conservative or conservative substitution, or a combination thereof. For example, the amino acid residues at positions 214 to 238, 297 to 299, 318 to 322, or 327 to 331, which are known to be important in the conjugation of an immunoglobulin Fc, may be used as suitable sites for modification. Additionally, various other kinds of derivatives are possible, including one that has a deletion of a region capable of forming a disulfide bond, or a deletion of some amino acid residues at the N-terminus of native Fc or an addition of a methionine residue at the N-terminus of native Fc. Further, to remove effector functions, a deletion may occur in a complement-binding site, such as a C1q-binding site and an antibody dependent cell mediated cytotoxicity (ADCC) site. Techniques of preparing such sequence derivatives of the immunoglobulin Fc region are disclosed in International Patent Publication Nos. WO 97/34631, WO 96/32478, *etc.*

Amino acid exchanges in proteins and peptides, which do not generally alter the activity of the proteins or peptides, are known in the art (H. Neurath, R. L. Hill, The Proteins, Academic Press, New York, 1979). The most commonly occurring exchanges are Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, the Fc region may be modified by phosphorylation, sulfation, acrylation, glycosylation, methylation, farnesylation, acetylation, amidation, *etc.*

The above-described immunoglobulin constant region derivatives may be those which show biological activity identical to that of the immunoglobulin constant region of the present invention and have improved structural stability against heat, pH, *etc.*

Additionally, the immunoglobulin Fc region may be obtained from native forms isolated *in vivo* from humans or animals such as cows, goats, pigs, mice, rabbits, hamsters, rats, guinea pigs, *etc.,* or may be recombinants or derivatives thereof, obtained from transformed animal cells or microorganisms. Herein, the Fc region may be obtained from a native immunoglobulin by isolating a whole immunoglobulin from a living human or animal body and treating the isolated immunoglobulin with protease. When the whole immunoglobulin is treated with papain, it is cleaved into Fab and Fc regions, whereas when the whole immunoglobulin is treated with pepsin, it is cleaved into pF'c and F(ab)₂ fragments. These fragments can be isolated using size exclusion chromatography, *etc.* In a more specific embodiment, the immunoglobulin Fc region may be a recombinant immunoglobulin Fc region obtained from a microorganism with regard to a human-derived Fc region.

Additionally, the immunoglobulin constant region may be in the form of native glycan, increased or decreased glycans compared to the native type, or in a deglycosylated form. The increase, decrease, or removal of the immunoglobulin Fc glycans may be achieved by conventional methods such as a chemical method, enzymatic method, and genetic engineering method using a microorganism. The immunoglobulin Fc region obtained by removal of glycans from the Fc region shows a significant decrease in binding affinity to the complement (C1q part) and a decrease or loss in antibody-dependent cytotoxicity or complement-dependent cytotoxicity, and thus it does not induce unnecessary immune responses *in vivo.* In this regard, an immunoglobulin Fc region in a deglycosylated or aglycosylated immunoglobulin Fc region may be a more suitable form to meet the original object of the present invention as a drug carrier.

As used herein, the term "deglycosylation" refers to enzymatically removing sugar moieties from an Fc region, and the term "aglycosylation" refers to an unglycosylated immunoglobulin constant region produced in prokaryotes, more specifically, *E. coli.* Meanwhile, the immunoglobulin Fc region may be derived from humans or animals including cows, goats, pigs, mice, rabbits, hamsters, rats, and guinea pigs, and preferably, it is derived from humans.

Additionally, the immunoglobulin (Ig) Fc region may be derived from IgG, IgA, IgD, IgE, IgM, or a combination or hybrid thereof. For example, it may be derived from IgG or IgM, which are among the most abundant proteins in human blood, and it may be derived from IgG, which is known to enhance the half-lives of ligand-binding proteins. Meanwhile, as used herein, the term "combination" means that polypeptides encoding single-chain immunoglobulin Fc regions of the same origin are linked to a single-chain polypeptide of a different origin to form a dimer or multimer. That is, a dimer or multimer may be prepared from two or more fragments selected from the group consisting of IgG Fc, IgA Fc, IgM Fc, IgD Fc, and IgE Fc fragments.

As used herein, the term "hybrid" means that sequences corresponding to two or more immunoglobulin Fc fragments of different origins are present in a single-chain of an immunoglobulin Fc region. In the present invention, various hybrid forms are possible. That is, the hybrid domain may be composed of one to four domains selected from the group consisting of CH1, CH2, CH3, and CH4 of IgG Fc, IgM Fc, IgA Fc, IgE Fc, and IgD Fc, and may further include a hinge region.

Meanwhile, IgG may also be divided into the IgG1, IgG2, IgG3, and IgG4 subclasses, and the present invention may include combinations or hybrids thereof, for example, the IgG2 and IgG4 subclasses, and specifically, the Fc region of IgG4 rarely having effector functions such as complement dependent cytotoxicity (CDC), but is not limited thereto. Additionally, in an embodiment, the immunoglobulin Fc region may be the IgG1 Fc region or IgG4 Fc region, but is not limited thereto.

As used herein, the term "Li and/or L₂" of Formula 1, which is a constituting element that forms a moiety of the long-acting conjugate for brain targeting, refers to a linker that links a peptide for brain targeting or physiologically active material to an immunoglobulin Fc region. The linker basically refers to a linking body that can link two fusion partners using a covalent bond, *etc.* In addition to linking fusion partners, a linker can also have a role of providing a gap of a certain size between the fusion partners or providing flexibility or rigidity, but the roles of a linker are not particularly limited thereto.

In a specific embodiment, one of L₁ and L₂ of Formula 1 may be a peptide linker and the other of L₁ and L₂ of Formula 1 may be a non-peptide linker. Alternatively, one of L₁ and L₂ of Formula 1 may be a peptide linker, the other of L₁ and L₂ of Formula 1 may be a non-peptide linker, and both Li and L₂ may be a peptide linker while both L₁ and L₂ may be a non-peptide linker, but L₁ and L₂ of Formula 1 are not particularly limited thereto.

In another specific embodiment, when any one or both of L₁ and L₂ of Formula 1 are a non-peptide linker, the non-peptide linker may be selected from the group consisting of polyethylene glycol, polypropylene glycol, an ethylene glycol-propylene glycol copolymer, polyoxyethylated polyol, polyvinyl alcohol, a polysaccharide, dextran, polyvinyl ethyl ether, a biodegradable polymer, a lipid polymer, chitins, hyaluronic acid, a fatty acid, a high molecular weight polymer, a low molecular weight compound, a nucleotide, and a combination thereof, but the non-peptide linker is not limited thereto.

In still another specific embodiment, when Li is a non-peptide linker and L₂ is a peptide linker, the peptide linker may contain 0 to 1,000 amino acid(s). In another specific embodiment, when Li is a non-peptide linker and L₂ is a peptide linker, and the peptide linker contains no amino acids, F and Y, which is a peptide for brain targeting, may be directly formed by fusion.

In still another specific embodiment, when any one or both of L₁ and L₂ are a peptide linker, X and F, or F and Y are linked to each other by L₁ and L₂ via a covalent chemical bond, non-covalent chemical bond, or a combination thereof; and Li and L₂ each contain 0 to 1,000 amino acid(s). When the peptide linker contains no amino acids, X and F, or F and Y may be linked by a peptide bond.

In still another specific embodiment, when any one or both of L₁ and L₂ are a peptide linker, and Li and L₂ each contain no amino acids, (i) X and F, or F and Y may be linked by a peptide bond; or (ii) X and F, or F and Y may be linked by a peptide bond.

As used herein, the term "peptide linker" includes a peptide conjugate connecting two fusion partners or a polymer of amino acids. Specifically, the peptide linker may contain 0 to 1,000 amino acid sequences, and more specifically, 0 to 900 amino acid sequences, 0 to 800 amino acid sequences, 0 to 700 amino acid sequences, 0 to 600 amino acid sequences, 0 to 500 amino acid sequences, 0 to 400 amino acid sequences, 0 to 300 amino acid sequences, 0 to 250 amino acid sequences, 0 to 200 amino acid sequences, 0 to 150 amino acid sequences, 0 to 100 amino acid sequences, 0 to 90 amino acid sequences, 0 to 80 amino acid sequences, 0 to 70 amino acid sequences, 0 to 60 amino acid sequences, 0 to 50 amino acid sequences, 0 to 40 amino acid sequences, 0 to 30 amino acid sequences, 0 to 25 amino acid sequences, 0 to 20 amino acid sequences, 0 to 15 amino acid sequences, or 0 to 10 amino acid sequences, but the number of amino acid sequences is not particularly limited thereto.

Additionally, examples of the peptide linker may include peptides which consist of an amino acid sequence, which is in a form where GGGGS (SEQ ID NO: 59) motif, GS motif, GGGS (SEQ ID NO: 60) motif, or GGSG (SEQ ID NO: 61) motif is repeated, and these motifs may be repeated 1 to 10 times, but the motifs are not particularly limited thereto.

Additionally, when L₂ is a peptide linker, L₂ is (GS)m, (GGS)m, (GGGS)m, or (GGGGS)m, and m may be 1 to 10, but is not limited thereto.

In still another specific embodiment, when any one of L₁ and L₂ is a peptide linker and the other of the two is a non-peptide linker, the peptide linker is a linker containing 0 to 1,000 amino acid(s) and the non-peptide linker is polyethylene glycol, but the linkers are not limited thereto.

As used herein, the term "non-peptide linker" refers to a biocompatible linker in which two or more repeating units are linked, and the repeating units are linked to each other through any covalent bond other than a peptide bond.

In still another specific embodiment, when the non-peptide linker and X and Y are each linked by their respective functional groups, the functional group of the non-peptide linker may be selected from the group consisting of an aldehyde group, a maleimide group, and a succinimide derivative. Li may be a non-peptide polymer having a functional group at both ends, and the functional group at both ends may be an amine-reactive group or thiol-reactive group, and the functional group at both ends may be the same kind as each other or different kinds from each other, but is not limited thereto.

The non-peptide polymer, which is a non-peptide linker to be used in the present invention, may be selected from the group consisting of polyethylene glycol, polypropylene glycol, an ethylene glycol-propylene glycol copolymer, polyoxyethylated polyol, polyvinyl alcohol, a polysaccharide, dextran, polyvinyl ethyl ether, biodegradable polymers such as polylactic acid (PLA) and polylactic-glycolic acid (PLGA), a lipid polymer, chitins, hyaluronic acid, an oligonucleotide, and a combination thereof, and for example polyethylene glycol, but the non-peptide polymer is not limited thereto. Derivatives thereof already known in the art and derivatives which can be easily prepared at the technical level of the art are also included within the scope of the present invention.

With respect to the non-peptide polymer to be used in the present invention, any polymer having resistance to protein degradation *in vivo* may be used without limitation. The molecular weight of the non-peptide polymer may be in the range of exceeding 0 kDa to about 100 kDa, *e.g.,* about 0.5 kDa to about 100 kDa, about 1 kDa to about 100 kDa, or about 1 kDa to about 20 kDa, but the molecular weight is not limited thereto.

As used herein, the term "about" refers to a range which includes all of ±0.5, ±0.4, ±0.3, ±0.2, ±0.1, *etc.,* and includes all of the values that are equivalent or similar to those following the values, but the range is not limited thereto.

Additionally, with respect to the non-peptide linker, not only a single kind of a polymer but also a combination of different kinds of polymers may be used.

Meanwhile, the non-peptide polymer, which is a non-peptide linker used in the present invention, may have a functional group to be linked to the functional group of F and X or Y, so that an immunoglobulin Fc region and a physiologically active material can form a covalent bond between them.

Specifically, the non-peptide linker may have at least two terminal functional groups, specifically 2 or 3 terminal functional groups, and more specifically two terminal functional groups.

The functional group may be selected from the group consisting of an aldehyde group, a maleimide group, and a succinimide derivative, but the functional group is not limited thereto.

In the above, examples of the aldehyde group may include a propionaldehyde group or butyraldehyde group, but the aldehyde group is not limited thereto.

In the above, examples of the succinimide derivative may include succinimidyl valerate, succinimidyl methylbutanoate, succinimidyl methylpropionate, succinimidyl butanoate, succinimidyl propionate, *N*-hydroxysuccinimide, hydroxy succinimidyl, succinimidyl carboxymethyl, or succinimidyl carbonate, but the succinimide derivative is not limited thereto.

The functional groups of both ends of the non-peptide linker may be the same as or different from each other.

For example, the functional group at one end of the non-peptide linker may be a maleimide group and the functional group at the other end may be an aldehyde group (*e.g*., propionaldehyde group or butyraldehyde group). When polyethylene glycol having a hydroxyl functional group at both ends is used as the non-peptide polymer, it is possible to activate the hydroxyl group by a known chemical reaction in the various reactive groups or to prepare the conjugate of the present invention using polyethylene glycol having a commercially available modified reactive group.

Additionally, the non-peptide polymer may be a homo-functional non-peptide polymer in which all of two ends or three ends of the non-peptide linker are aldehyde groups.

For example, the non-peptide linker may be a non-peptide polymer having a propionaldehyde group at both ends, and specifically polyethylene glycol having a propionaldehyde group at both ends, but is not particularly limited thereto.

When the non-peptide linker has a functional group of a reactive aldehyde group at both ends, it is effective in minimizing non-specific reactions and for linking to a physiologically active polypeptide and an immunoglobulin at both ends, respectively. The final product formed by reductive amination by an aldehyde bond is significantly more stable compared to those by an amide bond. The aldehyde functional group reacts selectively at the N-terminus at low pH and can form a covalent bond with a lysine residue at high pH *(e.g.,* pH 9.0).

The conjugate for brain targeting of the present invention may be a long-acting conjugate, in which physiologically active materials different from one another are contained, prepared in such a manner that the peptide conjugate for brain targeting, which is fused to an immunoglobulin Fc region, is encoded and then chemically bound, and then a different physiologically active material is linked to the chemically bound immunoglobulin Fc region.

The chemically bound immunoglobulin Fc region conjugate includes two immunoglobulin Fc regions (a dimeric form of an Fc region in which monomeric Fc regions are linked), and the physiologically active material which binds to each of the two immunoglobulin Fc regions may be linked to a single site of the immunoglobulin Fc region (a monomer Fc region) or both sites of the two immunoglobulin Fc regions (each dimeric form of Fc region).

The chemical binding may be a covalent bond, more specifically a disulfide bond, and even more specifically a disulfide bond formed in a hinge region of two immunoglobulin Fc regions, but the binding is not particularly limited thereto.

More specifically, the long-acting conjugate for brain targeting may be in a form in which a dimer is formed by a chemical bond between (i) a first immunoglobulin Fc region, in which a peptide for brain targeting is linked to the C-terminal region of one molecule of an immunoglobulin Fc region through the above-described peptide linker (*e.g.,* a peptide bond) and (ii) a second immunoglobulin Fc region, in which a peptide for brain targeting is linked to the C-terminal region of one molecule of an immunoglobulin Fc region through the above-described peptide linker (*e.g.,* a peptide bond), and one molecule of a physiologically active material may be linked to the N-terminal region of one of the two immunoglobulin Fc region molecules; or may be in a form in which a physiologically active material is linked to each of the N-terminal regions of both of the immunoglobulin Fc region molecules, but the forms are not particularly limited thereto. The first and second immunoglobulin Fc regions, which are in the form of a fusion protein, may be fused in-frame by a peptide bond between the immunoglobulin Fc region and the peptide for brain targeting, and the immunoglobulin Fc region may include a hinge region, CH2 domain, and CH3 domain, but the immunoglobulin Fc region is not particularly limited thereto.

The chemical bond between the first and second immunoglobulin Fc regions may specifically be a covalent bond, more specifically a disulfide bond, and more specifically, a disulfide bond formed in the hinge region of the two immunoglobulin Fc regions, but the chemical bond is not particularly limited thereto.

Still another aspect of the present invention provides a method for preparing the long-acting conjugate for brain targeting of Formula 1.

In a specific embodiment, the method for preparing the long-acting conjugate for brain targeting of Formula 1 may include: i) preparing: (a) X-L₁-F, wherein X, which is a physiologically active material, Li, which is a peptide or non-peptide linker, and F comprising an immunoglobulin Fc region, are linked; and (b) L₂-Y, wherein Y, which is a peptide for brain targeting, and L₂, which is a peptide or non-peptide linker, are linked; and (ii) linking (a) X-L₁-F and (b) L₂-Y. In particular, in a specific embodiment, Li of (a) may be a peptide linker and L₂ of (b) may be a non-peptide linker.

In another specific embodiment, the method for preparing the long-acting conjugate for brain targeting of Formula 1 may include: (i) preparing: (a) X-Li, wherein X, which is a physiologically active material, and Li, which is a peptide or non-peptide linker, are linked; and (b) F-L₂-Y, wherein Y, which is a peptide for brain targeting, L₂, which is a peptide or non-peptide linker, and F comprising an immunoglobulin Fc region, are linked; and (ii) linking (a) X-Li and (b) F-L₂-Y. In particular, in a specific embodiment, Li of (a) may be a non-peptide linker and L₂ of (b) may be a peptide linker.

In still another specific embodiment, the method for preparing the long-acting conjugate for brain targeting of Formula 1 may include: (i) preparing: (a) X-Li, wherein X, which is a physiologically active material, and Li, which is a peptide or non-peptide linker, are linked; and (b) F-L₂-Y, wherein Y, which is a peptide for brain targeting, L₂, which is a peptide or non-peptide linker, and F comprising an immunoglobulin Fc region, are linked; and (ii) linking (a) X-Li and (b) F-L₂-Y. In particular, in a specific embodiment, Li may be a non-peptide linker and L₂ may be a peptide linker. L₂ may contain no amino acids, in which case the long-acting conjugate may be directly fused in an F-Y form.

In still another specific embodiment, the non-peptide linker and X and Y may be linked by their respective functional groups, in which the functional group of the non-peptide linker may be selected from the group consisting of an aldehyde group, maleimide group, and succinimide derivative.

The long-acting conjugate containing a peptide for brain targeting and a physiologically active material of the present invention can pass through the blood-brain barrier, maintain physiological activity, and thereby exhibit improved durability and stability.

Still another aspect of the present invention provides the peptide for brain targeting, a separated polynucleotide which encodes the peptide for brain targeting, an expression vector containing the polynucleotide, and a transformant containing the expression vector.

Meanwhile, the peptide for brain targeting may include all of those in the form of the peptide itself, a salt thereof (*e.g.,* a pharmaceutically acceptable salt of the peptide), or a solvate thereof.

Additionally, the peptide for brain targeting may be in any pharmaceutically acceptable form.

The kind of the salt is not particularly limited. However, the salt is preferably one that is safe and effective to a subject, *e.g.,* a mammal, but is not particularly limited thereto.

The term "pharmaceutically acceptable" refers to a material which can be effectively used for the intended use within the scope of pharmaco-medical decisions without inducing excessive toxicity, irritation, allergic responses, *etc.*

As used herein, the term "pharmaceutically acceptable salt" refers to a salt derived from pharmaceutically acceptable inorganic salts, organic salts, or bases. Examples of the suitable salts may include hydrochloric acid, bromic acid, sulfuric acid, nitric acid, perchloric acid, fumaric acid, maleic acid, phosphoric acid, glycolic acid, lactic acid, salicylic acid, succinic acid, toluene-*p*-sulfonic acid, tartaric acid, acetic acid, citric acid, methanesulfonic acid, formic acid, benzoic acid, malonic acid, naphthalene-2-sulfonic acid, benzenesulfonic acid, *etc.* Examples of the salts derived from suitable bases may include alkali metals such as sodium, potassium, *etc.;* alkali earth metals such as magnesium; ammonium, *etc.*

As used herein, the term "solvate" refers to a complex formed between the peptide according to the present invention or a salt thereof and a solvent molecule.

The separated polynucleotide encoding the peptide for brain targeting includes polynucleotide sequences which have a sequence homology to the corresponding sequence of 75% or higher, specifically 85% or higher, more specifically 90% or higher, and even more specifically 95%, are included within the scope of the present invention.

The recombinant vector according to the present invention may typically be constructed as a vector or vector for expression for cloning, and may be constructed using prokaryotic or eukaryotic cells as a host cell.

As used herein, the term "vector" refers to a recombinant vector capable of expressing a target protein in an appropriate host cell, which is a gene construct including regulatory factors operably linked to enable the expression of a gene insert.

The long-acting conjugate for brain targeting of the present invention can be obtained by performing transformation or transfection of the recombinant vector into a host cell.

In the present invention, the polynucleotide encoding the peptide for brain targeting can be operably linked to a promoter.

The method for transforming the recombinant vector containing a polynucleotide according to the present invention is not limited to the above embodiments, and the transforming or transfection methods conventionally used in the art can be used without limitation.

The transformant of the present invention can be obtained by introducing a recombinant vector containing the polynucleotide according to the present invention into a host cell.

An appropriate host to be used in the present invention may not be particularly limited as long as it can express the polynucleotide of the present invention. Examples of the appropriate host may include bacteria belonging to the genus *Escherichia* such as *E. coli*; bacteria belonging to the genus *Bacillus* such as *Bacillus subtilis;* bacteria belonging to the genus *Pseudomonas* such as *Pseudomonas putida;* yeasts such as *Pichia pastoris, Saccharomyces cerevisiae,* and *Schizosaccharomyces pombe;* insect cells such as *Spodoptera frugiperda* (Sf9), and animal cells such as CHO, COS, and BSC.

Hereinafter, the present invention will be described in more detail with reference to the following Examples. However, these Examples are for illustrative purposes only, and the invention is not intended to be limited by these Examples.

### Example 1: Preparation of fusion proteins in which immunoglobulin Fc region and peptide for brain targeting are conjugated

For the preparation of a long-acting conjugate containing a peptide for brain targeting and a physiologically active polypeptide, in which a biocompatible material, which includes an immunoglobulin constant region and an FcRn-binding region, a peptide for brain targeting, and a physiologically active material are linked by a peptide linker, a fusion protein to which an immunoglobulin Fc region and a peptide for brain targeting are linked was prepared.

In this regard, five kinds of peptides were selected as the peptide for brain targeting (Table 1).

**[Table 1]**

| Peptides for Brain Targeting | | Sequence | SEQ ID NO |
|---|---|---|---|
| HAIYPRH (BTP1) | DNA | CATGCAATTTATCCGCGTCATTGA | 1 |
| | Protein | HAIYPRH | 2 |
| THR (BTP2) | DNA | ACCCATCGTCCGCCGATGTGGAGCCCGGTTT GGCCGTGA | 3 |
| | Protein | THRPPMWSP VWP | 4 |
| Angiopep-2 (BTP3) | DNA | ACCTTTTTTTATGGTGGCAGCCGTGGTAAACG CAATAACTTTAAAACCGAAGAATATTAA | 5 |
| | Protein | TFFYGGSRG KRNNFKTEEY | 6 |
| ApoB (BTP4) | DNA | AGCGTTATTGATGCACTGCAGTATAAACTGG AAGGTACCACACGTCTGACCCGCAAACGTGG TCTGAAACTGGCCACCGCACTGTCACTGAGC AATAAATTTGTTGAAGGTAGCTAA | 7 |
| | Protein | SVIDALQYK LEGTTRLTRK RGLKLATALS LSNKFVEGS | 8 |
| ApoE (BTP5) | DNA | CTGCGTAAACTGCGCAAACGTCTGTTACTGC GTAAACTGCGCAAACGTCTGTTATAA | 9 |
| | Protein | LRKLRKRLL LRKLRKRLL | 10 |

As the biocompatible material capable of increasing the half-life of a physiologically active polypeptide linked thereto, IgG4 Fc regions including a hinge region were selected, and the IgG4 Fc regions including a hinge region and the peptides for brain targeting were fused at a gene level, and each of the fusion products was inserted into respective expression vectors.

Fusion proteins were synthesized by linking the IgG4 Fc regions, which include a hinge region, and the peptides for brain targeting by a peptide linker or by a method of direct fusion, and specifically, by linking the IgG4 Fc regions, which include a hinge region, and the peptides for brain targeting by a peptide bond (synthesized by Bioneer Corporation, Korea) (Table 2). As can be seen in the sequences of Table 2 below, the fusion proteins containing the IgG4 Fc region, which includes a hinge region, and a peptide for brain targeting were prepared by linking the N-terminus of a peptide for brain targeting to the C-terminus of an IgG4 Fc region using a linker or by a method of direct fusion.

**[Table 2]**

| | | | Sequence | SEQ ID NO |
|---|---|---|---|---|
| Fc-BTP1 (HAIYPRH) | DNA | IgG4 Hinge | ATGCCATCATGCCCA | 11 |
| | | IgG4 CH2 | GCACCTGAGTTCCTGGGGGGACCATCAGTCT TCCTGTTCCCCCCAAAACCCAAGGACACCCT | 12 |
| | | | CATGATCTCCCGGACCCCTGAGGTCACATGC GTGGTGGTGGACGTGAGCCAGGAAGACCCT GAGGTCCAGTTCAACTGGTACGTGGACGGCG TGGAGGTGCATAATGCCAAGACAAAGCCGC GGGAGGAGCAGTTCAACAGCACGTACCGTG TGGTCAGCGTCCTCACCGTCCTGCACCAGGA CTGGCTGAATGGCAAGGAGTACAAGTGCAA GGTCTCCAACAAAGGCCTCCCATCCTCCATC GAGAAAACCATCTCCAAAGCCAAA | |
| | | IgG4 CH3 | GGGCAGCCCCGAGAACCACAGGTGTACACC CTGCCCCCATCCCAGGAGGAGATGACCAAG AACCAGGTCAGCCTGACCTGCCTGGTCAAAG GCTTCTATCCCAGCGACATCGCCGTGGAGTG GGAGAGCAATGGGCAGCCGGAGAACAACTA CAAGACCACGCCTCCCGTGCTGGACTCCGAC GGCTCCTTCTTCCTCTACAGCAGGCTAACCG TGGACAAGAGCAGGTGGCAGGAGGGGAACG TCTTCTCATGCTCCGTGATGCATGAGGCTCTG CACAACCACTACACGCAGAAGAGCCTCTCCC TGTCTCTGGGTAAA | 13 |
| | | HAIYP RH | CATGCAATTTATCCGCGTCATTGA | 1 |
| | Protein | IgG4 Hinge | PSCP | 14 |
| | | IgG4 CH2 | APEFLGGPSVFLFPPKPKDTLMISRTPEVTCVV VDVSQEDPEVQFNWYVDGVEVHNAKTKPREE QFNSTYRVVSVLTVLHQDWLNGKEYKCKVSN KGLPSSIEKTISKAK | 15 |
| | | IgG4 CH3 | GQPREPQVYTLPPSQEEMTKNQVSLTCLVKGF YPSDIAVEWESNGQPENNYKTTPPVLDSDGSFF | 16 |
| | | | LYSRLTVDKSRWQEGNVFSCSVMHEALHNHY TQKSLSLSLGK | |
| | | HAIYP RH | HAIYPRH | 2 |
| Fc-BTP2 (THR) | DNA | IgG4 Hinge | ATGCCATCATGCCCA | 11 |
| | | IgG4 CH2 | GCACCTGAGTTCCTGGGGGGACCATCAGTCT TCCTGTTCCCCCCAAAACCCAAGGACACCCT CATGATCTCCCGGACCCCTGAGGTCACATGC GTGGTGGTGGACGTGAGCCAGGAAGACCCT GAGGTCCAGTTCAACTGGTACGTGGACGGCG TGGAGGTGCATAATGCCAAGACAAAGCCGC GGGAGGAGCAGTTCAACAGCACGTACCGTG TGGTCAGCGTCCTCACCGTCCTGCACCAGGA CTGGCTGAATGGCAAGGAGTACAAGTGCAA GGTCTCCAACAAAGGCCTCCCATCCTCCATC GAGAAAACCATCTCCAAAGCCAAA | 12 |
| | | IgG4 CH3 | GGGCAGCCCCGAGAACCACAGGTGTACACC CTGCCCCCATCCCAGGAGGAGATGACCAAG AACCAGGTCAGCCTGACCTGCCTGGTCAAAG GCTTCTATCCCAGCGACATCGCCGTGGAGTG GGAGAGCAATGGGCAGCCGGAGAACAACTA CAAGACCACGCCTCCCGTGCTGGACTCCGAC GGCTCCTTCTTCCTCTACAGCAGGCTAACCG TGGACAAGAGCAGGTGGCAGGAGGGGAACG TCTTCTCATGCTCCGTGATGCATGAGGCTCTG CACAACCACTACACGCAGAAGAGCCTCTCCC TGTCTCTGGGTAAA | 13 |
| | | THR | ACCCATCGTCCGCCGATGTGGAGCCCGGTTT GGCCGTGA | 3 |
| | Protein | IgG4 Hinge | PSCP | 14 |
| | | IgG4 CH2 | APEFLGGPSVFLFPPKPKDTLMISRTPEVTCVV VDVSQEDPEVQFNWYVDGVEVHNAKTKPREE QFNSTYRVVSVLTVLHQDWLNGKEYKCKVSN KGLPSSIEKTISKAK | 15 |
| | | IgG4 CH3 | GQPREPQVYTLPPSQEEMTKNQVSLTCLVKGF YPSDIAVEWESNGQPENNYKTTPPVLDSDGSFF LYSRLTVDKSRWQEGNVFSCSVMHEALHNHY TQKSLSLSLGK | 16 |
| | | THR | THRPPMWSP VWP | 4 |
| Fc-BTP3 (Angiopep-2) | DNA | IgG4 Hinge | ATGCCATCATGCCCA | 11 |
| | | IgG4 CH2 | GCACCTGAGTTCCTGGGGGGACCATCAGTCT TCCTGTTCCCCCCAAAACCCAAGGACACCCT CATGATCTCCCGGACCCCTGAGGTCACATGC GTGGTGGTGGACGTGAGCCAGGAAGACCCT GAGGTCCAGTTCAACTGGTACGTGGACGGCG TGGAGGTGCATAATGCCAAGACAAAGCCGC GGGAGGAGCAGTTCAACAGCACGTACCGTG TGGTCAGCGTCCTCACCGTCCTGCACCAGGA CTGGCTGAATGGCAAGGAGTACAAGTGCAA GGTCTCCAACAAAGGCCTCCCATCCTCCATC GAGAAAACCATCTCCAAAGCCAAA | 12 |
| | | IgG4 CH3 | GGGCAGCCCCGAGAACCACAGGTGTACACC CTGCCCCCATCCCAGGAGGAGATGACCAAG AACCAGGTCAGCCTGACCTGCCTGGTCAAAG GCTTCTATCCCAGCGACATCGCCGTGGAGTG GGAGAGCAATGGGCAGCCGGAGAACAACTA | 13 |
| | | | CAAGACCACGCCTCCCGTGCTGGACTCCGAC GGCTCCTTCTTCCTCTACAGCAGGCTAACCG TGGACAAGAGCAGGTGGCAGGAGGGGAACG TCTTCTCATGCTCCGTGATGCATGAGGCTCTG CACAACCACTACACGCAGAAGAGCCTCTCCC TGTCTCTGGGTAAA | |
| | | Angiop ep-2 | ACCTTTTTTTATGGTGGCAGCCGTGGTAAAC GCAATAACTTTAAAACCGAAGAATATTAA | 5 |
| | Protein | IgG4 Hinge | PSCP | 14 |
| | | IgG4 CH2 | APEFLGGPSVFLFPPKPKDTLMISRTPEVTCVV VDVSQEDPEVQFNWYVDGVEVHNAKTKPREE QFNSTYRVVSVLTVLHQDWLNGKEYKCKVSN KGLPSSIEKTISKAK | 15 |
| | | IgG4 CH3 | GQPREPQVYTLPPSQEEMTKNQVSLTCLVKGF YPSDIAVEWESNGQPENNYKTTPPVLDSDGSFF LYSRLTVDKSRWQEGNVFSCSVMHEALHNHY TQKSLSLSLGK | 16 |
| | | Angiop ep-2 | TFFYGGSRG KRNNFKTEEY | 6 |
| Fc-BTP4 (ApoB) | DNA | IgG4 Hinge | ATGCCATCATGCCCA | 11 |
| | | IgG4 CH2 | GCACCTGAGTTCCTGGGGGGACCATCAGTCT TCCTGTTCCCCCCAAAACCCAAGGACACCCT CATGATCTCCCGGACCCCTGAGGTCACATGC GTGGTGGTGGACGTGAGCCAGGAAGACCCT GAGGTCCAGTTCAACTGGTACGTGGACGGCG TGGAGGTGCATAATGCCAAGACAAAGCCGC GGGAGGAGCAGTTCAACAGCACGTACCGTG TGGTCAGCGTCCTCACCGTCCTGCACCAGGA | 12 |
| | | | CTGGCTGAATGGCAAGGAGTACAAGTGCAA GGTCTCCAACAAAGGCCTCCCATCCTCCATC GAGAAAACCATCTCCAAAGCCAAA | |
| | | IgG4 CH3 | GGGCAGCCCCGAGAACCACAGGTGTACACC CTGCCCCCATCCCAGGAGGAGATGACCAAG AACCAGGTCAGCCTGACCTGCCTGGTCAAAG GCTTCTATCCCAGCGACATCGCCGTGGAGTG GGAGAGCAATGGGCAGCCGGAGAACAACTA CAAGACCACGCCTCCCGTGCTGGACTCCGAC GGCTCCTTCTTCCTCTACAGCAGGCTAACCG TGGACAAGAGCAGGTGGCAGGAGGGGAACG TCTTCTCATGCTCCGTGATGCATGAGGCTCTG CACAACCACTACACGCAGAAGAGCCTCTCCC TGTCTCTGGGTAAA | 13 |
| | | ApoB | AGCGTTATTGATGCACTGCAGTATAAACTGG AAGGTACCACACGTCTGACCCGCAAACGTGG TCTGAAACTGGCCACCGCACTGTCACTGAGC AATAAATTTGTTGAAGGTAGCTAA | 7 |
| | Protein | IgG4 Hinge | PSCP | 14 |
| | | IgG4 CH2 | APEFLGGPSVFLFPPKPKDTLMISRTPEVTCVV VDVSQEDPEVQFNWYVDGVEVHNAKTKPREE QFNSTYRVVSVLTVLHQDWLNGKEYKCKVSN KGLPSSIEKTISKAK | 15 |
| | | IgG4 CH3 | GQPREPQVYTLPPSQEEMTKNQVSLTCLVKGF YPSDIAVEWESNGQPENNYKTTPPVLDSDGSFF LYSRLTVDKSRWQEGNVFSCSVMHEALHNHY TQKSLSLSLGK | 16 |
| | | ApoB | SVIDALQYK LEGTTRLTRK RGLKLATALS LSNKFVEGS | 8 |
| Fc-BTP5 (ApoE) | DNA | IgG4 Hinge | ATGCCATCATGCCCA | 11 |
| | | IgG4 CH2 | GCACCTGAGTTCCTGGGGGGACCATCAGTCT TCCTGTTCCCCCCAAAACCCAAGGACACCCT CATGATCTCCCGGACCCCTGAGGTCACATGC GTGGTGGTGGACGTGAGCCAGGAAGACCCT GAGGTCCAGTTCAACTGGTACGTGGACGGCG TGGAGGTGCATAATGCCAAGACAAAGCCGC GGGAGGAGCAGTTCAACAGCACGTACCGTG TGGTCAGCGTCCTCACCGTCCTGCACCAGGA CTGGCTGAATGGCAAGGAGTACAAGTGCAA GGTCTCCAACAAAGGCCTCCCATCCTCCATC GAGAAAACCATCTCCAAAGCCAAA | 12 |
| | | IgG4 CH3 | GGGCAGCCCCGAGAACCACAGGTGTACACC CTGCCCCCATCCCAGGAGGAGATGACCAAG AACCAGGTCAGCCTGACCTGCCTGGTCAAAG GCTTCTATCCCAGCGACATCGCCGTGGAGTG GGAGAGCAATGGGCAGCCGGAGAACAACTA CAAGACCACGCCTCCCGTGCTGGACTCCGAC GGCTCCTTCTTCCTCTACAGCAGGCTAACCG TGGACAAGAGCAGGTGGCAGGAGGGGAACG TCTTCTCATGCTCCGTGATGCATGAGGCTCTG CACAACCACTACACGCAGAAGAGCCTCTCCC TGTCTCTGGGTAAA | 13 |
| | | ApoE | CTGCGTAAACTGCGCAAACGTCTGTTACTGC GTAAACTGCGCAAACGTCTGTTATAA | 9 |
| | Protein | IgG4 Hinge | PSCP | 14 |
| | | IgG4 | APEFLGGPSVFLFPPKPKDTLMISRTPEVTCVV | 15 |
| | | CH2 | VDVSQEDPEVQFNWYVDGVEVHNAKTKPREE QFNSTYRVVSVLTVLHQDWLNGKEYKCKVSN KGLPSSIEKTISKAK | |
| | | IgG4 CH3 | GQPREPQVYTLPPSQEEMTKNQVSLTCLVKGF YPSDIAVEWESNGQPENNYKTTPPVLDSDGSFF LYSRLTVDKSRWQEGNVFSCSVMHEALHNHY TQKSLSLSLGK | 16 |
| | | ApoE | LRKLRKRLL LRKLRKRLL | 10 |
| Fc-BTP6 (HAIYPRH) Linker 15a.a | DNA | IgG4 Hinge | ATGCCATCATGCCCA | 11 |
| | | IgG4 CH2 | GCACCTGAGTTCCTGGGGGGACCATCAGTCT TCCTGTTCCCCCCAAAACCCAAGGACACCCT CATGATCTCCCGGACCCCTGAGGTCACATGC GTGGTGGTGGACGTGAGCCAGGAAGACCCT GAGGTCCAGTTCAACTGGTACGTGGACGGCG TGGAGGTGCATAATGCCAAGACAAAGCCGC GGGAGGAGCAGTTCAACAGCACGTACCGTG TGGTCAGCGTCCTCACCGTCCTGCACCAGGA CTGGCTGAATGGCAAGGAGTACAAGTGCAA GGTCTCCAACAAAGGCCTCCCATCCTCCATC GAGAAAACCATCTCCAAAGCCAAA | 12 |
| | | IgG4 CH3 | GGGCAGCCCCGAGAACCACAGGTGTACACC CTGCCCCCATCCCAGGAGGAGATGACCAAG AACCAGGTCAGCCTGACCTGCCTGGTCAAAG GCTTCTATCCCAGCGACATCGCCGTGGAGTG GGAGAGCAATGGGCAGCCGGAGAACAACTA CAAGACCACGCCTCCCGTGCTGGACTCCGAC GGCTCCTTCTTCCTCTACAGCAGGCTAACCG TGGACAAGAGCAGGTGGCAGGAGGGGAACG | 13 |
| | | | TCTTCTCATGCTCCGTGATGCATGAGGCTCTG CACAACCACTACACGCAGAAGAGCCTCTCCC TGTCTCTGGGTAAA | |
| | | Linker | GGTGGAGGCGGTTCAGGCGGAGGTGGCTCT GGCGGTGGCGGATCG | 17 |
| | | HAIYP RH | CATGCAATTTATCCGCGTCATTGA | 1 |
| | Protein | IgG4 Hinge | PSCP | 14 |
| | | IgG4 CH2 | APEFLGGPSVFLFPPKPKDTLMISRTPEVTCVV VDVSQEDPEVQFNWYVDGVEVHNAKTKPREE QFNSTYRVVSVLTVLHQDWLNGKEYKCKVSN KGLPSSIEKTISKAK | 15 |
| | | IgG4 CH3 | GQPREPQVYTLPPSQEEMTKNQVSLTCLVKGF YPSDIAVEWESNGQPENNYKTTPPVLDSDGSFF LYSRLTVDKSRWQEGNVFSCSVMHEALHNHY TQKSLSLSLGK | 16 |
| | | Linker | GGGGSGGGGSGGGGS | 18 |
| | | HAIYP RH | HAIYPRH | 2 |
| Fc-BTP7 (THR) Linker 15a.a | DNA | IgG4 Hinge | ATGCCATCATGCCCA | 11 |
| | | IgG4 CH2 | GCACCTGAGTTCCTGGGGGGACCATCAGTCT TCCTGTTCCCCCCAAAACCCAAGGACACCCT CATGATCTCCCGGACCCCTGAGGTCACATGC GTGGTGGTGGACGTGAGCCAGGAAGACCCT GAGGTCCAGTTCAACTGGTACGTGGACGGCG TGGAGGTGCATAATGCCAAGACAAAGCCGC GGGAGGAGCAGTTCAACAGCACGTACCGTG | 12 |
| | | | TGGTCAGCGTCCTCACCGTCCTGCACCAGGA CTGGCTGAATGGCAAGGAGTACAAGTGCAA GGTCTCCAACAAAGGCCTCCCATCCTCCATC GAGAAAACCATCTCCAAAGCCAAA | |
| | | IgG4 CH3 | GGGCAGCCCCGAGAACCACAGGTGTACACC CTGCCCCCATCCCAGGAGGAGATGACCAAG AACCAGGTCAGCCTGACCTGCCTGGTCAAAG GCTTCTATCCCAGCGACATCGCCGTGGAGTG GGAGAGCAATGGGCAGCCGGAGAACAACTA CAAGACCACGCCTCCCGTGCTGGACTCCGAC GGCTCCTTCTTCCTCTACAGCAGGCTAACCG TGGACAAGAGCAGGTGGCAGGAGGGGAACG TCTTCTCATGCTCCGTGATGCATGAGGCTCTG CACAACCACTACACGCAGAAGAGCCTCTCCC TGTCTCTGGGTAAA | 13 |
| | | Linker | GGTGGAGGCGGTTCAGGCGGAGGTGGCTCT GGCGGTGGCGGATCG | 17 |
| | | THR | ACCCATCGTCCGCCGATGTGGAGCCCGGTTT GGCCGTGA | 3 |
| | Protein | IgG4 Hinge | PSCP | 14 |
| | | IgG4 CH2 | APEFLGGPSVFLFPPKPKDTLMISRTPEVTCVV VDVSQEDPEVQFNWYVDGVEVHNAKTKPREE QFNSTYRVVSVLTVLHQDWLNGKEYKCKVSN KGLPSSIEKTISKAK | 15 |
| | | IgG4 CH3 | GQPREPQVYTLPPSQEEMTKNQVSLTCLVKGF YPSDIAVEWESNGQPENNYKTTPPVLDSDGSFF LYSRLTVDKSRWQEGNVFSCSVMHEALHNHY TQKSLSLSLGK | 16 |
| | | Linker | GGGGSGGGGSGGGGS | 18 |
| | | THR | THRPPMWSP VWP | 4 |
| Fc-BTP8 (Angiopep-2) Linker 15a.a | DNA | IgG4 Hinge | ATGCCATCATGCCCA | 11 |
| | | IgG4 CH2 | GCACCTGAGTTCCTGGGGGGACCATCAGTCT TCCTGTTCCCCCCAAAACCCAAGGACACCCT CATGATCTCCCGGACCCCTGAGGTCACATGC GTGGTGGTGGACGTGAGCCAGGAAGACCCT GAGGTCCAGTTCAACTGGTACGTGGACGGCG TGGAGGTGCATAATGCCAAGACAAAGCCGC GGGAGGAGCAGTTCAACAGCACGTACCGTG TGGTCAGCGTCCTCACCGTCCTGCACCAGGA CTGGCTGAATGGCAAGGAGTACAAGTGCAA GGTCTCCAACAAAGGCCTCCCATCCTCCATC GAGAAAACCATCTCCAAAGCCAAA | 12 |
| | | IgG4 CH3 | GGGCAGCCCCGAGAACCACAGGTGTACACC CTGCCCCCATCCCAGGAGGAGATGACCAAG AACCAGGTCAGCCTGACCTGCCTGGTCAAAG GCTTCTATCCCAGCGACATCGCCGTGGAGTG GGAGAGCAATGGGCAGCCGGAGAACAACTA CAAGACCACGCCTCCCGTGCTGGACTCCGAC GGCTCCTTCTTCCTCTACAGCAGGCTAACCG TGGACAAGAGCAGGTGGCAGGAGGGGAACG TCTTCTCATGCTCCGTGATGCATGAGGCTCTG CACAACCACTACACGCAGAAGAGCCTCTCCC TGTCTCTGGGTAAA | 13 |
| | | Linker | GGTGGAGGCGGTTCAGGCGGAGGTGGCTCT GGCGGTGGCGGATCG | 17 |
| | | Angiop | ACCTTTTTTTATGGTGGCAGCCGTGGTAAAC | 5 |
| | | ep-2 | GCAATAACTTTAAAACCGAAGAATATTAA | |
| | Protein | IgG4 Hinge | PSCP | 14 |
| | | IgG4 CH2 | APEFLGGPSVFLFPPKPKDTLMISRTPEVTCVV VDVSQEDPEVQFNWYVDGVEVHNAKTKPREE QFNSTYRVVSVLTVLHQDWLNGKEYKCKVSN KGLPSSIEKTISKAK | 15 |
| | | IgG4 CH3 | GQPREPQVYTLPPSQEEMTKNQVSLTCLVKGF YPSDIAVEWESNGQPENNYKTTPPVLDSDGSFF LYSRLTVDKSRWQEGNVFSCSVMHEALHNHY TQKSLSLSLGK | 16 |
| | | Linker | GGGGSGGGGSGGGGS | 18 |
| | | Angiop ep-2 | TFFYGGSRG KRNNFKTEEY | 6 |
| Fc-BTP9 (ApoB) Linker 15a.a | DNA | IgG4 Hinge | ATGCCATCATGCCCA | 11 |
| | | IgG4 CH2 | GCACCTGAGTTCCTGGGGGGACCATCAGTCT TCCTGTTCCCCCCAAAACCCAAGGACACCCT CATGATCTCCCGGACCCCTGAGGTCACATGC GTGGTGGTGGACGTGAGCCAGGAAGACCCT GAGGTCCAGTTCAACTGGTACGTGGACGGCG TGGAGGTGCATAATGCCAAGACAAAGCCGC GGGAGGAGCAGTTCAACAGCACGTACCGTG TGGTCAGCGTCCTCACCGTCCTGCACCAGGA CTGGCTGAATGGCAAGGAGTACAAGTGCAA GGTCTCCAACAAAGGCCTCCCATCCTCCATC GAGAAAACCATCTCCAAAGCCAAA | 12 |
| | | IgG4 CH3 | GGGCAGCCCCGAGAACCACAGGTGTACACC CTGCCCCCATCCCAGGAGGAGATGACCAAG | 13 |
| | | | AACCAGGTCAGCCTGACCTGCCTGGTCAAAG GCTTCTATCCCAGCGACATCGCCGTGGAGTG GGAGAGCAATGGGCAGCCGGAGAACAACTA CAAGACCACGCCTCCCGTGCTGGACTCCGAC GGCTCCTTCTTCCTCTACAGCAGGCTAACCG TGGACAAGAGCAGGTGGCAGGAGGGGAACG TCTTCTCATGCTCCGTGATGCATGAGGCTCTG CACAACCACTACACGCAGAAGAGCCTCTCCC TGTCTCTGGGTAAA | |
| | | Linker | GGTGGAGGCGGTTCAGGCGGAGGTGGCTCT GGCGGTGGCGGATCG | 17 |
| | | ApoB | AGCGTTATTGATGCACTGCAGTATAAACTGG AAGGTACCACACGTCTGACCCGCAAACGTGG TCTGAAACTGGCCACCGCACTGTCACTGAGC AATAAATTTGTTGAAGGTAGCTAA | 7 |
| | Protein | IgG4 Hinge | PSCP | 14 |
| | | IgG4 CH2 | APEFLGGPSVFLFPPKPKDTLMISRTPEVTCVV VDVSQEDPEVQFNWYVDGVEVHNAKTKPREE QFNSTYRVVSVLTVLHQDWLNGKEYKCKVSN KGLPSSIEKTISKAK | 15 |
| | | IgG4 CH3 | GQPREPQVYTLPPSQEEMTKNQVSLTCLVKGF YPSDIAVEWESNGQPENNYKTTPPVLDSDGSFF LYSRLTVDKSRWQEGNVFSCSVMHEALHNHY TQKSLSLSLGK | 16 |
| | | Linker | GGGGSGGGGSGGGGS | 18 |
| | | ApoB | SVIDALQYK LEGTTRLTRK RGLKLATALS LSNKFVEGS | 8 |
| Fc-BTP10 (ApoE) Linker 15a.a | DNA | IgG4 Hinge | ATGCCATCATGCCCA | 11 |
| | | IgG4 CH2 | GCACCTGAGTTCCTGGGGGGACCATCAGTCT TCCTGTTCCCCCCAAAACCCAAGGACACCCT CATGATCTCCCGGACCCCTGAGGTCACATGC GTGGTGGTGGACGTGAGCCAGGAAGACCCT GAGGTCCAGTTCAACTGGTACGTGGACGGCG TGGAGGTGCATAATGCCAAGACAAAGCCGC GGGAGGAGCAGTTCAACAGCACGTACCGTG TGGTCAGCGTCCTCACCGTCCTGCACCAGGA CTGGCTGAATGGCAAGGAGTACAAGTGCAA GGTCTCCAACAAAGGCCTCCCATCCTCCATC GAGAAAACCATCTCCAAAGCCAAA | 12 |
| | | IgG4 CH3 | GGGCAGCCCCGAGAACCACAGGTGTACACC CTGCCCCCATCCCAGGAGGAGATGACCAAG AACCAGGTCAGCCTGACCTGCCTGGTCAAAG GCTTCTATCCCAGCGACATCGCCGTGGAGTG GGAGAGCAATGGGCAGCCGGAGAACAACTA CAAGACCACGCCTCCCGTGCTGGACTCCGAC GGCTCCTTCTTCCTCTACAGCAGGCTAACCG TGGACAAGAGCAGGTGGCAGGAGGGGAACG TCTTCTCATGCTCCGTGATGCATGAGGCTCTG CACAACCACTACACGCAGAAGAGCCTCTCCC TGTCTCTGGGTAAA | 13 |
| | | Linker | GGTGGAGGCGGTTCAGGCGGAGGTGGCTCT GGCGGTGGCGGATCG | 17 |
| | | ApoE | CTGCGTAAACTGCGCAAACGTCTGTTACTGC GTAAACTGCGCAAACGTCTGTTATAA | 9 |
| | Protein | IgG4 Hinge | PSCP | 14 |
| | | IgG4 | APEFLGGPSVFLFPPKPKDTLMISRTPEVTCVV | 15 |
| | | CH2 | VDVSQEDPEVQFNWYVDGVEVHNAKTKPREE QFNSTYRVVSVLTVLHQDWLNGKEYKCKVSN KGLPSSIEKTISKAK | |
| | | IgG4 CH3 | GQPREPQVYTLPPSQEEMTKNQVSLTCLVKGF YPSDIAVEWESNGQPENNYKTTPPVLDSDGSFF LYSRLTVDKSRWQEGNVFSCSVMHEALHNHY TQKSLSLSLGK | 16 |
| | | Linker | GGGGSGGGGSGGGGS | 18 |
| | | ApoE | LRKLRKRLL LRKLRKRLL | 10 |

Since the gene encoding the peptide for brain targeting, which was fused to the synthesized immunoglobulin Fc region, included *Nde*I and *Bam*HI at both ends thereof, the gene was inserted into the *pET22b* expression vector, which is digested by *Nde*I and *Bam*HI*.* The expression vector, to which the gene encoding the peptide for brain targeting fused to the synthesized immunoglobulin Fc region was inserted, was inserted into the *BL21 (DE3)* strain and thereby the long-acting fusion protein, in which the immunoglobulin Fc region and the peptide for brain targeting were linked, was expressed.

### Example 2: Preparation of a peptide for brain targeting

For the detection of various peptides for brain targeting which are fused to an immunoglobulin Fc region, additional peptides including BTP1 to BTP5 for brain targeting were designed and synthesized (Table 3).

**[Table 3]**

| | | Sequence | SEQ ID NO |
|---|---|---|---|
| BTP1 | DNA | CATGCAATTTATCCGCGTCAT | 1 |
| | Protein | HAIYPRH | 2 |
| BTP2 | DNA | ACCCATCGTCCGCCGATGTGGAGCCCGGTTTGGCCG | 3 |
| | Protein | THRPPMWSPVWP | 4 |
| BTP3 | DNA | ACCTTTTTTTATGGTGGCAGCCGTGGTAAACGCAATAA CTTTAAAACCGAAGAATAT | 5 |
| | Protein | TFFYGGSRGKRNNFKTEEY | 6 |
| BTP4 | DNA | AGCGTTATTGATGCACTGCAGTATAAACTGGAAGGTAC CACACGTCTGACCCGCAAACGTGGTCTGAAACTGGCCA CCGCACTGTCACTGAGCAATAAATTTGTTGAAGGTAGC | 7 |
| | Protein | SVIDALQYKLEGTTRLTRKRGLKLATALSLSNKFVEGS | 8 |
| BTP5 | DNA | CTGCGTAAACTGCGCAAACGTCTGTTACTGCGTAAACT GCGCAAACGTCTGTTA | 9 |
| | Protein | LRKLRKRLLLRKLRKRLL | 10 |
| BTP6 | DNA | TACCAACAGATCCTCACCAGTATGCCTTCCAGAAACGT GATCCAAATATCCAACGACCTGGAGAACCTCCGGGATC TTCTTCACGTGCTG | 19 |
| | Protein | YQQILTSMPSRNVIQISNDLENLRDLLHVL | 20 |
| BTP7 | DNA | TACACCATTTGGATGCCCGAGAATCCGAGACCTGGGAC ACCTTGTGACATTTTTACCAATAGTAGAGGGAAGAGAG CATCCAATGGG | 21 |
| | Protein | YTIWMPENPRPGTPCDIFTNSRGKRASNG | 22 |
| BTP8 | DNA | AAGTCAGTCAGAACTTGGAATGAGATCATACCTTCAAA AGGGTGTTTAAGAGTTGGGGGAGGTGTCATCCTCATG TGAACGGG | 23 |
| | Protein | KSVRTWNEIIPSKGCLRVGGRCHPHVNG | 24 |
| BTP9 | DNA | CATCTGAATATTCTGAGCACCCTGTGGAAATATCGT | 25 |
| | Protein | HLNILSTLWKYR | 26 |
| BTP10 | DNA | CTGCGCAAGCTGCGTAAGCGGCTCCTCCGCGATGCCGA TGACCTGTTACGTAAACTTCGCAAACGTCTGCTTCGTG ACGCAGACGATCTG | 27 |
| | Protein | LRKLRKRLLRDADDLLRKLRKRLLRDADDL | 28 |
| BTP11 | DNA | ACCGAGGAGCTGCGGGTGCGCCTCGCCTCCCACCTGCG CAAGCTGCGTAAGCGGCTCCTC | 29 |
| | Protein | TEELRVRLASHLRKLRKRLL | 30 |
| BTP12 | DNA | CTGCGGGTGCGCCTCGCCTCCCACCTGCGCAAGCTGCG TAAGCGGCTCCTCTTACGTGTTCGTCTGGCAAGCCATCT TCGTAAACTTCGCAAACGTCTGCTT | 31 |
| | Protein | LRVRLASHLRKLRKRLLLRVRLASHLRKLRKRLL | 32 |
| BTP13 | DNA | GGTAAAGGCCCGAAATGGATGCGTTGA | 33 |
| | Protein | GKGPKWMR | 34 |
| BTP14 | DNA | GGTCATAAAGCAAAAGGCCCGCGTAAATGA | 35 |
| | Protein | GHKAKGPRK | 36 |
| BTP15 | DNA | GTTATTGCAAAAATTAAGAAACCGAAATGA | 37 |
| | Protein | VIAKIKKPK | 38 |
| BTP16 | DNA | AAGTGGAAAACCCCGAAAGTTCGTGTGTGA | 39 |
| | Protein | KWKTPKVRV | 40 |

To confirm whether the peptides for brain targeting can actually serve in brain targeting, *in vitro* and *in vivo* tests were performed.

### Example 3: Transcytosis assay of peptides for brain targeting

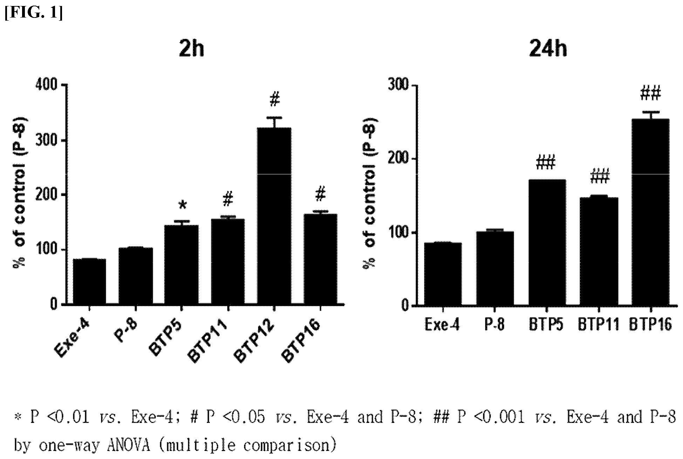

A transcytosis experiment was performed to determine the level of blood-brain barrier (BBB) passage of peptides for brain targeting. The *hCMEC*/*D3* cells, which are human cerebral endothelial cells, were cultured on a microporous membrane at a concentration of 5 × 10⁴ cells/cm² for 10 days. To confirm the BBB formation, a penetration test was performed with FITC-Dextran (70 kDa) and thereby the formation of tight junctions was confirmed. Then, 25 mM FITC-BTP5, FITC-BTP11, FITC-BTP12, FITC-BTP16, and FITC-Exendin4, FITC-P-8 (negative control, P-8) were placed on *hCMEC*/*D3* cells. After 2 hours and 24 hours, the fluorescence intensity of FITC was measured (FIG. 1).

As a result, all of the BTPs showed higher levels of passage compared to those of exendin-4 and the negative control (P-8). It was confirmed that the passage level of the peptides for brain targeting, such as BTP5, BTP11, and BTP16, increased with time.

These results suggest that the peptides for brain targeting confirmed in the present invention could effectively pass through the BBB.

### Example 4: Confirmation of brain distribution of peptides for brain targeting

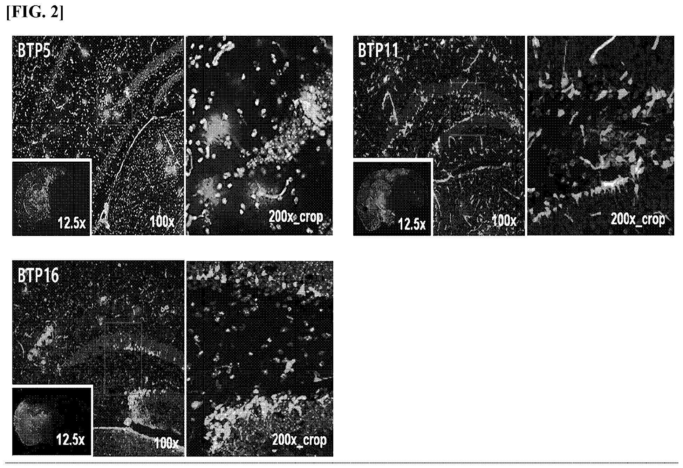

A brain distribution test was performed in mice to determine the level of BBB passage of peptides for brain targeting. Mice were administered through the common carotid artery (CCA) of the mice at a concentration of 2 mg/head and the brains of the mice were isolated and cut into fragments, and the level of passage through the BBB of peptides for brain targeting was observed under a microscope using the fluorescence of FITC (FIG. 2).

The peptides for brain targeting injected via CCA must spread to one side of the brain region in light of the injection characteristic, and as a result of the test, it was possible to observe the spreading to one side of the brain with a microscope of 12.5× magnification. Additionally, it was confirmed that the fluorescence of FITC was observed in the region where the cell bodies of the hippocampus, which plays an important role in long-term memory, are present with a microscope at magnification of 200× magnification. From these results, it was confirmed that the peptides for brain targeting passed through the BBB and entered the brain tissue.

These results suggest that the peptides for brain targeting of the present invention can be used for targeting not only *in vitro* but also into the brain *in vivo,* suggesting that physiologically active materials, which were linked to the peptides for brain targeting, could also pass through the BBB and be effectively delivered to the brain. Furthermore, these results suggest that with respect to the conjugate of the present invention, which is in the form of a long-acting conjugate, the *in vivo* duration is increased, and as the duration increases, the targeting to the brain can be more efficiently performed.

### Example 5: Preparation of expression vector for fusion proteins containing immunoglobulin Fc region and peptide for brain targeting

An expression vector was constructed to express additional fusion proteins. IgG4 Fc regions containing a hinge region were selected as a biocompatible material capable of increasing the half-life of the linked physiologically active polypeptide, and the IgG4 Fc regions containing a hinge region and the peptides for brain targeting were fused at the gene level, and the fused products was inserted into respective expression vectors.

Fusion proteins were synthesized by linking the IgG4 Fc regions, which include a hinge region, and the peptides for brain targeting by a peptide linker or by a method of direct fusion, and specifically, by linking the IgG4 Fc regions, which include a hinge region, and the peptides for brain targeting by a peptide bond (synthesized by Bioneer Corporation, Korea) (Table 4). As can be seen in the sequences of Table 4 below, the fusion proteins containing the IgG4 Fc regions, which have the sequences of Table 4 below and include a hinge region, and the peptides for brain targeting were prepared by linking the N-terminus of the peptides for brain targeting to the C-terminus of an IgG4 Fc region using a linker or by a method of direct fusion.

**[Table 4]**

| | | | Sequence | SEQ ID NO |
|---|---|---|---|---|
| Fc-BTP 11 (BTP6) | DNA | IgG4 Hinge | ATGCCATCATGCCCA | 11 |
| | | IgG4 CH2 | GCACCTGAGTTCCTGGGGGGACCATCAG TCTTCCTGTTCCCCCCAAAACCCAAGGAC ACCCTCATGATCTCCCGGACCCCTGAGGT CACATGCGTGGTGGTGGACGTGAGCCAG GAAGACCCTGAGGTCCAGTTCAACTGGT ACGTGGACGGCGTGGAGGTGCATAATGC CAAGACAAAGCCGCGGGAGGAGCAGTTC AACAGCACGTACCGTGTGGTCAGCGTCC TCACCGTCCTGCACCAGGACTGGCTGAAT GGCAAGGAGTACAAGTGCAAGGTCTCCA ACAAAGGCCTCCCATCCTCCATCGAGAA AACCATCTCCAAAGCCAAA | 12 |
| | | IgG4 CH3 | GGGCAGCCCCGAGAACCACAGGTGTACA CCCTGCCCCCATCCCAGGAGGAGATGAC CAAGAACCAGGTCAGCCTGACCTGCCTG GTCAAAGGCTTCTATCCCAGCGACATCGC CGTGGAGTGGGAGAGCAATGGGCAGCCG | 13 |
| | | | GAGAACAACTACAAGACCACGCCTCCCG TGCTGGACTCCGACGGCTCCTTCTTCCTC TACAGCAGGCTAACCGTGGACAAGAGCA GGTGGCAGGAGGGGAACGTCTTCTCATG CTCCGTGATGCATGAGGCTCTGCACAACC ACTACACGCAGAAGAGCCTCTCCCTGTCT CTGGGTAAA | |
| | | BTP6 | TACCAACAGATCCTCACCAGTATGCCTTC CAGAAACGTGATCCAAATATCCAACGAC CTGGAGAACCTCCGGGATCTTCTTCACGT GCTG | 19 |
| | Protein | IgG4 Hinge | PSCP | 14 |
| | | IgG4 CH2 | APEFLGGPSVFLFPPKPKDTLMISRTPEVTC VVVDVSQEDPEVQFNWYVDGVEVHNAKT KPREEQFNSTYRVVSVLTVLHQDWLNGKE YKCKVSNKGLPSSIEKTISKAK | 15 |
| | | IgG4 CH3 | GQPREPQVYTLPPSQEEMTKNQVSLTCLVK GFYPSDIAVEWESNGQPENNYKTTPPVLDS DGSFFLYSRLTVDKSRWQEGNVFSCSVMH EALHNHYTQKSLSLSLGK | 16 |
| | | BTP6 | YQQILTSMPSRNVIQISNDLENLRDLLHVL | 20 |
| Fc-BTP12 (BTP7) | DNA | IgG4 Hinge | ATGCCATCATGCCCA | 11 |
| | | IgG4 CH2 | GCACCTGAGTTCCTGGGGGGACCATCAG TCTTCCTGTTCCCCCCAAAACCCAAGGAC ACCCTCATGATCTCCCGGACCCCTGAGGT CACATGCGTGGTGGTGGACGTGAGCCAG GAAGACCCTGAGGTCCAGTTCAACTGGT ACGTGGACGGCGTGGAGGTGCATAATGC | 12 |
| | | | CAAGACAAAGCCGCGGGAGGAGCAGTTC AACAGCACGTACCGTGTGGTCAGCGTCC TCACCGTCCTGCACCAGGACTGGCTGAAT GGCAAGGAGTACAAGTGCAAGGTCTCCA ACAAAGGCCTCCCATCCTCCATCGAGAA AACCATCTCCAAAGCCAAA | |
| | | IgG4 CH3 | GGGCAGCCCCGAGAACCACAGGTGTACA CCCTGCCCCCATCCCAGGAGGAGATGAC CAAGAACCAGGTCAGCCTGACCTGCCTG GTCAAAGGCTTCTATCCCAGCGACATCGC CGTGGAGTGGGAGAGCAATGGGCAGCCG GAGAACAACTACAAGACCACGCCTCCCG TGCTGGACTCCGACGGCTCCTTCTTCCTC TACAGCAGGCTAACCGTGGACAAGAGCA GGTGGCAGGAGGGGAACGTCTTCTCATG CTCCGTGATGCATGAGGCTCTGCACAACC ACTACACGCAGAAGAGCCTCTCCCTGTCT CTGGGTAAA | 13 |
| | | BTP7 | TACACCATTTGGATGCCCGAGAATCCGA GACCTGGGACACCTTGTGACATTTTTACC AATAGTAGAGGGAAGAGAGCATCCAATG GG | 21 |
| | Protein | IgG4 Hinge | PSCP | 14 |
| | | IgG4 CH2 | APEFLGGPSVFLFPPKPKDTLMISRTPEVTC VVVDVSQEDPEVQFNWYVDGVEVHNAKT KPREEQFNSTYRVVSVLTVLHQDWLNGKE YKCKVSNKGLPSSIEKTISKAK | 15 |
| | | IgG4 CH3 | GQPREPQVYTLPPSQEEMTKNQVSLTCLVK GFYPSDIAVEWESNGQPENNYKTTPPVLDS | 16 |
| | | | DGSFFLYSRLTVDKSRWQEGNVFSCSVMH EALHNHYTQKSLSLSLGK | |
| | | BTP7 | YTIWMPENPRPGTPCDIFTNSRGKRASNG | 22 |
| Fc-BTP13 (BTP8) | DNA | IgG4 Hinge | ATGCCATCATGCCCA | 11 |
| | | IgG4 CH2 | GCACCTGAGTTCCTGGGGGGACCATCAG TCTTCCTGTTCCCCCCAAAACCCAAGGAC ACCCTCATGATCTCCCGGACCCCTGAGGT CACATGCGTGGTGGTGGACGTGAGCCAG GAAGACCCTGAGGTCCAGTTCAACTGGT ACGTGGACGGCGTGGAGGTGCATAATGC CAAGACAAAGCCGCGGGAGGAGCAGTTC AACAGCACGTACCGTGTGGTCAGCGTCC TCACCGTCCTGCACCAGGACTGGCTGAAT GGCAAGGAGTACAAGTGCAAGGTCTCCA ACAAAGGCCTCCCATCCTCCATCGAGAA AACCATCTCCAAAGCCAAA | 12 |
| | | IgG4 CH3 | GGGCAGCCCCGAGAACCACAGGTGTACA CCCTGCCCCCATCCCAGGAGGAGATGAC CAAGAACCAGGTCAGCCTGACCTGCCTG GTCAAAGGCTTCTATCCCAGCGACATCGC CGTGGAGTGGGAGAGCAATGGGCAGCCG GAGAACAACTACAAGACCACGCCTCCCG TGCTGGACTCCGACGGCTCCTTCTTCCTC TACAGCAGGCTAACCGTGGACAAGAGCA GGTGGCAGGAGGGGAACGTCTTCTCATG CTCCGTGATGCATGAGGCTCTGCACAACC ACTACACGCAGAAGAGCCTCTCCCTGTCT CTGGGTAAA | 13 |
| | | BTP8 | AAGTCAGTCAGAACTTGGAATGAGATCA | 23 |
| | | | TACCTTCAAAAGGGTGTTTAAGAGTTGG GGGGAGGTGTCATCCTCATGTGAACGGG | |
| | Protein | IgG4 Hinge | PSCP | 14 |
| | | IgG4 CH2 | APEFLGGPSVFLFPPKPKDTLMISRTPEVTC VVVDVSQEDPEVQFNWYVDGVEVHNAKT KPREEQFNSTYRVVSVLTVLHQDWLNGKE YKCKVSNKGLPSSIEKTISKAK | 15 |
| | | IgG4 CH3 | GQPREPQVYTLPPSQEEMTKNQVSLTCLVK GFYPSDIAVEWESNGQPENNYKTTPPVLDS DGSFFLYSRLTVDKSRWQEGNVFSCSVMH EALHNHYTQKSLSLSLGK | 16 |
| | | BTP8 | KSVRTWNEIIPSKGCLRVGGRCHPHVNG | 24 |
| Fc-BTP14 (BTP9) | DNA | IgG4 Hinge | ATGCCATCATGCCCA | 11 |
| | | IgG4 CH2 | GCACCTGAGTTCCTGGGGGGACCATCAG TCTTCCTGTTCCCCCCAAAACCCAAGGAC ACCCTCATGATCTCCCGGACCCCTGAGGT CACATGCGTGGTGGTGGACGTGAGCCAG GAAGACCCTGAGGTCCAGTTCAACTGGT ACGTGGACGGCGTGGAGGTGCATAATGC CAAGACAAAGCCGCGGGAGGAGCAGTTC AACAGCACGTACCGTGTGGTCAGCGTCC TCACCGTCCTGCACCAGGACTGGCTGAAT GGCAAGGAGTACAAGTGCAAGGTCTCCA ACAAAGGCCTCCCATCCTCCATCGAGAA AACCATCTCCAAAGCCAAA | 12 |
| | | IgG4 CH3 | GGGCAGCCCCGAGAACCACAGGTGTACA CCCTGCCCCCATCCCAGGAGGAGATGAC CAAGAACCAGGTCAGCCTGACCTGCCTG | 13 |
| | | | GTCAAAGGCTTCTATCCCAGCGACATCGC CGTGGAGTGGGAGAGCAATGGGCAGCCG GAGAACAACTACAAGACCACGCCTCCCG TGCTGGACTCCGACGGCTCCTTCTTCCTC TACAGCAGGCTAACCGTGGACAAGAGCA GGTGGCAGGAGGGGAACGTCTTCTCATG CTCCGTGATGCATGAGGCTCTGCACAACC ACTACACGCAGAAGAGCCTCTCCCTGTCT CTGGGTAAA | |
| | | BTP9 | CATCTGAATATTCTGAGCACCCTGTGGAA ATATCGT | 25 |
| | Protein | IgG4 Hinge | PSCP | 14 |
| | | IgG4 CH2 | APEFLGGPSVFLFPPKPKDTLMISRTPEVTC VVVDVSQEDPEVQFNWYVDGVEVHNAKT KPREEQFNSTYRVVSVLTVLHQDWLNGKE YKCKVSNKGLPSSIEKTISKAK | 15 |
| | | IgG4 CH3 | GQPREPQVYTLPPSQEEMTKNQVSLTCLVK GFYPSDIAVEWESNGQPENNYKTTPPVLDS DGSFFLYSRLTVDKSRWQEGNVFSCSVMH EALHNHYTQKSLSLSLGK | 16 |
| | | BTP9 | HLNILSTL WKYR | 26 |
| Fc-BTP15 (BTP10) | DNA | IgG4 Hinge | ATGCCATCATGCCCA | 11 |
| | | IgG4 CH2 | GCACCTGAGTTCCTGGGGGGACCATCAG TCTTCCTGTTCCCCCCAAAACCCAAGGAC ACCCTCATGATCTCCCGGACCCCTGAGGT CACATGCGTGGTGGTGGACGTGAGCCAG GAAGACCCTGAGGTCCAGTTCAACTGGT ACGTGGACGGCGTGGAGGTGCATAATGC | 12 |
| | | | CAAGACAAAGCCGCGGGAGGAGCAGTTC AACAGCACGTACCGTGTGGTCAGCGTCC TCACCGTCCTGCACCAGGACTGGCTGAAT GGCAAGGAGTACAAGTGCAAGGTCTCCA ACAAAGGCCTCCCATCCTCCATCGAGAA AACCATCTCCAAAGCCAAA | |
| | | IgG4 CH3 | GGGCAGCCCCGAGAACCACAGGTGTACA CCCTGCCCCCATCCCAGGAGGAGATGAC CAAGAACCAGGTCAGCCTGACCTGCCTG GTCAAAGGCTTCTATCCCAGCGACATCGC CGTGGAGTGGGAGAGCAATGGGCAGCCG GAGAACAACTACAAGACCACGCCTCCCG TGCTGGACTCCGACGGCTCCTTCTTCCTC TACAGCAGGCTAACCGTGGACAAGAGCA GGTGGCAGGAGGGGAACGTCTTCTCATG CTCCGTGATGCATGAGGCTCTGCACAACC ACTACACGCAGAAGAGCCTCTCCCTGTCT CTGGGTAAA | 13 |
| | | BTP10 | CTGCGCAAGCTGCGTAAGCGGCTCCTCC GCGATGCCGATGACCTGTTACGTAAACTT CGCAAACGTCTGCTTCGTGACGCAGACG ATCTG | 27 |
| | Protein | IgG4 Hinge | PSCP | 14 |
| | | IgG4 CH2 | APEFLGGPSVFLFPPKPKDTLMISRTPEVTC VVVDVSQEDPEVQFNWYVDGVEVHNAKT KPREEQFNSTYRVVSVLTVLHQDWLNGKE YKCKVSNKGLPSSIEKTISKAK | 15 |
| | | IgG4 CH3 | GQPREPQVYTLPPSQEEMTKNQVSLTCLVK GFYPSDIAVEWESNGQPENNYKTTPPVLDS | 16 |
| | | | DGSFFLYSRLTVDKSRWQEGNVFSCSVMH EALHNHYTQKSLSLSLGK | |
| | | BTP10 | LRKLRKRLLRDADDLLRKLRKRLLRDADD L | 28 |
| Fc-BTP16 (BTP11) | DNA | IgG4 Hinge | ATGCCATCATGCCCA | 11 |
| | | IgG4 CH2 | GCACCTGAGTTCCTGGGGGGACCATCAG TCTTCCTGTTCCCCCCAAAACCCAAGGAC ACCCTCATGATCTCCCGGACCCCTGAGGT CACATGCGTGGTGGTGGACGTGAGCCAG GAAGACCCTGAGGTCCAGTTCAACTGGT ACGTGGACGGCGTGGAGGTGCATAATGC CAAGACAAAGCCGCGGGAGGAGCAGTTC AACAGCACGTACCGTGTGGTCAGCGTCC TCACCGTCCTGCACCAGGACTGGCTGAAT GGCAAGGAGTACAAGTGCAAGGTCTCCA ACAAAGGCCTCCCATCCTCCATCGAGAA AACCATCTCCAAAGCCAAA | 12 |
| | | IgG4 CH3 | GGGCAGCCCCGAGAACCACAGGTGTACA CCCTGCCCCCATCCCAGGAGGAGATGAC CAAGAACCAGGTCAGCCTGACCTGCCTG GTCAAAGGCTTCTATCCCAGCGACATCGC CGTGGAGTGGGAGAGCAATGGGCAGCCG GAGAACAACTACAAGACCACGCCTCCCG TGCTGGACTCCGACGGCTCCTTCTTCCTC TACAGCAGGCTAACCGTGGACAAGAGCA GGTGGCAGGAGGGGAACGTCTTCTCATG CTCCGTGATGCATGAGGCTCTGCACAACC ACTACACGCAGAAGAGCCTCTCCCTGTCT CTGGGTAAA | 13 |
| | | BTP11 | ACCGAGGAGCTGCGGGTGCGCCTCGCCT CCCACCTGCGCAAGCTGCGTAAGCGGCT CCTC | 29 |
| | Protein | IgG4 Hinge | PSCP | 14 |
| | | IgG4 CH2 | APEFLGGPSVFLFPPKPKDTLMISRTPEVTC VVVDVSQEDPEVQFNWYVDGVEVHNAKT KPREEQFNSTYRVVSVLTVLHQDWLNGKE YKCKVSNKGLPSSIEKTISKAK | 15 |
| | | IgG4 CH3 | GQPREPQVYTLPPSQEEMTKNQVSLTCLVK GFYPSDIAVEWESNGQPENNYKTTPPVLDS DGSFFLYSRLTVDKSRWQEGNVFSCSVMH EALHNHYTQKSLSLSLGK | 16 |
| | | BTP11 | TEELRVRLASHLRKLRKRLL | 30 |
| Fc-BTP17 (BTP12) | DNA | IgG4 Hinge | ATGCCATCATGCCCA | 11 |
| | | IgG4 CH2 | GCACCTGAGTTCCTGGGGGGACCATCAG TCTTCCTGTTCCCCCCAAAACCCAAGGAC ACCCTCATGATCTCCCGGACCCCTGAGGT CACATGCGTGGTGGTGGACGTGAGCCAG GAAGACCCTGAGGTCCAGTTCAACTGGT ACGTGGACGGCGTGGAGGTGCATAATGC CAAGACAAAGCCGCGGGAGGAGCAGTTC AACAGCACGTACCGTGTGGTCAGCGTCC TCACCGTCCTGCACCAGGACTGGCTGAAT GGCAAGGAGTACAAGTGCAAGGTCTCCA ACAAAGGCCTCCCATCCTCCATCGAGAA AACCATCTCCAAAGCCAAA | 12 |
| | | IgG4 CH3 | GGGCAGCCCCGAGAACCACAGGTGTACA CCCTGCCCCCATCCCAGGAGGAGATGAC | 13 |
| | | | CAAGAACCAGGTCAGCCTGACCTGCCTG GTCAAAGGCTTCTATCCCAGCGACATCGC CGTGGAGTGGGAGAGCAATGGGCAGCCG GAGAACAACTACAAGACCACGCCTCCCG TGCTGGACTCCGACGGCTCCTTCTTCCTC TACAGCAGGCTAACCGTGGACAAGAGCA GGTGGCAGGAGGGGAACGTCTTCTCATG CTCCGTGATGCATGAGGCTCTGCACAACC ACTACACGCAGAAGAGCCTCTCCCTGTCT CTGGGTAAA | |
| | | BTP12 | CTGCGGGTGCGCCTCGCCTCCCACCTGCG CAAGCTGCGTAAGCGGCTCCTCTTACGTG TTCGTCTGGCAAGCCATCTTCGTAAACTT CGCAAACGTCTGCTT | 31 |
| | Protein | IgG4 Hinge | PSCP | 14 |
| | | IgG4 CH2 | APEFLGGPSVFLFPPKPKDTLMISRTPEVTC VVVDVSQEDPEVQFNWYVDGVEVHNAKT KPREEQFNSTYRVVSVLTVLHQDWLNGKE YKCKVSNKGLPSSIEKTISKAK | 15 |
| | | IgG4 CH3 | GQPREPQVYTLPPSQEEMTKNQVSLTCLVK GFYPSDIAVEWESNGQPENNYKTTPPVLDS DGSFFLYSRLTVDKSRWQEGNVFSCSVMH EALHNHYTQKSLSLSLGK | 16 |
| | | BTP12 | LRVRLASHLRKLRKRLLLRVRLASHLRKLR KRLL | 32 |
| Fc-BTP18 (BTP13) | DNA | IgG4 Hinge | ATGCCATCATGCCCA | 11 |
| | | IgG4 CH2 | GCACCTGAGTTCCTGGGGGGACCATCAG TCTTCCTGTTCCCCCCAAAACCCAAGGAC | 12 |
| | | | ACCCTCATGATCTCCCGGACCCCTGAGGT CACATGCGTGGTGGTGGACGTGAGCCAG GAAGACCCTGAGGTCCAGTTCAACTGGT ACGTGGACGGCGTGGAGGTGCATAATGC CAAGACAAAGCCGCGGGAGGAGCAGTTC AACAGCACGTACCGTGTGGTCAGCGTCC TCACCGTCCTGCACCAGGACTGGCTGAAT GGCAAGGAGTACAAGTGCAAGGTCTCCA ACAAAGGCCTCCCATCCTCCATCGAGAA AACCATCTCCAAAGCCAAA | |
| | | IgG4 CH3 | GGGCAGCCCCGAGAACCACAGGTGTACA CCCTGCCCCCATCCCAGGAGGAGATGAC CAAGAACCAGGTCAGCCTGACCTGCCTG GTCAAAGGCTTCTATCCCAGCGACATCGC CGTGGAGTGGGAGAGCAATGGGCAGCCG GAGAACAACTACAAGACCACGCCTCCCG TGCTGGACTCCGACGGCTCCTTCTTCCTC TACAGCAGGCTAACCGTGGACAAGAGCA GGTGGCAGGAGGGGAACGTCTTCTCATG CTCCGTGATGCATGAGGCTCTGCACAACC ACTACACGCAGAAGAGCCTCTCCCTGTCT CTGGGTAAA | 13 |
| | | BTP13 | GGTAAAGGCCCGAAATGGATGCGTTGA | 33 |
| | Protein | IgG4 Hinge | PSCP | 14 |
| | | IgG4 CH2 | APEFLGGPSVFLFPPKPKDTLMISRTPEVTC VVVDVSQEDPEVQFNWYVDGVEVHNAKT KPREEQFNSTYRVVSVLTVLHQDWLNGKE YKCKVSNKGLPSSIEKTISKAK | 15 |
| | | IgG4 | GQPREPQVYTLPPSQEEMTKNQVSLTCLVK | 16 |
| | | CH3 | GFYPSDIAVEWESNGQPENNYKTTPPVLDS DGSFFLYSRLTVDKSRWQEGNVFSCSVMH EALHNHYTQKSLSLSLGK | |
| | | BTP13 | GKGPKWMR | 34 |
| Fc-BTP19 (BTP14) | DNA | IgG4 Hinge | ATGCCATCATGCCCA | 11 |
| | | IgG4 CH2 | GCACCTGAGTTCCTGGGGGGACCATCAG TCTTCCTGTTCCCCCCAAAACCCAAGGAC ACCCTCATGATCTCCCGGACCCCTGAGGT CACATGCGTGGTGGTGGACGTGAGCCAG GAAGACCCTGAGGTCCAGTTCAACTGGT ACGTGGACGGCGTGGAGGTGCATAATGC CAAGACAAAGCCGCGGGAGGAGCAGTTC AACAGCACGTACCGTGTGGTCAGCGTCC TCACCGTCCTGCACCAGGACTGGCTGAAT GGCAAGGAGTACAAGTGCAAGGTCTCCA ACAAAGGCCTCCCATCCTCCATCGAGAA AACCATCTCCAAAGCCAAA | 12 |
| | | IgG4 CH3 | GGGCAGCCCCGAGAACCACAGGTGTACA CCCTGCCCCCATCCCAGGAGGAGATGAC CAAGAACCAGGTCAGCCTGACCTGCCTG GTCAAAGGCTTCTATCCCAGCGACATCGC CGTGGAGTGGGAGAGCAATGGGCAGCCG GAGAACAACTACAAGACCACGCCTCCCG TGCTGGACTCCGACGGCTCCTTCTTCCTC TACAGCAGGCTAACCGTGGACAAGAGCA GGTGGCAGGAGGGGAACGTCTTCTCATG CTCCGTGATGCATGAGGCTCTGCACAACC ACTACACGCAGAAGAGCCTCTCCCTGTCT CTGGGTAAA | 13 |
| | | BTP14 | GGTCATAAAGCAAAAGGCCCGCGTAAAT GA | 35 |
| | Protein | IgG4 Hinge | PSCP | 14 |
| | | IgG4 CH2 | APEFLGGPSVFLFPPKPKDTLMISRTPEVTC VVVDVSQEDPEVQFNWYVDGVEVHNAKT KPREEQFNSTYRVVSVLTVLHQDWLNGKE YKCKVSNKGLPSSIEKTISKAK | 15 |
| | | IgG4 CH3 | GQPREPQVYTLPPSQEEMTKNQVSLTCLVK GFYPSDIAVEWESNGQPENNYKTTPPVLDS DGSFFLYSRLTVDKSRWQEGNVFSCSVMH EALHNHYTQKSLSLSLGK | 16 |
| | | BTP14 | GHKAKGPRK | 36 |
| Fc-BTP20 (BTP15) | DNA | IgG4 Hinge | ATGCCATCATGCCCA | 11 |
| | | IgG4 CH2 | GCACCTGAGTTCCTGGGGGGACCATCAG TCTTCCTGTTCCCCCCAAAACCCAAGGAC ACCCTCATGATCTCCCGGACCCCTGAGGT CACATGCGTGGTGGTGGACGTGAGCCAG GAAGACCCTGAGGTCCAGTTCAACTGGT ACGTGGACGGCGTGGAGGTGCATAATGC CAAGACAAAGCCGCGGGAGGAGCAGTTC AACAGCACGTACCGTGTGGTCAGCGTCC TCACCGTCCTGCACCAGGACTGGCTGAAT GGCAAGGAGTACAAGTGCAAGGTCTCCA ACAAAGGCCTCCCATCCTCCATCGAGAA AACCATCTCCAAAGCCAAA | 12 |
| | | IgG4 CH3 | GGGCAGCCCCGAGAACCACAGGTGTACA CCCTGCCCCCATCCCAGGAGGAGATGAC CAAGAACCAGGTCAGCCTGACCTGCCTG | 13 |
| | | | GTCAAAGGCTTCTATCCCAGCGACATCGC CGTGGAGTGGGAGAGCAATGGGCAGCCG GAGAACAACTACAAGACCACGCCTCCCG TGCTGGACTCCGACGGCTCCTTCTTCCTC TACAGCAGGCTAACCGTGGACAAGAGCA GGTGGCAGGAGGGGAACGTCTTCTCATG CTCCGTGATGCATGAGGCTCTGCACAACC ACTACACGCAGAAGAGCCTCTCCCTGTCT CTGGGTAAA | |
| | | BTP15 | GTTATTGCAAAAATTAAGAAACCGAAAT GA | 37 |
| | Protein | IgG4 Hinge | PSCP | 14 |
| | | IgG4 CH2 | APEFLGGPSVFLFPPKPKDTLMISRTPEVTC VVVDVSQEDPEVQFNWYVDGVEVHNAKT KPREEQFNSTYRVVSVLTVLHQDWLNGKE YKCKVSNKGLPSSIEKTISKAK | 15 |
| | | IgG4 CH3 | GQPREPQVYTLPPSQEEMTKNQVSLTCLVK GFYPSDIAVEWESNGQPENNYKTTPPVLDS DGSFFLYSRLTVDKSRWQEGNVFSCSVMH EALHNHYTQKSLSLSLGK | 16 |
| | | BTP15 | VIAKIKKPK | 38 |
| Fc-BTP21 (BTP16) | DNA | IgG4 Hinge | ATGCCATCATGCCCA | 11 |
| | | IgG4 CH2 | GCACCTGAGTTCCTGGGGGGACCATCAG TCTTCCTGTTCCCCCCAAAACCCAAGGAC ACCCTCATGATCTCCCGGACCCCTGAGGT CACATGCGTGGTGGTGGACGTGAGCCAG GAAGACCCTGAGGTCCAGTTCAACTGGT ACGTGGACGGCGTGGAGGTGCATAATGC | 12 |
| | | | CAAGACAAAGCCGCGGGAGGAGCAGTTC AACAGCACGTACCGTGTGGTCAGCGTCC TCACCGTCCTGCACCAGGACTGGCTGAAT GGCAAGGAGTACAAGTGCAAGGTCTCCA ACAAAGGCCTCCCATCCTCCATCGAGAA AACCATCTCCAAAGCCAAA | |
| | | IgG4 CH3 | GGGCAGCCCCGAGAACCACAGGTGTACA CCCTGCCCCCATCCCAGGAGGAGATGAC CAAGAACCAGGTCAGCCTGACCTGCCTG GTCAAAGGCTTCTATCCCAGCGACATCGC CGTGGAGTGGGAGAGCAATGGGCAGCCG GAGAACAACTACAAGACCACGCCTCCCG TGCTGGACTCCGACGGCTCCTTCTTCCTC TACAGCAGGCTAACCGTGGACAAGAGCA GGTGGCAGGAGGGGAACGTCTTCTCATG CTCCGTGATGCATGAGGCTCTGCACAACC ACTACACGCAGAAGAGCCTCTCCCTGTCT CTGGGTAAA | 13 |
| | | BTP16 | AAGTGGAAAACCCCGAAAGTTCGTGTGT GA | 39 |
| | Protein | IgG4 Hinge | PSCP | 14 |
| | | IgG4 CH2 | APEFLGGPSVFLFPPKPKDTLMISRTPEVTC VVVDVSQEDPEVQFNWYVDGVEVHNAKT KPREEQFNSTYRVVSVLTVLHQDWLNGKE YKCKVSNKGLPSSIEKTISKAK | 15 |
| | | IgG4 CH3 | GQPREPQVYTLPPSQEEMTKNQVSLTCLVK GFYPSDIAVEWESNGQPENNYKTTPPVLDS DGSFFLYSRLTVDKSRWQEGNVFSCSVMH EALHNHYTQKSLSLSLGK | 16 |
| | | BTP16 | KWKTPKVRV | 40 |

Since the gene encoding the peptide for brain targeting, which was fused to the synthesized immunoglobulin Fc region, included *Nde*I and *Bam*HI at both ends thereof, the gene was inserted into the *pET22b* expression vector, which was digested by *Nde*I and *Bam*HI*.* The expression vector, to which the gene encoding the peptide for brain targeting fused to the synthesized immunoglobulin Fc region was inserted, was inserted into the *BL21 (DE3)* strain and thereby the long-acting fusion protein, in which the immunoglobulin Fc region and the peptide for brain targeting were linked, was expressed.

### Example 6: Preparation of expression vector for fusion proteins in which an immunoglobulin Fc region and at least two peptides for brain targeting are linked

Expression vectors were constructed to express additional fusion proteins to increase receptor binding affinity. IgG4 Fc regions including a hinge region were selected as a biocompatible material capable of increasing the half-life of linked physiologically active polypeptides, and linkers and peptides for brain targeting were inserted into the constructed expression vectors at the gene level by site-directed mutagenesis PCR. Specifically, the linkers and peptides for brain targeting were inserted between the C-terminus of an immunoglobulin Fc region and the N-terminus of the peptides for brain targeting, or the C-terminus of the peptides for brain targeting and the site where the *Bam*HI site of the vector is included, using the SEQ ID NO primers (Table 5). Expression vectors expressing fusion proteins (Table 6) containing two or more replicated peptides for brain targeting were constructed. These fusion proteins had the sequences shown in Table 6. For example, in the Fc-BTP22 conjugate, linkers are intercalated in two units of BTP5 peptides of Table 2 and thereby linked to the C-terminal direction of the CH3 region of an Fc fragment. Specifically, as can be confirmed in Table 6 below, fusion proteins were prepared by linking the C-terminus of an immunoglobulin Fc region and the N-terminus of each of the peptides for brain targeting, or by a method of direct fusion. In this case, the peptides for brain targeting in a form linked in tandem means that the peptides for brain targeting are in a form where single-unit peptides for brain targeting are linked by a linker.

**[Table 5]**

| | Sequence | SEQ ID NO |
|---|---|---|
| Fc-BTP22-F | 5′- TCTCCCTGTCTCTGGGTAAAGGTGGAGGCGGTTCACTTCGC AAGTTACGTAAACGCTTACTGTTACGTAAACTTCGGAAGC GCTTACTGGGCGGAGGTGGCTCTCTGCGTAAACTGCGCAA ACG-3′ | 62 |
| Fc-BTP22-R | 5′- CGTTTGCGCAGTTTACGCAGAGAGCCACCTCCGCCCAGTA AGCGCTTCCGAAGTTTACGTAACAGTAAGCGTTTACGTAA CTTGCGAAGTGAACCGCCTCCACCTTTACCCAGAGACAGG GAGA-3′ | 63 |
| Fc-BTP23-F | 5′- AACTGCGCAAACGTCTGTTAGGCGGTGGCGGATCGCTGCG TAAACTGCGCAAACGTCTGTTACTTCGGAAGCTGAGAAAA CGCTTACTTTAAGGATCCgaattcgagctccG-3′ | 64 |
| Fc-BTP23-R | 5′- CGGAGCTCGAATTCGGATCCTTAAAGTAAGCGTTTTCTCA GCTTCCGAAGTAACAGACGTTTGCGCAGTTTACGCAGCGA TCCGCCACCGCCTAACAGACGTTTGCGCAGTT-3′ | 65 |
| Fc-BTP24-F | 5′- TCTCCCTGTCTCTGGGTAAAGGTGGAGGCGGTTCAACAGA AGAGTTACGTGTTCGTCTGGCAAGCCATTTACGTAAACTTC GCAAACGTCTGTTAGGCGGAGGTGGCTCTACCGAGGAGCT GCGGGTGCG-3′ | 66 |
| Fc-BTP24-R | 5′- CGCACCCGCAGCTCCTCGGTAGAGCCACCTCCGCCTAACA GACGTTTGCGAAGTTTACGTAAATGGCTTGCCAGACGAAC ACGTAACTCTTCTGTTGAACCGCCTCCACCTTTACCCAGAG ACAGGGAGA-3′ | 67 |
| Fc-BTP25-F | 5′- AGCTGCGTAAGCGGCTCCTCGGCGGTGGCGGATCGACGGA AGAACTTCGCGTCCGGTTAGCGTCACATCTTCGGAAATTA CGGAAGCGCTTGCTGTGAGGATCCgaattcgagctccG-3′ | 68 |
| Fc-BTP25-R | 5′- CGGAGCTCGAATTCGGATCCTCACAGCAAGCGCTTCCGTA ATTTCCGAAGATGTGACGCTAACCGGACGCGAAGTTCTTC CGTCGATCCGCCACCGCCGAGGAGCCGCTTACGCAGCT-3′ | 69 |
| Fc-BTP26-F | 5′- TCTCCCTGTCTCTGGGTAAAGGTGGAGGCGGTTCAAAATG GAAGACACCAAAGGTGCGCGTTGGCGGAGGTGGCTCTAA GTGGAAAACCCCGAAAGT-3′ | 70 |
| Fc-BTP26-R | 5′- ACTTTCGGGGTTTTCCACTTAGAGCCACCTCCGCCAACGC GCACCTTTGGTGTCTTCCATTTTGAACCGCCTCCACCTTTA CCCAGAGACAGGGAGA-3′ | 71 |
| Fc-BTP27-F | 5′- AAACCCCGAAAGTTCGTGTGGGCGGTGGCGGATCGAAATG GAAAACTCCTAAAGTACGGGTCTGAGGATCCgaattcgagctccG -3′ | 72 |
| Fc-BTP27-R | 5′- CGGAGCTCGAATTCGGATCCTCAGACCCGTACTTTAGGAG TTTTCCATTTCGATCCGCCACCGCCCACACGAACTTTCGGG GTTT-3′ | 73 |

**[Table 6]**

| | | | | |
|---|---|---|---|---|
| Fc-BTP22 (BTP5 2x) | DNA | IgG4 Hinge | ATGCCATCATGCCCA | 11 |
| | | IgG4 CH2 | GCACCTGAGTTCCTGGGGGGACCATCAGT CTTCCTGTTCCCCCCAAAACCCAAGGACA | 12 |
| | | | CCCTCATGATCTCCCGGACCCCTGAGGTC ACATGCGTGGTGGTGGACGTGAGCCAGGA AGACCCTGAGGTCCAGTTCAACTGGTACG TGGACGGCGTGGAGGTGCATAATGCCAAG ACAAAGCCGCGGGAGGAGCAGTTCAACA GCACGTACCGTGTGGTCAGCGTCCTCACC GTCCTGCACCAGGACTGGCTGAATGGCAA GGAGTACAAGTGCAAGGTCTCCAACAAA GGCCTCCCATCCTCCATCGAGAAAACCAT CTCCAAAGCCAAA | |
| | | IgG4 CH3 | GGGCAGCCCCGAGAACCACAGGTGTACA CCCTGCCCCCATCCCAGGAGGAGATGACC AAGAACCAGGTCAGCCTGACCTGCCTGGT CAAAGGCTTCTATCCCAGCGACATCGCCG TGGAGTGGGAGAGCAATGGGCAGCCGGA GAACAACTACAAGACCACGCCTCCCGTGC TGGACTCCGACGGCTCCTTCTTCCTCTACA GCAGGCTAACCGTGGACAAGAGCAGGTG GCAGGAGGGGAACGTCTTCTCATGCTCCG TGATGCATGAGGCTCTGCACAACCACTAC ACGCAGAAGAGCCTCTCCCTGTCTCTGGG TAAA | 13 |
| | | Linker | GGTGGAGGCGGTTCA | 74 |
| | | BTP5 | CTTCGCAAGTTACGTAAACGCTTACTGTT ACGTAAACTTCGGAAGCGCTTACTG | 75 |
| | | Linker | GGTGGAGGCGGTTCA | 76 |
| | | BTP5 | CTGCGTAAACTGCGCAAACGTCTGTTACT GCGTAAACTGCGCAAACGTCTGTTA | 9 |
| | Protein | IgG4 Hinge | PSCP | 14 |
| | | IgG4 CH2 | APEFLGGPSVFLFPPKPKDTLMISRTPEVTCV VVDVSQEDPEVQFNWYVDGVEVHNAKTKP REEQFNSTYRVVSVLTVLHQDWLNGKEYK CKVSNKGLPSSIEKTISKAK | 15 |
| | | IgG4 CH3 | GQPREPQVYTLPPSQEEMTKNQVSLTCLVK GFYPSDIAVEWESNGQPENNYKTTPPVLDS DGSFFLYSRLTVDKSRWQEGNVFSCSVMHE ALHNHYTQKSLSLSLGK | 16 |
| | | Linker | GGGGS | 77 |
| | | BTP5 | LRKLRKRLLLRKLRKRLL | 10 |
| | | Linker | GGGGS | 77 |
| | | BTP5 | LRKLRKRLLLRKLRKRLL | 10 |
| Fc-BTP23 (BTP5 3x) | DNA | IgG4 Hinge | ATGCCATCATGCCCA | 11 |
| | | IgG4 CH2 | GCACCTGAGTTCCTGGGGGGACCATCAGT CTTCCTGTTCCCCCCAAAACCCAAGGACA CCCTCATGATCTCCCGGACCCCTGAGGTC ACATGCGTGGTGGTGGACGTGAGCCAGGA AGACCCTGAGGTCCAGTTCAACTGGTACG TGGACGGCGTGGAGGTGCATAATGCCAAG ACAAAGCCGCGGGAGGAGCAGTTCAACA GCACGTACCGTGTGGTCAGCGTCCTCACC GTCCTGCACCAGGACTGGCTGAATGGCAA GGAGTACAAGTGCAAGGTCTCCAACAAA GGCCTCCCATCCTCCATCGAGAAAACCAT CTCCAAAGCCAAA | 12 |
| | | IgG4 CH3 | GGGCAGCCCCGAGAACCACAGGTGTACA CCCTGCCCCCATCCCAGGAGGAGATGACC AAGAACCAGGTCAGCCTGACCTGCCTGGT CAAAGGCTTCTATCCCAGCGACATCGCCG | 13 |
| | | | TGGAGTGGGAGAGCAATGGGCAGCCGGA GAACAACTACAAGACCACGCCTCCCGTGC TGGACTCCGACGGCTCCTTCTTCCTCTACA GCAGGCTAACCGTGGACAAGAGCAGGTG GCAGGAGGGGAACGTCTTCTCATGCTCCG TGATGCATGAGGCTCTGCACAACCACTAC ACGCAGAAGAGCCTCTCCCTGTCTCTGGG TAAA | |
| | | Linker | GGTGGAGGCGGTTCA | 74 |
| | | BTP5 | CTTCGCAAGTTACGTAAACGCTTACTGTT ACGTAAACTTCGGAAGCGCTTACTG | 75 |
| | | Linker | GGCGGAGGTGGCTCT | 76 |
| | | BTP5 | CTGCGTAAACTGCGCAAACGTCTGTTACT GCGTAAACTGCGCAAACGTCTGTTA | 9 |
| | | Linker | GGCGGTGGCGGATCG | 78 |
| | | BTP5 | CTGCGTAAACTGCGCAAACGTCTGTTACT TCGGAAGCTGAGAAAACGCTTACTTTAA | 79 |
| | Protein | IgG4 Hinge | PSCP | 14 |
| | | IgG4 CH2 | APEFLGGPSVFLFPPKPKDTLMISRTPEVTCV VVDVSQEDPEVQFNWYVDGVEVHNAKTKP REEQFNSTYRVVSVLTVLHQDWLNGKEYK CKVSNKGLPSSIEKTISKAK | 15 |
| | | IgG4 CH3 | GQPREPQVYTLPPSQEEMTKNQVSLTCLVK GFYPSDIAVEWESNGQPENNYKTTPPVLDS DGSFFLYSRLTVDKSRWQEGNVFSCSVMHE ALHNHYTQKSLSLSLGK | 16 |
| | | Linker | GGGGS | 77 |
| | | BTP5 | LRKLRKRLLLRKLRKRLL | 10 |
| | | Linker | GGGGS | 77 |
| | | BTP5 | LRKLRKRLLLRKLRKRLL | 10 |
| | | Linker | GGGGS | 77 |
| | | BTP5 | LRKLRKRLLLRKLRKRLL | 10 |
| Fc-BTP24 (BTP11 2x) | DNA | IgG4 Hinge | ATGCCATCATGCCCA | 11 |
| | | IgG4 CH2 | GCACCTGAGTTCCTGGGGGGACCATCAGT CTTCCTGTTCCCCCCAAAACCCAAGGACA CCCTCATGATCTCCCGGACCCCTGAGGTC ACATGCGTGGTGGTGGACGTGAGCCAGGA AGACCCTGAGGTCCAGTTCAACTGGTACG TGGACGGCGTGGAGGTGCATAATGCCAAG ACAAAGCCGCGGGAGGAGCAGTTCAACA GCACGTACCGTGTGGTCAGCGTCCTCACC GTCCTGCACCAGGACTGGCTGAATGGCAA GGAGTACAAGTGCAAGGTCTCCAACAAA GGCCTCCCATCCTCCATCGAGAAAACCAT CTCCAAAGCCAAA | 12 |
| | | IgG4 CH3 | GGGCAGCCCCGAGAACCACAGGTGTACA CCCTGCCCCCATCCCAGGAGGAGATGACC AAGAACCAGGTCAGCCTGACCTGCCTGGT CAAAGGCTTCTATCCCAGCGACATCGCCG TGGAGTGGGAGAGCAATGGGCAGCCGGA GAACAACTACAAGACCACGCCTCCCGTGC TGGACTCCGACGGCTCCTTCTTCCTCTACA GCAGGCTAACCGTGGACAAGAGCAGGTG GCAGGAGGGGAACGTCTTCTCATGCTCCG TGATGCATGAGGCTCTGCACAACCACTAC ACGCAGAAGAGCCTCTCCCTGTCTCTGGG TAAA | 13 |
| | | Linker | GGTGGAGGCGGTTCA | 74 |
| | | BTP11 | ACAGAAGAGTTACGTGTTCGTCTGGCAAG CCATTTACGTAAACTTCGCAAACGTCTGTT A | 80 |
| | | Linker | GGCGGAGGTGGCTCT | 76 |
| | | BTP11 | ACCGAGGAGCTGCGGGTGCGCCTCGCCTC CCACCTGCGCAAGCTGCGTAAGCGGCTCC TC | 29 |
| | Protein | IgG4 Hinge | PSCP | 14 |
| | | IgG4 CH2 | APEFLGGPSVFLFPPKPKDTLMISRTPEVTCV VVDVSQEDPEVQFNWYVDGVEVHNAKTKP REEQFNSTYRVVSVLTVLHQDWLNGKEYK CKVSNKGLPSSIEKTISKAK | 15 |
| | | IgG4 CH3 | GQPREPQVYTLPPSQEEMTKNQVSLTCLVK GFYPSDIAVEWESNGQPENNYKTTPPVLDS DGSFFLYSRLTVDKSRWQEGNVFSCSVMHE ALHNHYTQKSLSLSLGK | 16 |
| | | Linker | GGGGS | 77 |
| | | BTP11 | TEELRVRLASHLRKLRKRLL | 30 |
| | | Linker | GGGGS | 77 |
| | | BTP11 | TEELRVRLASHLRKLRKRLL | 30 |
| Fc-BTP25 (BTP11 3x) | DNA | IgG4 Hinge | ATGCCATCATGCCCA | 11 |
| | | IgG4 CH2 | GCACCTGAGTTCCTGGGGGGACCATCAGT CTTCCTGTTCCCCCCAAAACCCAAGGACA CCCTCATGATCTCCCGGACCCCTGAGGTC ACATGCGTGGTGGTGGACGTGAGCCAGGA AGACCCTGAGGTCCAGTTCAACTGGTACG TGGACGGCGTGGAGGTGCATAATGCCAAG | 12 |
| | | | ACAAAGCCGCGGGAGGAGCAGTTCAACA GCACGTACCGTGTGGTCAGCGTCCTCACC GTCCTGCACCAGGACTGGCTGAATGGCAA GGAGTACAAGTGCAAGGTCTCCAACAAA GGCCTCCCATCCTCCATCGAGAAAACCAT CTCCAAAGCCAAA | |
| | | IgG4 CH3 | GGGCAGCCCCGAGAACCACAGGTGTACA CCCTGCCCCCATCCCAGGAGGAGATGACC AAGAACCAGGTCAGCCTGACCTGCCTGGT CAAAGGCTTCTATCCCAGCGACATCGCCG TGGAGTGGGAGAGCAATGGGCAGCCGGA GAACAACTACAAGACCACGCCTCCCGTGC TGGACTCCGACGGCTCCTTCTTCCTCTACA GCAGGCTAACCGTGGACAAGAGCAGGTG GCAGGAGGGGAACGTCTTCTCATGCTCCG TGATGCATGAGGCTCTGCACAACCACTAC ACGCAGAAGAGCCTCTCCCTGTCTCTGGG TAAA | 13 |
| | | Linker | GGTGGAGGCGGTTCA | 74 |
| | | BTP11 | ACAGAAGAGTTACGTGTTCGTCTGGCAAG CCATTTACGTAAACTTCGCAAACGTCTGTT A | 80 |
| | | Linker | GGCGGAGGTGGCTCT | 76 |
| | | BTP11 | ACCGAGGAGCTGCGGGTGCGCCTCGCCTC CCACCTGCGCAAGCTGCGTAAGCGGCTCC TC | 29 |
| | | Linker | GGCGGTGGCGGATCG | 78 |
| | | BTP11 | ACGGAAGAACTTCGCGTCCGGTTAGCGTC ACATCTTCGGAAATTACGGAAGCGCTTGC TGTGA | 79 |
| | Protein | IgG4 Hinge | PSCP | 14 |
| | | IgG4 CH2 | APEFLGGPSVFLFPPKPKDTLMISRTPEVTCV VVDVSQEDPEVQFNWYVDGVEVHNAKTKP REEQFNSTYRVVSVLTVLHQDWLNGKEYK CKVSNKGLPSSIEKTISKAK | 15 |
| | | IgG4 CH3 | GQPREPQVYTLPPSQEEMTKNQVSLTCLVK GFYPSDIAVEWESNGQPENNYKTTPPVLDS DGSFFLYSRLTVDKSRWQEGNVFSCSVMHE ALHNHYTQKSLSLSLGK | 16 |
| | | Linker | GGGGS | 77 |
| | | BTP11 | TEELRVRLASHLRKLRKRLL | 30 |
| | | Linker | GGGGS | 77 |
| | | BTP11 | TEELRVRLASHLRKLRKRLL | 30 |
| | | Linker | GGGGS | 77 |
| | | BTP11 | TEELRVRLASHLRKLRKRLL | 30 |
| Fc-BTP26 (BTP16 2x) | DNA | IgG4 Hinge | ATGCCATCATGCCCA | 11 |
| | | IgG4 CH2 | GCACCTGAGTTCCTGGGGGGACCATCAGT CTTCCTGTTCCCCCCAAAACCCAAGGACA CCCTCATGATCTCCCGGACCCCTGAGGTC ACATGCGTGGTGGTGGACGTGAGCCAGGA AGACCCTGAGGTCCAGTTCAACTGGTACG TGGACGGCGTGGAGGTGCATAATGCCAAG ACAAAGCCGCGGGAGGAGCAGTTCAACA GCACGTACCGTGTGGTCAGCGTCCTCACC GTCCTGCACCAGGACTGGCTGAATGGCAA GGAGTACAAGTGCAAGGTCTCCAACAAA GGCCTCCCATCCTCCATCGAGAAAACCAT CTCCAAAGCCAAA | 12 |
| | | IgG4 CH3 | GGGCAGCCCCGAGAACCACAGGTGTACA CCCTGCCCCCATCCCAGGAGGAGATGACC AAGAACCAGGTCAGCCTGACCTGCCTGGT CAAAGGCTTCTATCCCAGCGACATCGCCG TGGAGTGGGAGAGCAATGGGCAGCCGGA GAACAACTACAAGACCACGCCTCCCGTGC TGGACTCCGACGGCTCCTTCTTCCTCTACA GCAGGCTAACCGTGGACAAGAGCAGGTG GCAGGAGGGGAACGTCTTCTCATGCTCCG TGATGCATGAGGCTCTGCACAACCACTAC ACGCAGAAGAGCCTCTCCCTGTCTCTGGG TAAA | 13 |
| | | Linker | GGTGGAGGCGGTTCA | 74 |
| | | BTP16 | AAATGGAAGACACCAAAGGTGCGCGTT | 80 |
| | | Linker | GGCGGAGGTGGCTCT | 76 |
| | | BTP16 | AAGTGGAAAACCCCGAAAGTTCGTGTGTG A | 39 |
| | Protein | IgG4 Hinge | PSCP | 14 |
| | | IgG4 CH2 | APEFLGGPSVFLFPPKPKDTLMISRTPEVTCV VVDVSQEDPEVQFNWYVDGVEVHNAKTKP REEQFNSTYRVVSVLTVLHQDWLNGKEYK CKVSNKGLPSSIEKTISKAK | 15 |
| | | IgG4 CH3 | GQPREPQVYTLPPSQEEMTKNQVSLTCLVK GFYPSDIAVEWESNGQPENNYKTTPPVLDS DGSFFLYSRLTVDKSRWQEGNVFSCSVMHE ALHNHYTQKSLSLSLGK | 16 |
| | | Linker | GGGGS | 77 |
| | | BTP16 | KWKTPKVRV | 40 |
| | | Linker | GGGGS | 77 |
| | | BTP16 | KWKTPKVRV | 40 |
| Fc-BTP27 (BTP16 3x) | DNA | IgG4 Hinge | ATGCCATCATGCCCA | 11 |
| | | IgG4 CH2 | GCACCTGAGTTCCTGGGGGGACCATCAGT CTTCCTGTTCCCCCCAAAACCCAAGGACA CCCTCATGATCTCCCGGACCCCTGAGGTC ACATGCGTGGTGGTGGACGTGAGCCAGGA AGACCCTGAGGTCCAGTTCAACTGGTACG TGGACGGCGTGGAGGTGCATAATGCCAAG ACAAAGCCGCGGGAGGAGCAGTTCAACA GCACGTACCGTGTGGTCAGCGTCCTCACC GTCCTGCACCAGGACTGGCTGAATGGCAA GGAGTACAAGTGCAAGGTCTCCAACAAA GGCCTCCCATCCTCCATCGAGAAAACCAT CTCCAAAGCCAAA | 12 |
| | | IgG4 CH3 | GGGCAGCCCCGAGAACCACAGGTGTACA CCCTGCCCCCATCCCAGGAGGAGATGACC AAGAACCAGGTCAGCCTGACCTGCCTGGT CAAAGGCTTCTATCCCAGCGACATCGCCG TGGAGTGGGAGAGCAATGGGCAGCCGGA GAACAACTACAAGACCACGCCTCCCGTGC TGGACTCCGACGGCTCCTTCTTCCTCTACA GCAGGCTAACCGTGGACAAGAGCAGGTG GCAGGAGGGGAACGTCTTCTCATGCTCCG TGATGCATGAGGCTCTGCACAACCACTAC ACGCAGAAGAGCCTCTCCCTGTCTCTGGG TAAA | 13 |
| | | Linker | GGTGGAGGCGGTTCA | 74 |
| | | BTP16 | AAATGGAAGACACCAAAGGTGCGCGTT | 80 |
| | | Linker | GGCGGAGGTGGCTCT | 76 |
| | | BTP16 | AAGTGGAAAACCCCGAAAGTTCGTGTG | 81 |
| | | Linker | GGCGGTGGCGGATCG | 78 |
| | | BTP16 | AAATGGAAAACTCCTAAAGTACGGGTCTG A | 80 |
| | Protein | IgG4 Hinge | PSCP | 14 |
| | | IgG4 CH2 | APEFLGGPSVFLFPPKPKDTLMISRTPEVTCV VVDVSQEDPEVQFNWYVDGVEVHNAKTKP REEQFNSTYRVVSVLTVLHQDWLNGKEYK CKVSNKGLPSSIEKTISKAK | 15 |
| | | IgG4 CH3 | GQPREPQVYTLPPSQEEMTKNQVSLTCLVK GFYPSDIAVEWESNGQPENNYKTTPPVLDS DGSFFLYSRLTVDKSRWQEGNVFSCSVMHE ALHNHYTQKSLSLSLGK | 16 |
| | | Linker | GGGGS | 77 |
| | | BTP16 | KWKTPKVRV | 40 |
| | | Linker | GGGGS | 77 |
| | | BTP16 | KWKTPKVRV | 40 |
| | | Linker | GGGGS | 77 |
| | | BTP16 | KWKTPKVRV | 40 |

### Example 7: Expression of fusion proteins in which immunoglobulin Fc region and peptide for brain targeting are fused

The expression of fusion proteins, in which an immunoglobulin Fc region and the peptides for brain targeting were linked, was performed under the control of the *T7* promoter of *pET22b* vector (Novagen). *E. coli BL21-DE3 (E. coli B F-dcm ompT hsdS (rB⁻mB⁻) gal λDE3*); Novagen) was transformed using each of the constructed expression vectors. The transformation was performed according to the method recommended by Novagen. Single colonies, in which each recombinant expression vector was transformed, were collected and inoculated into 2× Luria broth (LB) media containing ampicillin (50 µg/mL) and cultured at 37°C for 15 hours. The recombinant strain culture broth and 2× LB media containing 30% glycerol were mixed at a 1:1 ratio (v/v), and each 1 mL of the mixture was aliquoted into cryotubes and stored at -140°C, and the resultant was used as a cell stock for the production of recombinant fusion proteins.

For the expression of conjugates in which an immunoglobulin Fc region and a peptide for brain targeting are fused, each vial of the cell stock was dissolved, inoculated into 500 mL of 2× Luria broth (LB), and cultured in a shaking water bath at 37°C for 14 to 16 hours. When the OD at 600 nm reached 4.0 or higher, the cultivation was terminated and the resulting culture was used as a seed culture medium. The seed culture medium (1.6 L) was inoculated into a fermentation medium and the initial batch fermentation was begun. The cultivation conditions were 37°C, air supply (2 L/minute; 1 vvm), and stirring speed (650 rpm), and the pH was maintained at 6.70 using a 20% aqueous ammonia solution. With regard to the progress of the fermentation, when the nutrients in the culture media were limited, fed-batch culture was processed by adding a feeding solution. The bacterial growth was monitored based on OD values and when the OD value reached 120 or higher, IPTG was introduced thereto to a final concentration of 0.5 mM. The culture was processed further for about 23 to 25 hours after the introduction. Upon completion of the cultivation, the recombinant strains were collected by a centrifuge and stored at -80°C until use.

### Example 8: Recovery and refolding of fusion proteins in which immunoglobulin Fc region and peptide for brain targeting are fused

Cells were disrupted and refolded to convert the fusion proteins expressed in Example 7, in which each of immunoglobulin Fc regions and each of the peptides for brain targeting were fused, into a soluble form. Cell pellets corresponding to 200 mL of each cell culture broth was resuspended in 200 mL of a lysis buffer (50 mM Tris, pH 9.0, 1 mM EDTA (pH 8.0), 0.2 M NaCl, and 0.5% Triton X-100). The cells were disrupted at a pressure of 15,000 psi using the microfluidizer processor M-110EH (Model M1475C, AC Technology Corp.). The disrupted cell lysate was centrifuged at 12,200×g at 4°C for 30 minutes and the supernatant was discarded and resuspended in 200 mL of lysis buffer (0.5% Triton X-100 and 1 mM EDTA (pH 8.0)). The pellet was resuspended in 200 mL of wash buffer (20 mM Tris (pH 8.0)) after centrifugation at 12,200×g at 4°C for 30 minutes and then centrifuged in the same manner. The pellet was resuspended in 100 mL of buffer (8 M guanidine-HCl and 50 mM Tris (pH 8.0)) and stirred at room temperature for 2 hours. After 2 hours, the resultant was centrifuged at 12,200×g at 23°C for 30 minutes and the supernatant was collected. Then, 2.5 mM dithiothreitol was added thereto and the mixture was stirred at 37°C for 30 minutes. For the refolding of fusion proteins, in which solubilized immunoglobulin Fc regions and the peptides for brain targeting were fused, 3.5 L of buffer (3 M urea, 1 mM cysteine, 0.160 M arginine, 50 mM Tris (pH 9.2), and 20% glycerol) was added thereto at a flow rate of 1 mL/min using a peristaltic pump while stirring at 4°C for 36 hours. The immunoglobulin Fc regions within the fusion proteins, in which solubilized immunoglobulin Fc regions and the peptides for brain targeting were fused, were expressed as dimers.

### Example 9: Purification by affinity chromatography

Upon completion of refolding, samples were centrifuged at 12,200×g at 4°C for 30 minutes and the supernatant was collected and filtered with a Satorius filter (0.22 µm), and the pH of the samples was adjusted to pH 7.5 with 50% HCl. Samples were allowed to bind to a Protein A-sepharose (GE healthcare) column equilibrated with 10 mM Tris (pH 7.0) buffer, and then conjugate proteins in which the immunoglobulin Fc regions and the peptides for brain targeting were fused were eluted.

### Example 10: Purification by hydrophobic chromatography

After introducing 0.6 M ammonium sulfate into the eluted samples, the samples introduced with 0.6 M ammonium sulfate were allowed to bind to a phenyl HP (GE healthcare) column equilibrated using 10 mM Tris (pH 7.0) buffer and 0.6 M ammonium sulfate as buffer, fusion proteins, in which the immunoglobulin Fc regions and the peptides for brain targeting were fused, were eluted using 10 mM Tris (pH 7.5) buffer in a linear concentration gradient from 0% to 100% with 6 column volumes.

### Example 11: Confirmation of brain distribution of fusion protein in which immunoglobulin Fc region and a peptide for brain targeting are linked

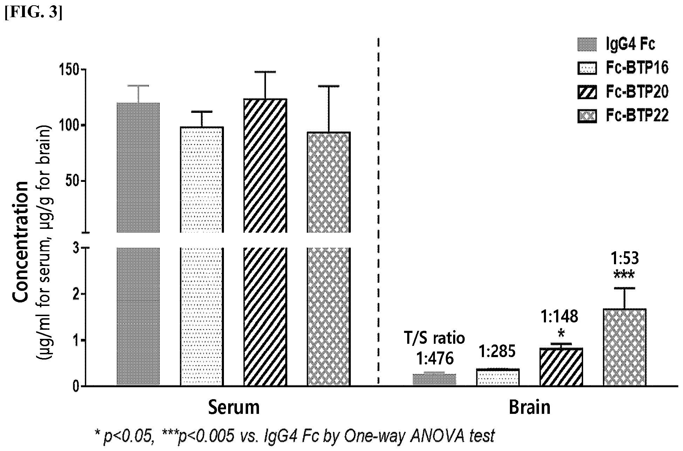

A brain distribution test was performed in mice to determine the level of blood-brain barrier passage of fusion proteins, in which the immunoglobulin Fc regions and the peptides for brain targeting were linked. Mice were administered via intravenous injection at a concentration of 10 mg/kg. After two hours, the brains of the mice were separated and the concentration of the fusion proteins, in which the immunoglobulin Fc regions and the peptides for brain targeting were linked, was measured and thereby the level of blood-brain barrier passage of the fusion proteins was confirmed (FIG. 3).

As a result, the levels of blood-brain barrier passage of the immunoglobulin Fc regions and the peptides for brain targeting measured were shown to be higher compared to those of immunoglobulin Fc regions, and the concentrations of the immunoglobulin Fc regions and the peptides for brain targeting were higher compared to those of immunoglobulin Fc regions in the brain than in the serum. It was confirmed that, in the case a fusion protein where two or more of the same kinds of peptides for brain targeting are linked to an immunoglobulin Fc region, the concentrations was measured higher compared to when only one peptide for brain targeting was linked, and was also shown to have a higher T/S ratio.

These results suggest that the fusion proteins of the present invention, in which the immunoglobulin Fc regions and the peptide for brain targeting can effectively pass through the BBB and delivered into the brain. Furthermore, these results suggest that the fusion proteins of the present invention in the form of a long-acting conjugate increase their *in vivo* duration, and as the duration increases the targeting into the brain can be more effectively performed.

Hereinafter, further experiments were performed to confirm whether the long-acting fusion proteins of the present invention, even in a case where physiologically active materials are linked to the long-acting fusion proteins in which immunoglobulin Fc regions and peptides for brain targeting are linked, can also be effectively delivered to the brain by passing through the BBB and thereby exhibit physiological activity.

### Example 12: Preparation of conjugate comprising idursulfase and PEG

A long-acting conjugate for brain targeting containing idursulfase (approximately 72 kDa) was prepared as an example of a long-acting conjugate for brain targeting that contains a typical size of protein as a physiologically active material. As a precursor of the long-acting conjugate for brain targeting, a mono-pegylated conjugate in which a polyethylene glycol was linked as a linker to idursulfase was prepared first as follows.

After concentrating idursulfase (Shire, Ireland) to an appropriate concentration, for the pegylation of 10 K butyl-ALD(2) PEG (10 kDa polyethylene glycol where the hydrogen at both ends is each modified to butyraldehyde, NOF, Japan), a reaction was performed at 4°C for about 2 hours, under the conditions where the molar ratio of idursulfase to 10 K butyl-ALD(2) PEG was set at 1:10 and the idursulfase concentration was set at 10 mg/mL. In particular, the reaction was performed by adding sodium cyanoborohydride (NaBH₃CN), *i.e.*, a reducing agent, to 0.1 M potassium phosphate butter (pH 6.0). The idursulfase-10 K PEG, which is a mono-pegylated conjugate, was purified by applying the reactants to the Source 15Q column (GE, USA) using potassium phosphate buffer (pH 6.0) and a concentration gradient of NaCl.

### Example 13: Preparation of idursulfase-10 kDa PEG-Fc-BTP22 conjugate which is a long-acting conjugate for brain targeting

Then, a long-acting conjugate for brain targeting was prepared by linking an idursulfase-10 kDa PEG conjugate to an Fc fragment containing a peptide for brain targeting. A reaction was performed 25°C for 15 hours by setting the molar ratio of idursulfase-10 K PEG to Fc-BTP22 (*i.e.*, a representative example of a fusion protein for brain targeting) at 1:10 and the idursulfase concentration at 10 mg/mL. In particular, Dulbecco's phosphate buffer (DPBS) and 10 mM NaBH₃CN as a reducing agent were added as reactants.

Upon completion of the reaction, the reactants were applied to the Source 15Q column (GE, USA) using sodium phosphate buffer (pH 6.0) and a concentration gradient of NaCl, and then, applied to the Source 15ISO column (GE, USA) using a concentration gradient of ammonium sulfate and sodium phosphate (pH 6.0), and thereby idursulfase-10 K PEG-Fc-BTP22, which is the intended long-acting conjugate for brain targeting, was purified. The idursulfase-10 K PEG-Fc-BTP22 can be used interchangeably with idursulfase-Fc-BTP22.

### Example 14: Preparation of conjugate comprising GIP derivative and PEG

A long-acting conjugates for brain targeting of an artificial GIP derivative having glucose-dependent insulinotropic polypeptide (GIP) receptor-stimulating activity, which consists of 40 amino acid residues, was prepared as an example of long-acting conjugates for brain targeting that contains a peptide of a typical size as a physiologically active material. As a precursor of the long-acting conjugate, a mono-pegylated conjugate, to which polyethylene glycol as a linker is linked to the artificial GIP derivative, was prepared as follows.

For the pegylation of 10 K MAL-PEG-ALD (10 kDa polyethylene glycol where the hydrogen atom at one end is modified to 3-[*N*-(3-*N*-maleimido-1-oxoprolyl)amino]propyl group and the hydrogen atom at the opposite end is modified to 3-oxopropyl group (propylaldehyde group), NOF, Japan) to the C-terminal cysteine of the GIP derivative, a reaction was performed at 4°C for about 1 hour, under the conditions where the molar ratio of GIP derivative to PEG was set at 1:1.3 and the GIP derivative concentration was set at 3 mg/mL. In particular, the reaction was performed in a solvent (50 mM Tris-HCl (pH 7.5) + 45% isopropanol). The GIP derivative-10 K PEG, which is a mono-pegylated conjugate, was purified using the SP HP (GE, USA) column, which utilizes sodium citrate buffer (pH 3.0) + 45% ethanol and a concentration gradient of KCl.

### Example 15: Preparation of GIP derivative-10 K PEG-Fc-BTP22a conjugate

Then, a long-acting conjugate for brain targeting was prepared by linking a GIP derivative-10 K PEG conjugate to an Fc fragment containing a peptide for brain targeting. A reaction was performed at 4°C for 16 hours by setting the total reaction concentration at 4.55 mg/mL, while setting the molar ratio of the GIP derivative-10 K PEG (purified in Example 14) to the Fc-BTP22 at 1:2. In particular, 20 mM NaBH₃CN as a reducing agent was added to the reactants.

Upon completion of the reaction, the reactants were applied to the Source 15ISO column (GE, USA) using sodium phosphate buffer (pH 7.5) and a concentration gradient of ammonium sulfate, and then, applied again to the same column, and thereby the GIP derivative-10 K PEG-Fc-BTP22, which is the intended long-acting conjugate for brain targeting, was purified. The GIP derivative-10 K PEG-Fc-BTP22 can be used interchangeably with GIP derivative-Fc BTP22.

### Example 16: Analysis of transcytosis of long-acting conjugates for brain targeting

A transcytosis experiment was performed for confirming the passage level of the long-acting conjugates for brain targeting obtained in Examples through the blood-brain barrier. The *hCMEC*/*D3* cells, which are human cerebral endothelial cells, were cultured on a microporous membrane at a concentration of 5 × 10⁴ cells/cm² for 10 days. To confirm the BBB formation, a penetration test was performed with FITC-Dextran (70 kDa) and thereby the formation of tight junctions was confirmed. Then, 25 mM long-acting conjugates were placed on *hCMEC*/*D3* cells. After 2 hours and 4 hours, the protein concentration was measured using the ELISA kit.

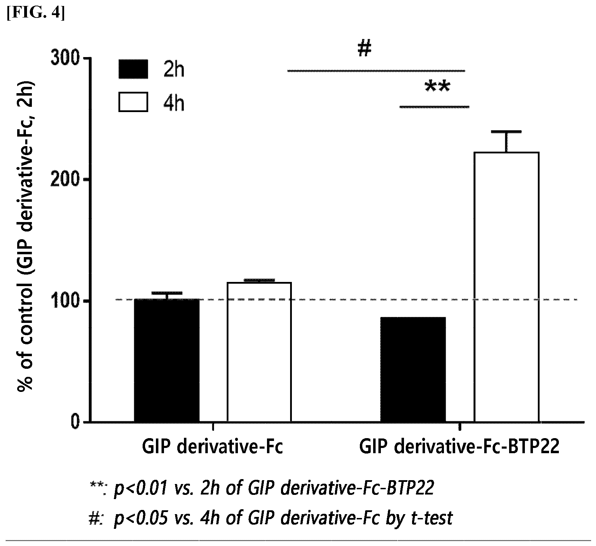

As a result, GIP derivative-10 K PEG-Fc-BTP22 showed a higher level of passage through the blood-brain barrier compared to the control group, and it was confirmed that the passage level of the GIP derivative-10 K PEG-Fc-BTP22 increased with time (FIG. 4). A similar passage level was observed for the idursulfase-10 K PEG-Fc-BTP22 (data not shown).

These results suggest that the brain targeting long-acting conjugate confirmed in the present invention could effectively pass through the BBB.

### Example 17: Measurement of activity of idursulfase-10 kDa PEG-Fc-BTP22 conjugate

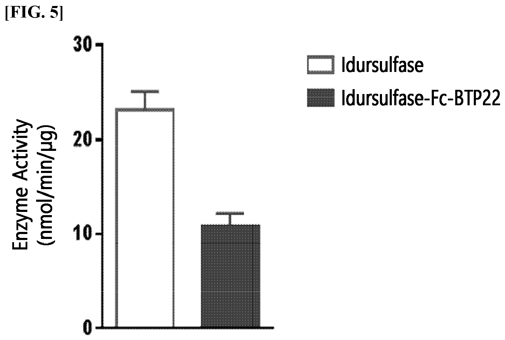

To examine the presence of any change in specific activity of idursulfase according to the preparation of idursulfase-10 K PEG-Fc-BTP22 conjugate, an *in vitro* experiment for specific activity of enzymes was performed. 4-Methylumbelliferyl α-L-idopyranosiduronic acid-2-sulfate sodium salt (4MU-α-idopyraA-2), which is known as an enzyme substrate for idursulfase, was reacted with idursulfase β and IDS-Fc-BTP22 at 37°C for 4 hours, and then, reacted with α-iduronidase, which is a secondary reaction enzyme, at 37°C for 24 hours. The enzyme activity of 4-methylumbelliferone (4 MU), the final product, was measured by the measurement of the fluorescence of the corresponding substance (FIG. 5).

As a result, the enzyme activity of free idursulfase and idursulfase-10 K PEG-Fc-BTP22 were shown to be 23.4 ± 1.94 nmol/min/µg and 10.8 ± 1.39 nmol/min/µg, respectively. In particular, the values of enzyme activity were calculated in terms of specific activity per converted rate of weight (µg). That is, in the case of free idursulfase, the value was obtained by dividing the number of enzyme reactions by weight, and in the case of idursulfase-10 K PEG-Fc-BTP22, the value was obtained by dividing the number of enzyme reactions by the converted rate of weight possessed by the idursulfase part within the conjugate. Conclusively, the enzyme activity of idursulfase-10 K PEG-Fc-BTP22 was 46.6% and reduced slightly in part, compared to that of idursulfase. However, it was confirmed that even when the enzyme was prepared as the long-acting conjugate for brain targeting of the present invention, a significant enzyme activity was observed in the corresponding conjugate.

Due to the peptide for brain targeting linked to an Fc region, the long-acting conjugate prepared above can pass through the blood-brain barrier, maintain the physiological activity of the linked physiologically active material, and thus can provide a long-acting conjugate with improved duration and stability thereby being capable of providing a platform for the development of novel therapeutic agents enabling passage through the BBB.

From the foregoing, a skilled person in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

## Claims

1. A long-acting conjugate for brain targeting of Formula 1:
[Formula 1] X-L₁-F-L₂-Y
wherein:
X is a physiologically active material;
Y is a peptide for brain targeting;
L₁ and L₂ are peptide linkers or non-peptide linkers, in which when L₁ and L₂ are peptide linkers, the peptide linkers comprise 0 to 1,000 amino acids; and
F is an immunoglobulin constant region comprising an FcRn-binding region.

2. The long-acting conjugate for brain targeting of claim 1, wherein the long-acting conjugate passes through the blood-brain barrier thereby delivering a physiologically active material into the brain tissue.

3. The long-acting conjugate for brain targeting of claim 1, wherein the peptide for brain targeting comprises a peptide, protein, or antibody, and wherein each of the peptide, protein, or antibody comprises an amino acid sequence allowing passage through the blood-brain barrier.

4. The long-acting conjugate for brain targeting of claim 3, wherein the peptide for brain targeting passes through the blood-brain barrier through a pathway by passive transport or a pathway by receptor-mediated transport.

5. The long-acting conjugate for brain targeting of claim 1, wherein the physiologically active material is selected from the group consisting of toxins; or glucagon like peptide-1 (GLP-1) receptor agonists; glucagon receptor agonists; gastric inhibitory polypeptide (GIP) receptor agonists; fibroblast growth factor (FGF) receptor agonists; cholecystokinin receptor agonists; gastrin receptor agonists; melanocortin receptor agonists; human growth hormone; growth hormone-releasing hormone; growth hormone-releasing peptide; interferons; interferon receptors; colony-stimulating factors; interleukins; interleukin receptors; enzymes; interleukin-binding proteins; cytokine-binding proteins; macrophage-activating factors; macrophage peptides; B cell factors; T cell factors; protein A; allergy-inhibiting factors; necrosis glycoproteins; immunotoxins; lymphotoxins; tumor necrosis factors; tumor suppressors; transforming growth factors; α-1 antitrypsin; albumin; α-lactalbumin; apolipoprotein-E; erythropoietin; high-glycosylated erythropoietin; angiopoietins; hemoglobins; thrombin; thrombin receptor-activating peptide; thrombomodulin; blood coagulation factor VII; blood coagulation factor VIIa; blood coagulation factor VIII; blood coagulation factor IX; blood coagulation factor XIII; plasminogen activators; fibrin-binding peptides; urokinase; streptokinase; hirudin; protein C; C-reactive protein; renin inhibitor; collagenase inhibitors; superoxide dismutase; leptin; platelet-derived growth factor; epithelial growth factor; epidermal growth factor; angiostatin; angiotensin; bone morphogenetic growth factor; bone morphogenetic protein; calcitonin; insulin; atriopeptin; cartilage-inducing factor; elcatonin; connective tissue-activating factor; tissue factor pathway inhibitor; follicle-stimulating hormone; luteinizing hormone; luteinizing hormone-releasing hormone; nerve growth factors; axogenesis factor-1; brain-natriuretic peptide; glial-derived neurotrophic factor; netrin; neutrophil inhibitory factor; neurotrophic factor; neurturin; parathyroid hormone; relaxin; secretin; somatomedin; insulin-like growth factor; adrenocortical hormone; glucagon; cholecystokinin; pancreatic polypeptides; gastrin-releasing peptides; corticotropin-releasing factor; thyroid-stimulating hormone; autotaxin; lactoferrin; myostatin; activity-dependent neuroprotective protein (ADNP), β-secretase1 (BACE1), amyloid precursor protein (APP), neural cell adhesion molecule (NCAM), amyloid β, tau, receptor for advanced glycation endproducts (RAGE), α-synuclein, or agonists or antagonists thereof; receptors, receptor agonists; cell surface antigens; monoclonal antibody; polyclonal antibody; antibody fragments; virus-derived vaccine antigens; hybrid polypeptides or chimeric polypeptides that activate at least one receptor agonist; and analogues thereof.

6. The long-acting conjugate for brain targeting of claim 5, wherein:
the toxin is selected from the group consisting of maytansine or a derivative thereof, auristatin or a derivative thereof, duocarmycin or a derivative thereof, and pyrrolobenzodiazepine (PBD) or a derivative thereof;
the glucagon like peptide-1 (GLP-1) receptor agonist is selected from the group consisting of native exendin-3 or native exendin-4, and analogues thereof;
the FGF receptor agonist is selected from the group consisting of FGF1 or an analogue thereof, FGF19 or an analogue thereof, FGF21 or an analogue thereof, and FGF23 or an analogue thereof;
the interferon is selected from the group consisting of interferon-a, interferon-β, and interferon-γ;
the interferon receptor is selected from the group consisting of interferon-a receptor, interferon-β receptor, interferon-γ receptor, and soluble type I interferon receptors;
the interleukin is selected from the group consisting of interleukin-1, interleukin-2, interleukin-3, interleukin-4, interleukin-5, interleukin-6, interleukin-7, interleukin-8, interleukin-9, interleukin-10, interleukin-11, interleukin-12, interleukin-13, interleukin-14, interleukin-15, interleukin-16, interleukin-17, interleukin-18, interleukin-19, interleukin-20, interleukin-21, interleukin-22, interleukin-23, interleukin-24, interleukin-25, interleukin-26, interleukin-27, interleukin-28, interleukin-29, and interleukin-30;
the interleukin receptor is interleukin-1 receptor or interleukin-4 receptor;
the enzyme is selected from the group consisting of β-glucosidase, α-galactosidase, β-galactosidase, iduronidase, iduronate-2-sulfatase, galactose-6-sulfatase, acid α-glucosidase, acid ceramidase, acid sphingomyelinsase, galactocerebrosidsase, arylsulfatase A, arylsulfatase B, β-hexosaminidase A, β-hexosaminidase B, heparin *N*-sulfatase, α-D-mannosidase, β-glucuronidase, *N*-acetylgalactosamine-6 sulfatase, lysosomal acid lipase, α-*N*-acetyl-glucosaminidase, glucocerebrosidase, butyrylcholinesterase, chitinase, glutamate decarboxylase, imiglucerase, lipase, uricase, platelet-activating factor acetylhydrolase, neutral endopeptidase, myeloperoxidase, α-galactosidase-A, agalsidase α, agalsidase β, α-L-iduronidase, butyrylcholinesterase, chitinase, glutamate decarboxylase, and imiglucerase;
the interleukin-binding protein is IL-18bp;
the cytokine-binding protein is TNF-binding protein;
the nerve growth factors are selected from the group consisting of nerve growth factor, ciliary neurotrophic factor, axogenesis factor-1, brain-natriuretic peptide, glial-derived neurotrophic factor, netrin, neutrophil inhibitory factor, neurotrophic factor, and neurturin;
the myostatin receptor is selected from the group consisting of TNFR (P75), TNFR (P55), IL-1 receptor, VEGF receptor, and B cell activating factor receptor;
the myostatin receptor antagonist is IL1-Ra;
the cell surface antigen is selected from the group consisting of CD2, CD3, CD4, CD5, CD7, CD11a, CD11b, CD18, CD19, CD20, CD23, CD25, CD33, CD38, CD40, CD45, and CD69; and
the antibody fragments are selected from the group consisting of scFv, Fab, Fab', F(ab')₂, and Fd.

7. The long-acting conjugate for brain targeting of claim 6, wherein the GLP-1 receptor agonist is selected from the group consisting of a native exendin-4; an exendin-4 derivative in which the N-terminal amine group of exendin-4 is deleted; an exendin-4 derivative in which the N-terminal amine group of exendin-4 is substituted with a hydroxyl group; an exendin-4 derivative in which the N-terminal amine group of exendin-4 is modified with a dimethyl group; an exendin-4 derivative in which the N-terminal amine group of exendin-4 is substituted with a carboxyl group; an exendin-4 derivative in which the α-carbon of the 1^{st} amino acid of exendin-4, histidine, is deleted; an exendin-4 derivative in which the 12^{th} amino acid of exendin-4, lysine, is substituted with serine, and an exendin-4 derivative in which the 12^{th} amino acid of exendin-4, lysine, is substituted with arginine.

8. The long-acting conjugate for brain targeting of claim 4, wherein, in the pathway by receptor-mediated transport, the long-acting conjugate for brain targeting passes through the blood-brain barrier by the receptor-mediated transport pathway through any one selected from the group consisting of insulin receptor, transferrin receptor, low-density lipoprotein receptor, low-density lipoprotein receptor-related protein, leptin receptor, nicotinic acetylcholine receptor, glutathione transporter, calcium-activated potassium channel, and receptor for advanced glycation endproducts (RAGE), and ligands of the receptors and antibody binding to the receptors or ligands.

9. The long-acting conjugate for brain targeting of claim 1, wherein L₁ is linked to the N-terminal region of F, and L₂ is linked to the C-terminal region of F.

10. The long-acting conjugate for brain targeting of claim 1, wherein the F-L₂-Y of Formula 1 is represented by the following Formula 2: 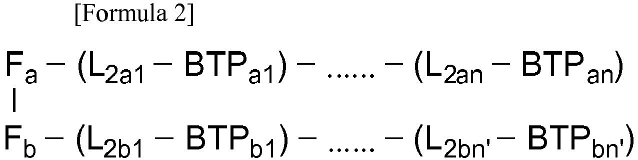 wherein:
Fₐ and F_{b} are each a single-stranded polypeptide chain, which comprises an immunoglobulin Fc region comprising a hinge region, CH2 domain, and CH3 domain, in which Fₐ and F_{b} are linked by a disulfide bond in the hinge region and thereby comprise an Fc fragment, and Fₐ and L₁ are covalently bonded with each other;
each of BTPₐ₁, ......, BTPₐₙ, being the same as or different from one another, is a peptide for brain targeting;
each of BTP_{b1}, ......, BTP_{bn'}, being the same as or different from one another, is a peptide for brain targeting;
each of L₂ₐ₁, ......, L₂ₐₙ, being the same as or different from one another, is a peptide linker; and
each of L_{2b1}, ......, L_{2bn'}, being the same as or different from one another, is a peptide linker; wherein n and n' are each independently an integer.

11. The long-acting conjugate for brain targeting of claim 10, wherein L₁ is linked to the N-terminus of Fₐ, and L₂ₐ₁ and L_{2b1} are each linked to the C-terminus of Fₐ and F_{b}, respectively.

12. The long-acting conjugate for brain targeting of claim 11, wherein n = n', and each meets the conditions of L₂ₐ₁ = L_{2b1}, ......, L₂ₐₙ = L_{2bn'} and BTPₐ₁ = BTP_{b1}, ......, BTPₐₙ = BTP_{bn'}.

13. The long-acting conjugate for brain targeting of claim 10, wherein L₁ is linked to the N-terminal amine group of X, the amine group located at a side chain of a lysine residue, or the -SH group (thiol group) located at a side chain of a cysteine residue.

14. The long-acting conjugate for brain targeting of claim 10, wherein X, which is a physiologically active material, is a polypeptide consisting of 2 to 1,000 amino acids.

15. The long-acting conjugate for brain targeting of claim 10, wherein n and n' are integers from 1 to 5.

16. The long-acting conjugate for brain targeting of claim 10, wherein L₁ is a non-peptide linker having a size of 0.5 kDa to 100 kDa.

17. The long-acting conjugate for brain targeting of claim 16, wherein the non-peptide linker is polyethylene glycol.

18. The long-acting conjugate for brain targeting of claim 1, wherein L₂, being a peptide linker, is (GS)ₘ, (GGS)ₘ, (GGGS)ₘ, or (GGGGS)ₘ, in which m is 1 to 10.

19. The long-acting conjugate for brain targeting of claim 1, wherein one of L₁ and L₂ is a peptide linker and the other is a non-peptide linker.

20. The long-acting conjugate for brain targeting of claim 1, wherein when L₁ is a non-peptide linker and L₂ is a peptide linker, the peptide linker comprises 0 to 1,000 amino acids.

21. The long-acting conjugate for brain targeting of claim 1 or 10, wherein when any one or both of L₁ and L₂ are a non-peptide linker, the non-peptide linker is selected from the group consisting of polyethylene glycol, polypropylene glycol, an ethylene glycol-propylene glycol copolymer, polyoxyethylated polyol, polyvinyl alcohol, a polysaccharide, dextran, polyvinyl ethyl ether, a biodegradable polymer, a lipid polymer, chitins, hyaluronic acid, a fatty acid, a high molecular weight polymer, a low molecular weight compound, a nucleotide, and a combination thereof.

22. The long-acting conjugate for brain targeting of claim 1, wherein when any one or both of L₁ and L₂ are a peptide linker, X and F, or F and Y are linked to each other by L₁ and L₂ via a covalent chemical bond, non-covalent chemical bond, or a combination thereof; and L₁ and L₂ each comprise 0 to 1,000 amino acids.

23. The long-acting conjugate for brain targeting of claim 1, wherein when any one or both of L₁ and L₂ are a peptide linker, L₁ and L₂ each comprise 0 to 1,000 amino acids, wherein:
(i) X and F, or F and Y are linked by a peptide bond; or
(ii) X and F, and F and Y are linked by a peptide bond.

24. The long-acting conjugate for brain targeting of claim 1, wherein when any one of L₁ and L₂ is a peptide linker and the other of the two is a non-peptide linker, the peptide linker is a linker comprising 0 to 1,000 amino acids and the non-peptide linker is polyethylene glycol.

25. The long-acting conjugate for brain targeting of claim 1, wherein F comprises an immunoglobulin Fc region.

26. The long-acting conjugate for brain targeting of claim 25, wherein the immunoglobulin Fc region comprises one to four domains selected from the group consisting of CH1, CH2, CH3, and CH4 domains.

27. The long-acting conjugate for brain targeting of claim 25, wherein the immunoglobulin Fc region further comprises a hinge region.

28. The long-acting conjugate for brain targeting of claim 10 or 25, wherein the immunoglobulin Fc region is an Fc region of IgG.

29. The long-acting conjugate for brain targeting of claim 28, wherein the immunoglobulin Fc region is an IgG4 Fc region.

30. The long-acting conjugate for brain targeting of claim 29, wherein the immunoglobulin Fc region is an aglycosylated IgG4 Fc region of a human sequence.

31. The long-acting conjugate for brain targeting of claim 1, wherein the N-terminus of X and the N-terminus of F are linked by L₁ and the N-terminus of Y and the C-terminus of F are linked by L₂.

32. The long-acting conjugate for brain targeting of claim 1, wherein:
an end of L₁ is linked to a lysine residue or cysteine residue of X and the other end of L₁ is linked to the N-terminus of F; and
the N-terminus of Y and the C-terminus of F are linked by L₂.

33. A method for preparing the long-acting conjugate for brain targeting of claim 1, comprising:
(i) preparing:
(a) X-L₁-F, wherein X, which is a physiologically active material, L₁, which is a peptide or non-peptide linker, and F comprising an immunoglobulin Fc region, are linked; and
(b) L₂-Y, wherein Y, which is a peptide for brain targeting, and L₂, which is a peptide or non-peptide linker, are linked; and
(ii) linking (a) X-L₁-F and (b) L₂-Y.

34. A method for preparing the long-acting conjugate for brain targeting of claim 1, comprising:
(i) preparing:
(a) X-L₁, wherein X, which is a physiologically active material, and L₁, which is a peptide or non-peptide linker, are linked; and
(b) F-L₂-Y, wherein Y, which is a peptide for brain targeting, L₂, which is a peptide or non-peptide linker, and F comprising an immunoglobulin Fc region, are linked; and
(ii) linking (a) X-L₁ and (b) F-L₂-Y.

35. A method for preparing the long-acting conjugate for brain targeting of claim 1, comprising:
(i) preparing F-L₂-Y, wherein Y, which is a peptide for brain targeting, L₂, which is a peptide or non-peptide linker, and F comprising an immunoglobulin Fc region are linked; and
(ii) linking F-L₂-Y and X by L₁, which is a peptide or non-peptide linker.

36. The method of any one of claims 33 to 35, wherein Li of (a) is a peptide linker, and L₂ of (b) is a non-peptide linker.

37. The method of any one of claims 33 to 35, wherein Li of (a) is a non-peptide linker, and L₂ of (b) is a peptide linker.

38. The long-acting conjugate for brain targeting of claim 1, wherein Li is a non-peptide linker having a functional group at both ends, in which the functional group at both ends is each an amine-reactive group or thiol-reactive group, and the functional groups at both ends are the same kind as each other or different kinds from each other.

39. The method of any one of claims 33 to 35, wherein a non-peptide linker and X and Y are linked by each of their functional groups, in which the functional group of the non-peptide linker is selected from the group consisting of an aldehyde group, a maleimide group, and a succinimide derivative.

40. A peptide for brain targeting consisting of any one amino acid sequence selected from amino acid sequences of SEQ ID NOS: 2, 4, 6, 8, 10, 20, 22, 24, 26, 28, 30, 32, 34, 36, 38, and 40.

41. A separated polynucleotide encoding the peptide for brain targeting of claim 40.

42. A recombinant expression vector comprising the polynucleotide of claim 41.

43. A transformant comprising the recombinant expression vector of claim 42.


<110>     HANMI PHARM. CO., LTD.

<120>     Long-acting protein conjugates for brain targeting

<130>     OPA17345-PCT

<150>     KR 10-2016-0173863
<151>     2016-12-19

<150>     KR 10-2017-0126537
<151>     2017-09-28

<160>     81

<170>     KoPatentIn 3.0

<210>     1
<211>     21
<212>     DNA
<213>     Artificial Sequence

<220>
<223>     BTP1


<400>     1
catgcaattt atccgcgtca t                                                  21


<210>     2
<211>     7
<212>     PRT
<213>     Artificial Sequence

<220>
<223>     BTP1


<400>     2
His Ala Ile Tyr Pro Arg His
  1               5


<210>     3
<211>     36
<212>     DNA
<213>     Artificial Sequence

<220>
<223>     BTP2


<400>     3
acccatcgtc cgccgatgtg gagcccggtt tggccg                                  36


<210>     4
<211>     12
<212>     PRT
<213>     Artificial Sequence

<220>
<223>     BTP2

<400>    4
Thr His Arg Pro Pro Met Trp Ser Pro Val Trp Pro
1               5                   10

<210>    5
<211>    57
<212>    DNA
<213>    Artificial Sequence

<220>
<223>    BTP3

<400>    5
accttttttt atggtggcag ccgtggtaaa cgcaataact ttaaaaccga agaatat          57

<210>    6
<211>    19
<212>    PRT
<213>    Artificial Sequence

<220>
<223>    BTP3

<400>    6
Thr Phe Phe Tyr Gly Gly Ser Arg Gly Lys Arg Asn Asn Phe Lys Thr
1               5                   10                  15

Glu Glu Tyr

<210>    7
<211>    114
<212>    DNA
<213>    Artificial Sequence

<220>
<223>    BTP4

<400>    7
agcgttattg atgcactgca gtataaactg gaaggtacca cacgtctgac ccgcaaacgt          60

ggtctgaaac tggccaccgc actgtcactg agcaataaat ttgttgaagg tagc          114

<210>    8
<211>    38
<212>    PRT
<213>    Artificial Sequence

<220>
<223>    BTP4

<400>    8
Ser Val Ile Asp Ala Leu Gln Tyr Lys Leu Glu Gly Thr Thr Arg Leu

1               5                   10                  15

Thr Arg Lys Arg Gly Leu Lys Leu Ala Thr Ala Leu Ser Leu Ser Asn
            20                  25                  30

Lys Phe Val Glu Gly Ser
        35

<210>   9
<211>   54
<212>   DNA
<213>   Artificial Sequence

<220>
<223>   BTP5

<400>   9
ctgcgtaaac tgcgcaaacg tctgttactg cgtaaactgc gcaaacgtct gtta          54

<210>   10
<211>   18
<212>   PRT
<213>   Artificial Sequence

<220>
<223>   BTP5

<400>   10
Leu Arg Lys Leu Arg Lys Arg Leu Leu Leu Arg Lys Leu Arg Lys Arg
1               5                   10                  15

Leu Leu

<210>   11
<211>   15
<212>   DNA
<213>   Artificial Sequence

<220>
<223>   Hinge of IgG4

<400>   11
atgccatcat gccca                                                     15

<210>   12
<211>   330
<212>   DNA
<213>   Artificial Sequence

<220>
<223>   CH2 of IgG4

<400>   12
gcacctgagt tcctgggggg accatcagtc ttcctgttcc ccccaaaacc caaggacacc    60

ctcatgatct cccggacccc tgaggtcaca tgcgtggtgg tggacgtgag ccaggaagac 120

cctgaggtcc agttcaactg gtacgtggac ggcgtggagg tgcataatgc caagacaaag 180

ccgcgggagg agcagttcaa cagcacgtac cgtgtggtca gcgtcctcac cgtcctgcac 240

caggactggc tgaatggcaa ggagtacaag tgcaaggtct ccaacaaagg cctcccatcc 300

tccatcgaga aaaccatctc caaagccaaa 330

<210> 13
<211> 321
<212> DNA
<213> Artificial Sequence

<220>
<223> CH3 of IgG4

<400> 13
gggcagcccc gagaaccaca ggtgtacacc ctgcccccat cccaggagga gatgaccaag 60

aaccaggtca gcctgacctg cctggtcaaa ggcttctatc ccagcgacat cgccgtggag 120

tgggagagca atgggcagcc ggagaacaac tacaagacca cgcctcccgt gctggactcc 180

gacggctcct tcttcctcta cagcaggcta accgtggaca agagcaggtg gcaggagggg 240

aacgtcttct catgctccgt gatgcatgag gctctgcaca accactacac gcagaagagc 300

ctctccctgt ctctgggtaa a 321

<210> 14
<211> 4
<212> PRT
<213> Artificial Sequence

<220>
<223> Hinge of IgG4

<400> 14
Pro Ser Cys Pro
1

<210> 15
<211> 110
<212> PRT
<213> Artificial Sequence

<220>
<223> CH2 of IgG4

<400> 15
Ala Pro Glu Phe Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val

20                  25                  30

Val Val Asp Val Ser Gln Glu Asp Pro Glu Val Gln Phe Asn Trp Tyr
        35                  40                  45

Val Asp Gly Val Glu Val His Asn Ala Lys Thr Lys Pro Arg Glu Glu
    50                  55                  60

Gln Phe Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Gly Leu Pro Ser Ser Ile Glu Lys Thr Ile Ser Lys Ala Lys
            100                 105                 110


<210>    16
<211>    107
<212>    PRT
<213>    Artificial Sequence

<220>
<223>    CH3 of IgG4


<400>    16
Gly Gln Pro Arg Glu Pro Gln Val Tyr Thr Leu Pro Pro Ser Gln Glu
1               5                   10                  15

Glu Met Thr Lys Asn Gln Val Ser Leu Thr Cys Leu Val Lys Gly Phe
            20                  25                  30

Tyr Pro Ser Asp Ile Ala Val Glu Trp Glu Ser Asn Gly Gln Pro Glu
        35                  40                  45

Asn Asn Tyr Lys Thr Thr Pro Pro Val Leu Asp Ser Asp Gly Ser Phe
    50                  55                  60

Phe Leu Tyr Ser Arg Leu Thr Val Asp Lys Ser Arg Trp Gln Glu Gly
65                  70                  75                  80

Asn Val Phe Ser Cys Ser Val Met His Glu Ala Leu His Asn His Tyr
                85                  90                  95

Thr Gln Lys Ser Leu Ser Leu Ser Leu Gly Lys
            100                 105


<210>    17
<211>    45
<212>    DNA
<213>    Artificial Sequence

<220>
<223>    Linker 15a.a.


<400>    17
ggtggaggcg gttcaggcgg aggtggctct ggcggtggcg gatcg                45

<210>    18
<211>    15
<212>    PRT
<213>    Artificial Sequence

<220>
<223>    Linker 15a.a.


<400>    18
Gly Gly Gly Gly Ser Gly Gly Gly Gly Ser Gly Gly Gly Gly Ser
1               5                   10                  15


<210>    19
<211>    90
<212>    DNA
<213>    Artificial Sequence

<220>
<223>    BTP6


<400>    19
taccaacaga tcctcaccag tatgccttcc agaaacgtga tccaaatatc caacgacctg        60

gagaacctcc gggatcttct tcacgtgctg                                          90


<210>    20
<211>    30
<212>    PRT
<213>    Artificial Sequence

<220>
<223>    BTP6


<400>    20
Tyr Gln Gln Ile Leu Thr Ser Met Pro Ser Arg Asn Val Ile Gln Ile
1               5                   10                  15

Ser Asn Asp Leu Glu Asn Leu Arg Asp Leu Leu His Val Leu
            20                  25                  30


<210>    21
<211>    87
<212>    DNA
<213>    Artificial Sequence

<220>
<223>    BTP7


<400>    21
   tacaccattt ggatgcccga gaatccgaga cctgggacac cttgtgacat ttttaccaat         60
   agtagaggga agagagcatc caatggg                                             87


<210>    22
<211>    29

<212>    PRT
<213>    Artificial Sequence

<220>
<223>    BTP7


<400>    22
Tyr Thr Ile Trp Met Pro Glu Asn Pro Arg Pro Gly Thr Pro Cys Asp
1               5                   10                  15

Ile Phe Thr Asn Ser Arg Gly Lys Arg Ala Ser Asn Gly
            20                  25


<210>    23
<211>    84
<212>    DNA
<213>    Artificial Sequence

<220>
<223>    BTP8


<400>    23
aagtcagtca gaacttggaa tgagatcata ccttcaaaag ggtgtttaag agttgggggg        60

aggtgtcatc ctcatgtgaa cggg                                               84


<210>    24
<211>    28
<212>    PRT
<213>    Artificial Sequence

<220>
<223>    BTP8


<400>    24
Lys Ser Val Arg Thr Trp Asn Glu Ile Ile Pro Ser Lys Gly Cys Leu
1               5                   10                  15

Arg Val Gly Gly Arg Cys His Pro His Val Asn Gly
            20                  25


<210>    25
<211>    36
<212>    DNA
<213>    Artificial Sequence

<220>
<223>    BTP9


<400>    25
catctgaata ttctgagcac cctgtggaaa tatcgt                                  36


<210>    26
<211>    12
<212>    PRT

<213>    Artificial Sequence

<220>
<223>    BTP9


<400>    26
His Leu Asn Ile Leu Ser Thr Leu Trp Lys Tyr Arg
1               5                   10


<210>    27
<211>    90
<212>    DNA
<213>    Artificial Sequence

<220>
<223>    BTP10


<400>    27
ctgcgcaagc tgcgtaagcg gctcctccgc gatgccgatg acctgttacg taaacttcgc        60

aaacgtctgc ttcgtgacgc agacgatctg                                          90


<210>    28
<211>    30
<212>    PRT
<213>    Artificial Sequence

<220>
<223>    BTP10


<400>    28
Leu Arg Lys Leu Arg Lys Arg Leu Leu Arg Asp Ala Asp Asp Leu Leu
1               5                   10                  15

Arg Lys Leu Arg Lys Arg Leu Leu Arg Asp Ala Asp Asp Leu
            20                  25                  30


<210>    29
<211>    60
<212>    DNA
<213>    Artificial Sequence

<220>
<223>    BTP11


<400>    29
accgaggagc tgcgggtgcg cctcgcctcc cacctgcgca agctgcgtaa gcggctcctc        60

                                                                          60


<210>    30
<211>    20
<212>    PRT
<213>    Artificial Sequence

<220>
<223>    BTP11


<400>    30
Thr Glu Glu Leu Arg Val Arg Leu Ala Ser His Leu Arg Lys Leu Arg
1               5                   10                  15

Lys Arg Leu Leu
            20


<210>    31
<211>    102
<212>    DNA
<213>    Artificial Sequence

<220>
<223>    BTP12


<400>    31
ctgcgggtgc gcctcgcctc ccacctgcgc aagctgcgta agcggctcct cttacgtgtt        60

cgtctggcaa gccatcttcg taaacttcgc aaacgtctgc tt                          102


<210>    32
<211>    34
<212>    PRT
<213>    Artificial Sequence

<220>
<223>    BTP12


<400>    32
Leu Arg Val Arg Leu Ala Ser His Leu Arg Lys Leu Arg Lys Arg Leu
1               5                   10                  15

Leu Leu Arg Val Arg Leu Ala Ser His Leu Arg Lys Leu Arg Lys Arg
            20                  25                  30

Leu Leu


<210>    33
<211>    27
<212>    DNA
<213>    Artificial Sequence

<220>
<223>    BTP13


<400>    33
ggtaaaggcc cgaaatggat gcgttga                                            27


<210>    34
<211>    8
<212>    PRT

<213>    Artificial Sequence

<220>
<223>    BTP13


<400>    34
Gly Lys Gly Pro Lys Trp Met Arg
1               5


<210>    35
<211>    30
<212>    DNA
<213>    Artificial Sequence

<220>
<223>    BTP14


<400>    35
ggtcataaag caaaaggccc gcgtaaatga                                        30


<210>    36
<211>    9
<212>    PRT
<213>    Artificial Sequence

<220>
<223>    BTP14


<400>    36
Gly His Lys Ala Lys Gly Pro Arg Lys
1               5


<210>    37
<211>    30
<212>    DNA
<213>    Artificial Sequence

<220>
<223>    BTP15


<400>    37
gttattgcaa aaattaagaa accgaaatga                                        30


<210>    38
<211>    9
<212>    PRT
<213>    Artificial Sequence

<220>
<223>    BTP15


<400>    38
Val Ile Ala Lys Ile Lys Lys Pro Lys
1               5

<210>    39
<211>    30
<212>    DNA
<213>    Artificial Sequence

<220>
<223>    BTP16


<400>    39
aagtggaaaa ccccgaaagt tcgtgtgtga                                        30


<210>    40
<211>    9
<212>    PRT
<213>    Artificial Sequence

<220>
<223>    BTP16


<400>    40
Lys Trp Lys Thr Pro Lys Val Arg Val
1               5


<210>    41
<211>    19
<212>    PRT
<213>    Artificial Sequence

<220>
<223>    BTP


<400>    41
Thr Phe Phe Tyr Gly Gly Ser Arg Gly Lys Arg Asn Asn Phe Lys Thr
1               5                   10                  15

Glu Glu Tyr


<210>    42
<211>    38
<212>    PRT
<213>    Artificial Sequence

<220>
<223>    BTP


<400>    42
Ser Val Ile Asp Ala Leu Gln Tyr Lys Leu Glu Gly Thr Thr Arg Leu
1               5                   10                  15

Thr Arg Lys Arg Gly Leu Lys Leu Ala Thr Ala Leu Ser Leu Ser Asn
            20                  25                  30

Lys Phe Val Glu Gly Ser

        35

<210>    43
<211>    18
<212>    PRT
<213>    Artificial Sequence

<220>
<223>    BTP


<400>    43
Leu Arg Lys Leu Arg Lys Arg Leu Leu Leu Arg Lys Leu Arg Lys Arg
  1               5                   10                  15

Leu Leu




<210>    44
<211>    8
<212>    PRT
<213>    Artificial Sequence

<220>
<223>    BTP


<220>
<221>    MISC_FEATURE
<222>    (1)
<223>    Xaa1 is acetylated


<400>    44
Cys Met Pro Arg Leu Arg Gly Cys
  1               5


<210>    45
<211>    12
<212>    PRT
<213>    Artificial Sequence

<220>
<223>    BTP


<400>    45
Thr His Arg Pro Pro Met Trp Ser Pro Val Trp Pro
  1               5                   10


<210>    46
<211>    12
<212>    PRT
<213>    Artificial Sequence

<220>
<223>    BTP

<400>    46
Pro Trp Val Pro Ser Trp Met Pro Pro Arg His Thr
1               5                   10

<210>    47
<211>    10
<212>    PRT
<213>    Artificial Sequence

<220>
<223>    BTP

<400>    47
Cys Cys Arg Thr Ile Gly Pro Ser Val Cys
1               5                   10

<210>    48
<211>    30
<212>    PRT
<213>    Artificial Sequence

<220>
<223>    BTP

<400>    48
Tyr Gln Gln Ile Leu Thr Ser Met Pro Ser Arg Asn Val Ile Gln Ile
1               5                   10                  15

Ser Asn Asp Leu Glu Asn Leu Arg Asp Leu Leu His Val Leu
            20                  25                  30

<210>    49
<211>    29
<212>    PRT
<213>    Artificial Sequence

<220>
<223>    BTP

<400>    49
Tyr Thr Ile Trp Met Pro Glu Asn Pro Arg Pro Gly Thr Pro Cys Asp
1               5                   10                  15

Ile Phe Thr Asn Ser Arg Gly Lys Arg Ala Ser Asn Gly
            20                  25

<210>    50
<211>    16
<212>    PRT
<213>    Artificial Sequence

<220>
<223>    BTP

<400>    50

Gly Arg Glu Ile Arg Thr Gly Arg Ala Glu Arg Trp Ser Glu Lys Phe
1               5                   10                  15

<210>    51
<211>    18
<212>    PRT
<213>    Artificial Sequence

<220>
<223>    BTP

<400>    51
Cys Asn Cys Lys Ala Pro Glu Thr Ala Leu Cys Ala Arg Arg Cys Gln
1               5                   10                  15

Gln His

<210>    52
<211>    9
<212>    PRT
<213>    Artificial Sequence

<220>
<223>    BTP

<220>
<221>    MISC_FEATURE
<222>    (1)
<223>    Xaa is diaminopropionic acid

<400>    52
Xaa Lys Ala Pro Glu Thr Ala Leu Asp
1               5

<210>    53
<211>    13
<212>    PRT
<213>    Artificial Sequence

<220>
<223>    BTP

<400>    53
His Leu Asn Ile Leu Ser Thr Leu Trp Lys Tyr Arg Cys
1               5                   10

<210>    54
<211>    7
<212>    PRT
<213>    Artificial Sequence

<220>
<223>    BTP

<220>
<221>    MISC_FEATURE
<222>    (7)
<223>    O-beta-Glc is attaced thereto

<400>    54
Gly Phe Thr Gly Phe Leu Ser
1               5

<210>    55
<211>    12
<212>    PRT
<213>    Artificial Sequence

<220>
<223>    BTP

<400>    55
Thr Gly Asn Tyr Lys Ala Leu His Pro His Asn Gly
1               5                   10

<210>    56
<211>    11
<212>    PRT
<213>    Artificial Sequence

<220>
<223>    BTP

<400>    56
Tyr Gly Arg Lys Lys Arg Arg Gln Arg Arg Arg
1               5                   10

<210>    57
<211>    18
<212>    PRT
<213>    Artificial Sequence

<220>
<223>    BTP

<400>    57
Arg Gly Gly Arg Leu Ser Tyr Ser Arg Arg Arg Phe Ser Thr Ser Thr
1               5                   10                  15

Gly Arg

<210>    58
<211>    8
<212>    PRT
<213>    Artificial Sequence

<220>
<223>    BTP


<400>    58
Phe Pro Phe Pro Phe Pro Phe Pro
1               5


<210>    59
<211>    5
<212>    PRT
<213>    Artificial Sequence

<220>
<223>    Peptide linker motif


<400>    59
Gly Gly Gly Gly Ser
1               5


<210>    60
<211>    4
<212>    PRT
<213>    Artificial Sequence

<220>
<223>    Peptide linker motif


<400>    60
Gly Gly Gly Ser
1


<210>    61
<211>    4
<212>    PRT
<213>    Artificial Sequence

<220>
<223>    Peptide linker motif


<400>    61
Gly Gly Ser Gly
1


<210>    62
<211>    124
<212>    DNA
<213>    Artificial Sequence

<220>
<223>    Fc-BTP22-F


<400>    62
tctccctgtc tctgggtaaa ggtggaggcg gttcacttcg caagttacgt aaacgcttac        60

tgttacgtaa acttcggaag cgcttactgg gcggaggtgg ctctctgcgt aaactgcgca 120

aacg 124

<210> 63
<211> 124
<212> DNA
<213> Artificial Sequence

<220>
<223> Fc-BTP22-R

<400> 63
cgtttgcgca gtttacgcag agagccacct ccgcccagta agcgcttccg aagtttacgt 60

aacagtaagc gtttacgtaa cttgcgaagt gaaccgcctc cacctttacc cagagacagg 120

gaga 124

<210> 64
<211> 112
<212> DNA
<213> Artificial Sequence

<220>
<223> Fc-BTP23-F

<400> 64
aactgcgcaa acgtctgtta ggcggtggcg gatcgctgcg taaactgcgc aaacgtctgt 60

tacttcggaa gctgagaaaa cgcttacttt aaggatccga attcgagctc cg 112

<210> 65
<211> 112
<212> DNA
<213> Artificial Sequence

<220>
<223> Fc-BTP23-R

<400> 65
cggagctcga attcggatcc ttaaagtaag cgttttctca gcttccgaag taacagacgt 60

ttgcgcagtt tacgcagcga tccgccaccg cctaacagac gtttgcgcag tt 112

<210> 66
<211> 130
<212> DNA
<213> Artificial Sequence

<220>
<223> Fc-BTP24-F

<400> 66
tctccctgtc tctgggtaaa ggtggaggcg gttcaacaga agagttacgt gttcgtctgg 60

caagccattt acgtaaactt cgcaaacgtc tgttaggcgg aggtggctct accgaggagc 120

tgcgggtgcg 130

<210> 67
<211> 130
<212> DNA
<213> Artificial Sequence

<220>
<223> Fc-BTP24-R

<400> 67
cgcacccgca gctcctcggt agagccacct ccgcctaaca gacgtttgcg aagtttacgt 60

aaatggcttg ccagacgaac acgtaactct tctgttgaac cgcctccacc tttacccaga 120

gacagggaga 130

<210> 68
<211> 118
<212> DNA
<213> Artificial Sequence

<220>
<223> Fc-BTP25-F

<400> 68
agctgcgtaa gcggctcctc ggcggtggcg gatcgacgga agaacttcgc gtccggttag 60

cgtcacatct tcggaaatta cggaagcgct tgctgtgagg atccgaattc gagctccg 118

<210> 69
<211> 118
<212> DNA
<213> Artificial Sequence

<220>
<223> Fc-BTP25-R

<400> 69
cggagctcga attcggatcc tcacagcaag cgcttccgta atttccgaag atgtgacgct 60

aaccggacgc gaagttcttc cgtcgatccg ccaccgccga ggagccgctt acgcagct 118

<210> 70
<211> 97
<212> DNA
<213> Artificial Sequence

<220>
<223> Fc-BTP26-F

<400> 70

tctccctgtc tctgggtaaa ggtggaggcg gttcaaaatg gaagacacca aaggtgcgcg 60

ttggcggagg tggctctaag tggaaaaccc cgaaagt 97

<210> 71
<211> 97
<212> DNA
<213> Artificial Sequence

<220>
<223> Fc-BTP26-R

<400> 71
actttcgggg ttttccactt agagccacct ccgccaacgc gcacctttgg tgtcttccat 60

tttgaaccgc ctccaccttt acccagagac agggaga 97

<210> 72
<211> 85
<212> DNA
<213> Artificial Sequence

<220>
<223> Fc-BTP27-F

<400> 72
aaaccccgaa agttcgtgtg ggcggtggcg gatcgaaatg gaaaactcct aaagtacggg 60

tctgaggatc cgaattcgag ctccg 85

<210> 73
<211> 85
<212> DNA
<213> Artificial Sequence

<220>
<223> Fc-BTP27-R

<400> 73
cggagctcga attcggatcc tcagacccgt actttaggag ttttccattt cgatccgcca 60

ccgcccacac gaactttcgg ggttt 85

<210> 74
<211> 15
<212> DNA
<213> Artificial Sequence

<220>
<223> Linker

<400> 74
ggtggaggcg gttca 15

<210>    75
<211>    54
<212>    DNA
<213>    Artificial Sequence

<220>
<223>    BTP5


<400>    75
cttcgcaagt tacgtaaacg cttactgtta cgtaaacttc ggaagcgctt actg          54


<210>    76
<211>    15
<212>    DNA
<213>    Artificial Sequence

<220>
<223>    Linker


<400>    76
ggcggaggtg gctct                                                     15


<210>    77
<211>    5
<212>    PRT
<213>    Artificial Sequence

<220>
<223>    Linker


<400>    77
Gly Gly Gly Gly Ser
1               5


<210>    78
<211>    15
<212>    DNA
<213>    Artificial Sequence

<220>
<223>    Linker


<400>    78
ggcggtggcg gatcg                                                     15


<210>    79
<211>    57
<212>    DNA
<213>    Artificial Sequence

<220>
<223>    BTP5


<400>    79

ctgcgtaaac tgcgcaaacg tctgttactt cggaagctga gaaaacgctt actttaa 57

<210> 80
<211> 60
<212> DNA
<213> Artificial Sequence

<220>
<223> BTP11

<400> 80
acagaagagt tacgtgttcg tctggcaagc catttacgta aacttcgcaa acgtctgtta 60

60

<210> 81
<211> 27
<212> DNA
<213> Artificial Sequence

<220>
<223> BTP16

<400> 81
aagtggaaaa ccccgaaagt tcgtgtg 27